# EUROPEAN PATENT APPLICATION

(11) **EP 3 758 004 A1**
(43) Date of publication of application: **30.12.2020**
(21) Application number: 19183026.4
(22) Date of filing: 27.06.2019
(51) Int. Cl.: G16B 5/00, A61K 39/00

(54) **IMMUNOGEN**

(71) Applicant: Ecole Polytechnique Federale De Lausanne (EPFL) EPFL-TTO, 1015 Lausanne (CH)
(72) Inventor: CORREIA, Bruno, 1015 Lausanne (CH); SESTERHENN, Fabian, 1015 Lausanne (CH); YANG, Che, 1015 Lausanne (CH); BONET, Jaume, 1015 Lausanne (CH)
(74) Representative: Williams, Gareth Owen

(57) **Abstract**

Polypeptides useful in the preparation of vaccine compositions against RSV are provided. Also disclosed are methods of enhancing subdominant antibody responses in a subject.

## Description

### FIELD OF THE INVENTION

The present invention relates to a polypeptide which may be used as an immunogen to provoke an immune response. The invention further relates to a vaccine composition comprising the polypeptide. Aspects of the invention further relate to methods for enhancing a subdominant antibody response in a subject. Yet further aspects of the invention relate to methods for designing a peptide, preferably an immunogen, to mimic a complex and/or discontinuous structural configuration of a target peptide.

### BACKGROUND OF THE INVENTION

Throughout the last decades, vaccination has been a key countermeasure to control and eradicate infectious diseases. However, many pathogens (Respiratory Syncytial Virus (RSV), Influenza, Dengue and others) evade the immune system mounting antibody responses that fail to confer broad and potent protection against reinfection, and, in some cases, mediating disease enhancement. Deep profiling of human B-cells often reveals potent neutralizing antibodies emerging from natural infection, but are generally subdominant. A major challenge for next-generation vaccines is to overcome established immunodominance hierarchies, and focus antibody responses on crucial neutralization epitopes.

Vaccination has proven to be one of the most successful medical interventions to reduce the burden of infectious diseases, and the major correlate of vaccine-induced immunity is induction of neutralizing antibodies that block infection. However, classical vaccine approaches relying on inactivated or attenuated pathogen formulations have failed to induce protective immunity against numerous important pathogens, urging the need for novel vaccine development strategies. Structure-based approaches for immunogen design have emerged as promising strategies to elicit antibody responses focused on structurally defined epitopes sensitive to antibody mediated neutralization.

In recent years, advances in high-throughput B-cell technologies have revealed an impressive wealth of potently neutralizing antibodies (nAbs) for different pathogens which have resisted the traditional means of vaccine development for several decades, including HIV-1, Influenza, Respiratory Syncytial Virus (RSV), Zika, Dengue and others. Many of these antibodies have been structurally characterized in complex with their viral target proteins, unveiling the atomic details of neutralization-sensitive epitopes. The large-scale campaigns in antibody isolation, together with detailed functional and structural studies have provided comprehensive antigenic maps of the viral fusion proteins, which delineate epitopes susceptible to antibody-mediated neutralization and provide a roadmap for rational and structure-based vaccine design approaches.

The conceptual framework to leverage neutralizing antibody-defined epitopes for vaccine development is commonly referred to as reverse vaccinology. Although the reverse vaccinology inspired approaches have yielded a number of exciting advances in the last decade, the design of immunogens that elicit such focused antibody responses remains challenging. Successful examples of structure-based immunogen design approaches include the conformational stabilization of RSVF in its prefusion state (preRSVF), yielding superior serum neutralization titers when compared to its postfusion conformation. In the case of Influenza, several epitopes targeted by broadly neutralizing antibodies (bnAbs) were identified within the hemagglutinin (HA) stem domain, and an HA stem-only immunogen elicited a broader neutralizing antibody response than that of full length HA. Commonly, these approaches have aimed to focus antibody responses on specific conformations or subdomains of viral proteins. In a more aggressive approach, Correia et al. computationally designed a synthetic immunogen presenting the RSV antigenic site II, and provided a proof-of-principle for the induction of RSV neutralizing activity mediated by a single epitope in non-human primates.

Efforts to design novel proteins from first principles have revealed a variety of rules to control the structural features of *de novo* proteins (*1-4*). The design of function using computational approaches has been far more challenging as it requires high-precision energy functions and may entail critical parameters that are modelled inaccurately or neglected (e.g. molecular environment, conformational dynamics). Nevertheless, important advances have been made in the design of molecular recognition events by endowing designed proteins with structural motifs which perform their function by binding other proteins. With rare exceptions, the binding motifs transplanted were commonly found in existing protein structures, such as linear helical segments, allowing the grafting of such motifs without extensive backbone adjustments (*5-8*). Commonly, protein function is not contained within single, regular segments in protein structures but it arises from the 3-dimensional arrangement of several, often irregular structural elements that are supported by defined topological features of the overall structure (*9*, *10*). As such, it is of utmost importance for the field to develop computational approaches to endow *de novo* designed proteins with irregular and multi-segment complex structural motifs that can perform the desired functions.

An important domain where functional protein design has raised expectations was on the modulation of immune responses, in particular, on the induction of neutralizing antibodies (nAbs) *in vivo* (*11*). Inducing nAbs targeting defined epitopes remains an overarching challenge for vaccine development. Our increasing structural understanding of many nAb-antigen interactions has provided templates for the rational design of immunogens for respiratory syncytial virus (RSV), influenza, HIV, dengue and others. Despite this extended structural knowledge, these and other pathogens are still lacking efficacious vaccines, highlighting the need for next-generation vaccines that efficiently guide antibody responses towards key neutralization epitopes in both naïve and pre-exposed immune systems. Indeed, the elicitation of antibody responses with defined epitope specificities has been a long-lasting challenge for immunogens derived from modified viral proteins.

A *de novo* design approach towards focusing antibody responses was shown by Correia and colleagues (*11*). Using computational protein design, the RSVF antigenic site II, a linear helix-turn-helix motif that is targeted by a clinically approved monoclonal antibody, was transplanted onto a heterologous protein scaffold. *In vivo,* the epitope-focused immunogen elicited nAbs in non-human-primates (NHPs) after repeated boosting immunizations. Albeit a proof-of-principle for the induction of functional antibodies with a computationally designed immunogen was demonstrated, several major bottlenecks arose: the lack of applicability of the computational approach to structurally complex epitopes and the inconsistent neutralization titers observed in the immunogenicity studies.

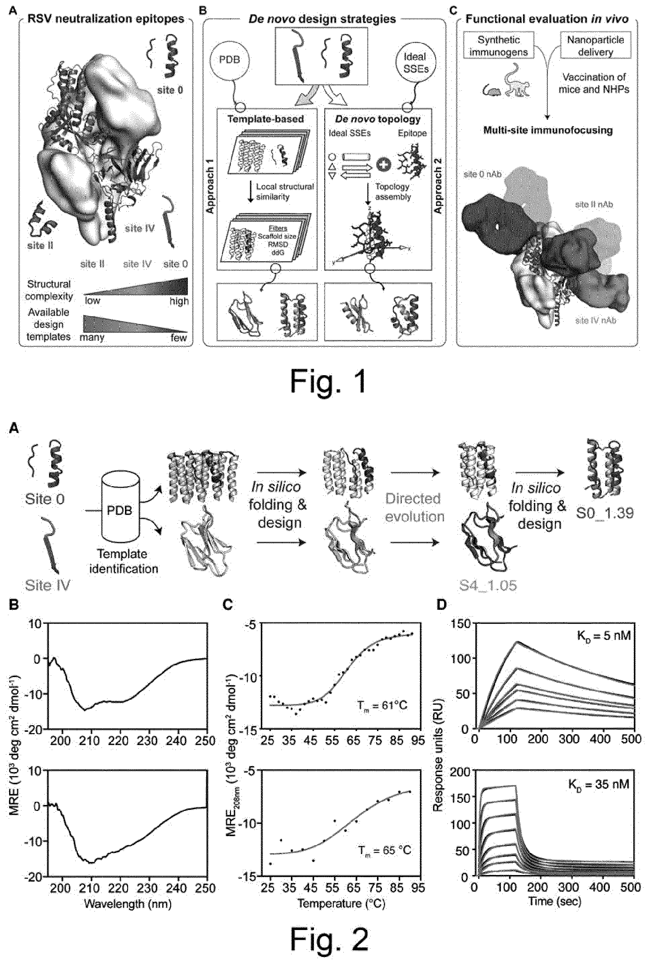

To address these limitations, here we designed epitope-focused immunogens mimicking two irregular and discontinuous RSV neutralization epitopes (site 0 (*12*) and IV (*13*) shown in Fig 1) and showcase two computational design methodologies that enable the presentation of these structurally challenging motifs in *de novo* designed proteins. *In vivo,* cocktail formulations including a previously designed site II immunogen yielded consistent neutralization levels above the protective threshold directed against all three epitopes. The design strategies presented provide a blueprint to engineer proteins stabilizing irregular and discontinuous binding sites, applicable to vaccine design for pathogens that require the fine control over the antibody specificities induced, and more generally to the design of *de novo* proteins displaying complex functional motifs.

### SUMMARY OF THE INVENTION

According to a first aspect of the present invention, there is provided a vaccine composition against a target pathogen, the composition comprising a plurality of non-naturally occurring immunogenic polypeptides; at least a first of said immunogenic polypeptides comprising a mimic peptide having an amino acid sequence having a tertiary structure which, when folded, mimics that of a complex and/or discontinuous neutralisation epitope from said target pathogen.

By "mimics" is meant that the tertiary structure of the amino acid sequence largely replicates that of the complex and/or discontinuous neutralisation epitope from said target pathogen; preferably there is sufficient similarity between the two tertiary structures at least to the extent that the mimic peptide can be bound by a neutralising antibody which targets the complex and/or discontinuous neutralisation epitope from said target pathogen. In most preferred embodiments, either and preferably both of the affinity and avidity of the antibody binding to the mimic peptide are at least 30%, 40%, 50%, 60%, 70%, 80%, 90%, 95% or more of that of the antibody binding to the epitope from the target pathogen.

The invention is based on the design principles and peptides disclosed herein, which permit a complex or discontinuous epitope from a pathogen to be mimicked by a mimic peptide. In the examples herein, the pathogen is RSV. We have found that using a combination of immunogens in a composition - each of which may be designed as described herein, or which may be a combination of said designed immunogens and other immunogens - permits a range of immune responses to be elicited which provides a satisfactory immune response against the pathogen. In certain embodiments, the vaccine composition may be used to enhance an initial subdominant neutralising antibody response (for example, such a subdominant response may occur in response to an initial exposure to the pathogen; as the response is subdominant, it may be insufficient to neutralise the pathogen on subsequent exposure. Enhancing the subdominant response with the vaccine composition described herein may result in a neutralising response on subsequent exposure to the pathogen).

In preferred embodiments, each of said plurality of non-naturally occurring immunogenic polypeptides comprises a mimic peptide having an amino acid sequence which, when folded, mimics a complex and/or discontinuous neutralisation epitope from said target pathogen. Preferably each of said complex and/or discontinuous neutralisation epitopes are non-overlapping. In embodiments where a single mimic peptide is used (and other immunogens are also present), it is preferred that each of the immunogens presents a non-overlapping epitope. It is not necessarily that case, however, that all immunogens comprise a mimic peptide; at least one of the immunogens may be a naturally-occurring immunogen. In this way, multiple separate antibody responses may be elicited against a single pathogen. The combined immune response may be synergistic compared with eliciting individual immune responses to single immunogens.

In preferred embodiments, and as described in the examples herein, said target pathogen is RSV. However, the design principles illustrated herein may be used to prepare vaccines against other pathogens, and in particular against pathogens which may be resistant to conventional vaccine design, for example by virtue of being prone to eliciting subdominant neutralising antibody responses, and/or by virtue of frequent mutation in surface molecules which result in antibody targeting of strain specific epitopes rather than potent neutralising epitopes. Examples of other potentially suitable target pathogens include influenza, HIV, Dengue.

Where the target pathogen is RSV, the complex and/or discontinuous neutralisation epitopes are preferably selected from the group consisting of RSV site 0, site II, and site IV; more preferably epitopes from both sites 0 and IV are used, and most preferably epitopes from all of RSV sites 0, II, and IV are used.

Preferably a mimic peptide targeting RSV site 0 comprises or consists of an amino acid sequence selected from Tables 4 or 6, preferably from table 6, and most preferably comprises or consists of the S0_2.126 peptide sequence. A mimic peptide targeting RSV site IV may comprise of consist of an amino acid sequence selected from Tables 3 or 5, preferably from table 5, and most preferably comprises or consists of the S4_2.45 peptide sequence. A mimic peptide targeting RSV site II may comprise or consist of the FFL_001 or FFLM peptides (and preferably the FFLM peptide) described in Sesterhenn et al 2019 (PLoS Biol. 2019 Feb; 17(2): e3000164, doi: 10.1371/journal.pbio.3000164). The FFLM peptide has the amino acid sequence
ASREDMREEADEDFKSFVEAAKDNFNKFKARLRKGKITREHREMMKKLAKQNANKAKEAV RKRLSELLSKINDMPITNDQKKLMSNQVLQFADDAEAEIDQLAADATKEFTG (SEQ ID NO: 1), and is also referred to herein as S2_1 or S2_1.2.

In certain embodiments of the invention, the immunogenic peptide may comprise a scaffold, preferably a peptide scaffold, which presents the mimic peptide so as to assist the mimicking of the complex and/or discontinuous neutralisation epitope. For example, a designed mimic sequence may be fused to a scaffold sequence in a linear manner. Alternatively, a mimic sequence may be grafted onto or fused to two or more structural framework elements (eg, helices, sheets, etc) in a non-linear manner, so as to present the mimic sequence in a desired structural manner. The mimic sequence itself may comprise multiple sequences, in particular if presented on multiple structural elements. The scaffold may form a nanoparticle comprising multiple immunogenic peptides, with said nanoparticle preferably being soluble. In preferred embodiments, the scaffold may be selected from RSVN and ferritin.

The vaccine composition of the invention may be provided in combination with a vaccine composition comprising a native immunogen from the target pathogen. These may be provided separately (as separate compositions) or together (as a single vaccine composition). Also provided by the invention is a kit comprising multiple vaccine compositions, as described herein. For example, where the pathogen is RSV, the native immunogen may be an additional RSV-derived protein or glycoprotein, and most preferably the RSVF glycoprotein, or an RSVF protein precursor (for example, the core peptide sequence, or preRSVF). An example RSVF protein sequence is given in the UniProt KB database as entry A0A110BF16 (A0A110BF16_HRSV). Note that reference to a "native immunogen" does not require that the immunogen is obtained directly from the pathogen; clearly other expression systems or synthetic sources can be used. The intention is that the immunogen has the same sequence / configuration as the immunogen when present in or on the pathogen. Where the vaccines are provided as separate compositions, the vaccines may be administered in a prime:boost schedule (that is, administration of the native immunogen vaccine as a "prime" administration, followed thereafter by the other vaccine as a "boost"); such a schedule is believed to enhance an initial subdominant neutralising immune response seen in response to the prime vaccine. In certain embodiments of the invention, the vaccine composition (without the native immunogen) may be administered to a subject who has previously been exposed to the native immunogen. Such an administration schedule is believed to have a similar effect to the prime:boost schedule without the need for a separate prime administration. The schedule may comprise administering multiple boost vaccinations. The prime and first boost vaccinations may be administered according to any suitable schedule; for example, the two vaccinations may be administered one, two, three, four or more weeks apart. Where multiple boost vaccinations are administered, these too may be administered according to any suitable schedule; for example, the two vaccinations may be administered one, two, three, four or more weeks apart. Preferably two boost vaccinations are administered.

In one particularly preferred aspect of the invention, there is provided a vaccine composition comprising the S0_2.126 peptide sequence as described herein, and the S4_2.45 peptide sequence as described herein. The composition may further comprise the FFL_001 or FFLM peptides described in Sesterhenn et al 2019 (FFLM is also referred to herein as S2_1 or S2_1.2). Either or preferably both of the S0_2.126 and the S4_2.45 peptide sequences may be conjugated to ferritin. The FFL_001 or FFLM peptide sequence may be conjugated to RSVN.

Vaccine compositions described herein may further comprise one or more pharmaceutically acceptable carriers, and/or adjuvants. The adjuvant may be AS04, AS03, alhydrogel, and so forth.

The vaccine compositions described herein may be administered via any route including, but not limited to, oral, intramuscular, parenteral, subcutaneous, intranasal, buccal, pulmonary, rectal, or intravenous administration.

Also provided is a vaccine composition as described herein, wherein said target pathogen is RSV, for use in a method for immunising a subject against RSV, the method comprising a) administering said vaccine composition to a subject; and b) prior to said administration, administering a further vaccine composition comprising an RSV-derived protein or glycoprotein, preferably the RSVF glycoprotein, or wherein the vaccine composition of any preceding claim is administered to a subject who has previously been exposed to RSV infection.

Yet further provided by the present invention is a peptide sequence as described herein; a nucleic acid sequence encoding a peptide sequence as described herein; and a vector comprising such a nucleic acid sequence. Still further provided is use of a peptide sequence as described herein in the manufacture of a vaccine composition. Also provided is a method of vaccinating a subject, the method comprising administering a vaccine composition as described herein.

A further aspect of the invention provides a method for designing a peptide (preferably an immunogen) to mimic a complex and/or discontinuous structural configuration of a target peptide (preferably also an immunogen), the method comprising the steps of:
determining a complex and/or discontinuous structural configuration of a target peptide to mimic;
identifying a preliminary mimic peptide having an amino acid sequence;
determining likely structural configuration of said preliminary mimic peptide amino acid sequence by in silico analysis of said sequence;
performing directed evolution on said preliminary mimic peptide to generate a range of variants of said peptide; (preferably wherein directed evolution may be performed by mutagenesis to generate variants and expression of said variants); and
selecting for variants of said peptide which display an improvement in a desired characteristic seen in said target peptide (said characteristic may be, for example, binding affinity to a target such as an antibody; thermal stability; susceptibility or resistance to an enzyme).

The method may further comprise the steps of identifying a plurality of said variants having improvements, and providing a further peptide having a combination of variations from said plurality of variants. The method may further be repeated for further rounds of generation and selection of variants.

The step of identifying a preliminary mimic peptide may comprise selecting a peptide from a peptide database having a structural similarity to the desired target peptide; or said step may comprise combining an amino acid sequence from said target peptide with one or more structural peptide elements such that said preliminary mimic peptide has a structural similarity to the desired target peptide.

Yet further provided herein is a design protocol as described; at least in part with reference to the TopoBuilder design protocol as described herein. In said design protocol, the placement of idealized secondary structure elements are sampled parametrically, and are then connected by loop segments (for example, structural elements such as loops, sheets, helices), to assemble topologies that can stabilize the desired conformation of the structural motif. These topologies are then diversified to enhance structural and sequence diversity with a folding and design stage. This permits two design objectives to be achieved: (1) building stable topologies de novo that stabilize the epitope, while mimicking its native quaternary environment; (2) Fine-tuning the topology's secondary structure arrangement to maximize the fold stability and optimize epitope presentation for high affinity antibody binding.

### BRIEF SUMMARY OF THE FIGURES

These and other aspects of the invention will now be described in detail, and with reference to the following figures.
Fig 1 - **Conceptual overview of the computational design of synthetic immunogens to elicit RSV neutralizing antibodies focused on three distal epitopes.** (A) Prefusion RSVF structure (PDB 4JHW) with sites 0, II and IV highlighted. An immunogen for site II was previously reported (*11*). (B) Computational protein design strategies. Approach 1: Design templates were identified in the PDB based on loose structural similarity to site 0/IV, followed by *in silico* folding and design, and sequence optimization through directed evolution. Approach 2: A motif-centric design *de novo* design approach was developed to tailor the protein topology to the motif's structural constraints. Bottom: Computational models of designed immunogens with the different approaches. (C) Cocktail formulations of three synthetic immunogen nanoparticles elicit neutralizing antibodies (nAbs) focused on three non-overlapping epitopes.
Fig 2 - **Templated computational design and biophysical characterization of RSVF synthetic immunogen.** (A) Protein design strategy. Templates with structural similarity to sites 0 and IV were identified in the PDB, followed by *in silico* folding, design and directed evolution optimization. An additional in silico folding and design step was necessary to install site 0 on a truncated template sequence revealed by directed evolution. Computational models of intermediates and final designs (S0_1.39 and S4_1.5) are shown. (B) CD spectra measured at 20 °C of S0_1.39 (top) and S4_1.5 (bottom), are in agreement with the expected secondary structure content of the design model. (C) Thermal melting curves measured by CD at 208 nm in presence of 5 mM TCEP reducing agent. (D) Binding affinity measured by SPR against target antibodies D25 (top) and 101F (bottom). CD = Circular dichroism, Tₘ = melting temperature. SPR = Surface plasmon resonance.

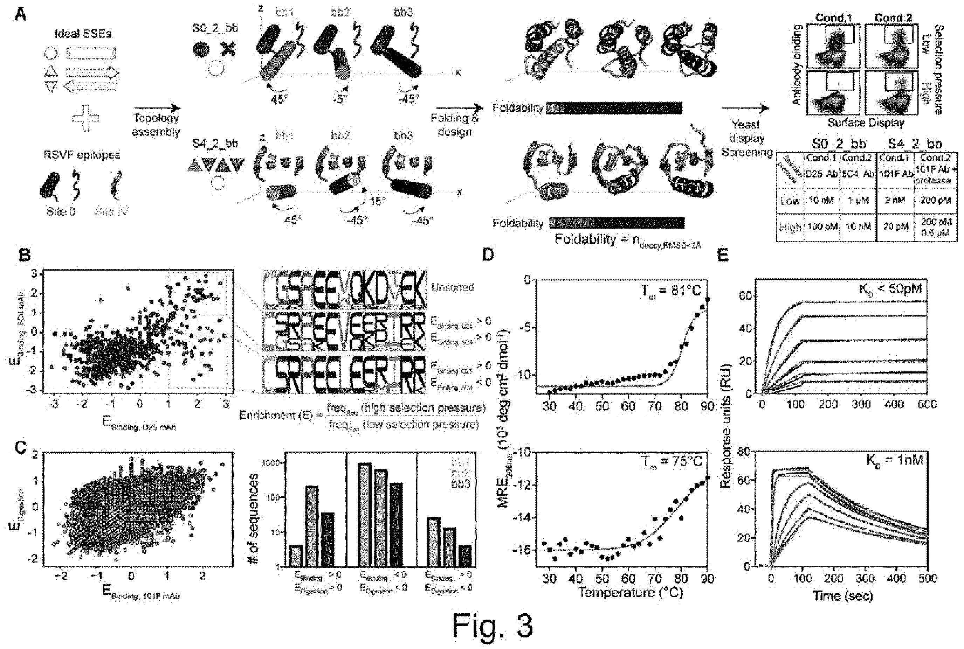

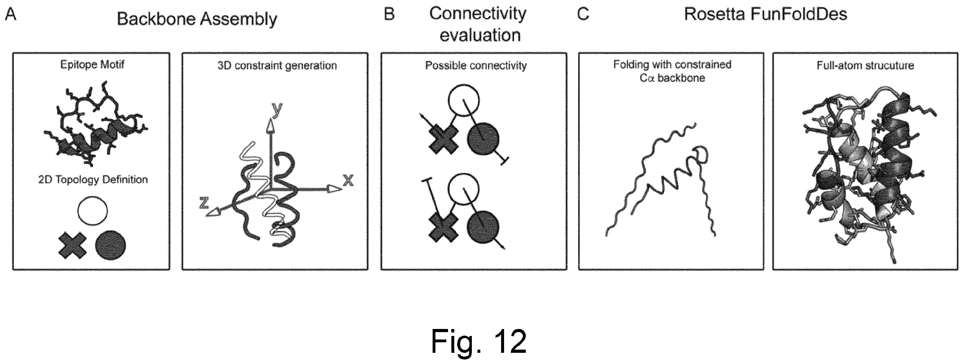

Fig 3 - **Motif-centric *de novo* design of epitope-focused immunogens.** (A) Ideal secondary structure elements (SSE) are assembled around RSVF epitopes, sampling different orientations within the same topology, followed by a single round of *in silico* folding and design. See Fig 12 for further details. Rosetta *abinitio* simulations are performed for each topology to assess its propensity to fold into the designed structures, returning a foldability score. Selected designs are then displayed on yeast surface and sorted under two different selection pressures for subsequent deep sequencing. (B) Enrichment analysis of sorted populations under high and low selective pressures. Sequences highly enriched for both D25 and 5C4 binding show convergent sequence features in critical core positions of the site 0 scaffold. (C) All three designed topological variations were screened for high affinity binding and resistance to chymotrypsin to select stably folded proteins. Enrichment analysis revealed a strong preference for one of the designed helix orientations (S4_2_bb2) to resist to protease digestion and bind with high affinity to 101F (green). (D) Thermal melting curves measured by CD for best designs (S0_2.126, top and S4_2.45, bottom) showing high thermostability. (E) Dissociation constants (K_{D}) of S0_2.126 to D25 (top) and S4_2.45 to 101F antibodies measured by SPR.

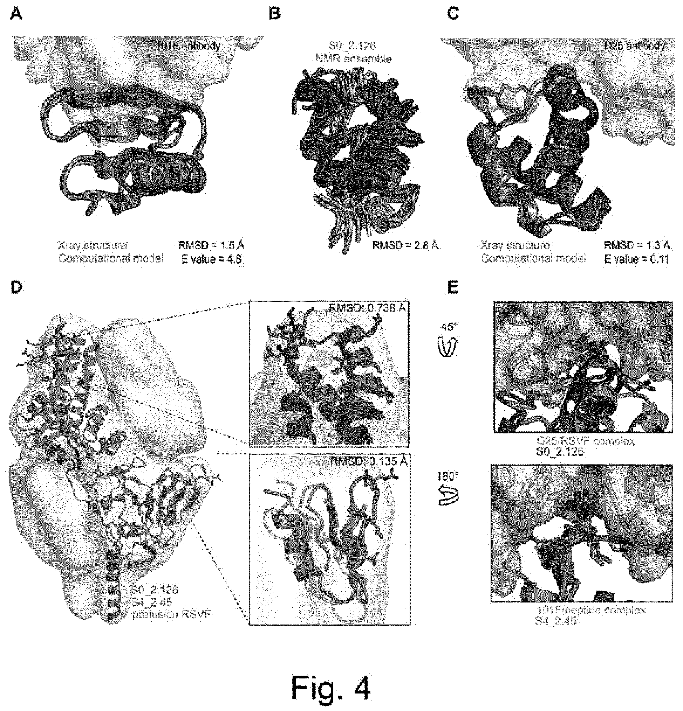

Fig 4 - **Structural characterization of *de novo* designed immunogens.** (A) Crystal structure of S4_2.45 (orange) bound to 101F Fab closely matches design model (grey, RMSD = 1.5 Å). (B) NMR structural ensemble of S0_2.126, D25 epitope highlighted in purple. The NMR structure is well in agreement with the design model (backbone RMSD of 2.8 Å). (C) Crystal structure of S0_2.126 (purple) bound to D25 Fab closely resembles the design model (grey, RMSD = 1.3 Å). (D) Superposition of the native preRSVF site 0/IV and designed immunogens shows sub-angstrom mimicry of the epitopes. Designed scaffolds are compatible with the shape constraints of preRSVF (surface representation). (E) Close-up view of the interfacial side-chain interactions between D25 (top) and 101F (bottom) with designed immunogens as compared to the starting epitope structures (preRSVF, site IV peptide).

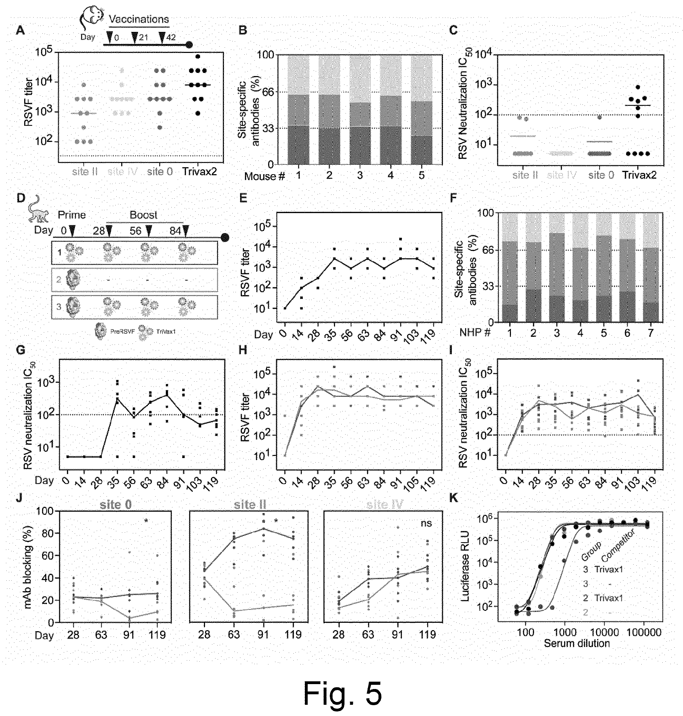

Fig 5 - **Synthetic immunogens elicit neutralizing serum responses in mice and NHPs and focus pre-existing immunity on sites 0 and II.** (A-C) Trivax2 immunization study in mice. (A) PreRSVF cross-reactive serum levels following three immunizations with single immunogens or Trivax2 cocktail (day 56). (B) Serum specificity shown for 5 representative mice immunized with Trivax2, as measured by an SPR competition assay with D25, Motavizumab and 101F IgGs as competitors, shows an equally balanced response towards all sites. (C) RSV neutralization titer of mice at day 56, immunized with Trivax2 components individually and as cocktail. Dotted line (IC₅₀ = 100) indicates protective threshold. (D-K) Trivax1 immunization study in NHPs. (D) NHP immunization scheme. (E) PreRSVF cross-reactive serum levels for group 1. (F) Serum antibodies target all three antigenic sites in all 7 animals as measured by an SPR competition assay. (G) RSV neutralization titers of group 1. (H) PreRSVF titer in group 2 (grey) and 3 (blue). (I) RSV neutralization titer of group 2 and 3. (J) Site-specific antibody levels measured by SPR competition assay. Site 0 and site II-specific titers were significantly higher in group 3 compared to 2 following Trivax1 boosting (p < 0.05, Mann-Whitney U test). (K) RSV neutralization curves upon depletion of day 91 sera with site 0, II, IV-specific scaffolds. 60% of the neutralizing activity is competed in group 3, whereas no significant decrease is observed in the control group 2.

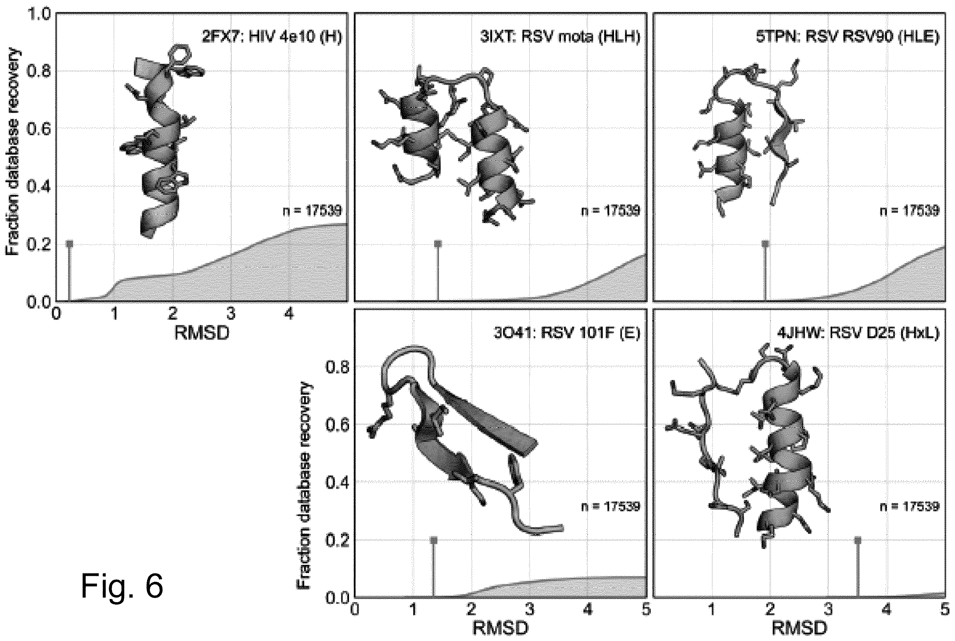

Fig 6 - **The increase in structural complexity of the functional motifs determine the number of designable templates that are found in known structures.** A MASTER search {Zhou, 2015 #1431} was performed over the nrPDB30 database containing a total of 17539 structures, querying the number of matches for different neutralization epitopes (colored in blue in the structures) of increasing structural complexity. The fraction of the database recovered is plotted on the y-axis. Matches were filtered for protein size <180 residues. The vertical line (orange) indicates the RMSD cutoff for the first 10 scaffold identified. Secondary structure composition of the motifs is represented by: E - strand; L - Loop; H - helix; x - chain break.

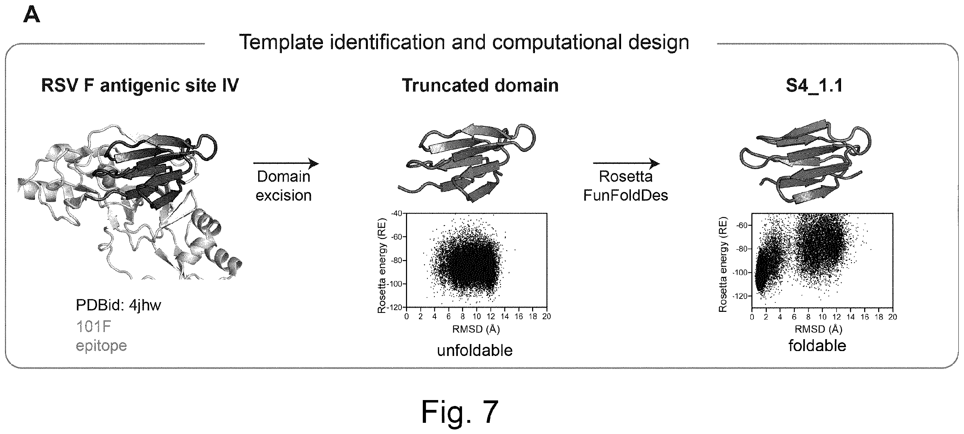

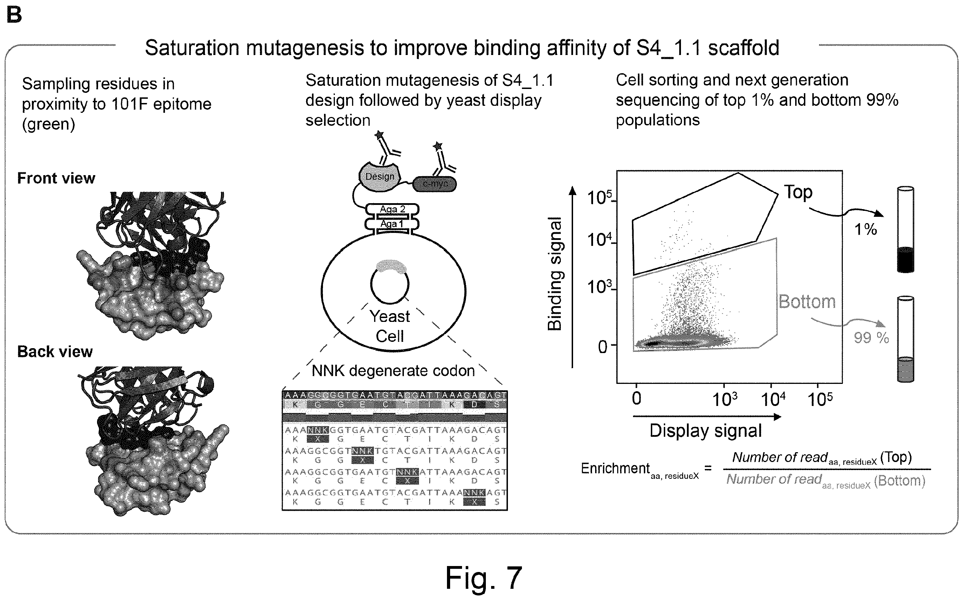

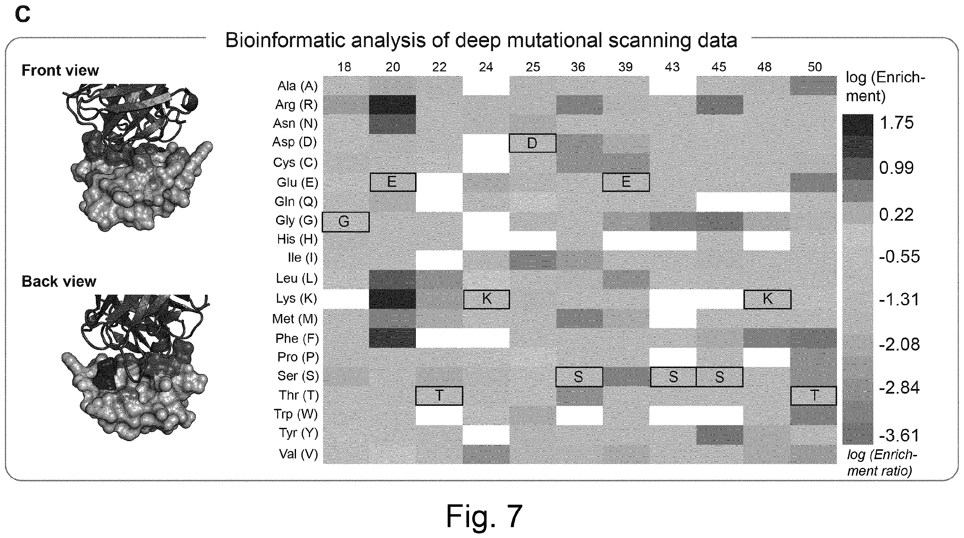

Fig 7 - **Computational design and experimental optimization of S4_1 design series.** A) Template identification and computational design of S4_1.1. RSVF antigenic site IV is located in a small contained domain of preRSVF. This excised domain failed to show a folding funnel in Rosetta abinitio predictions, and failed to express recombinantly in E.coli. Using the excised domain as template, we folded and sequence-designed this topology using Rosetta FunFolDes, yielding design S4_1.1 which showed a strong funnel-shape energy landscape in abinitio folding simulation. B) Experimental optimization of S4_1.1 through saturation mutagenesis. A saturation mutagenesis library was constructed using overhang PCR for 11 positions proximal to the site IV epitope, allowing one position at a time to mutate to any of the 20 amino acids, encoded by the degenerate codon 'NNK'. The library (size 11 positions x 32 codons = 352) was transformed in yeast, and designs were displayed on the cell surface. The selection was done by labeling the cells with 125 nM of 101F antibody. The top 1 % of clones binding with high affinity to 101F antibody were then sorted, as well as the bottom 99 % as shown. Following next-generation sequencing of the two populations, the enrichment values were computed for each sequence variant. C) Bioinformatic analysis of deep mutational scanning data. The log(enrichment) is shown as heatmap for each sequence variant. White indicates missing data. Position 20 showed the highest enrichment for arginine and lysine, together with other less pronounced enrichments seen for other positions.

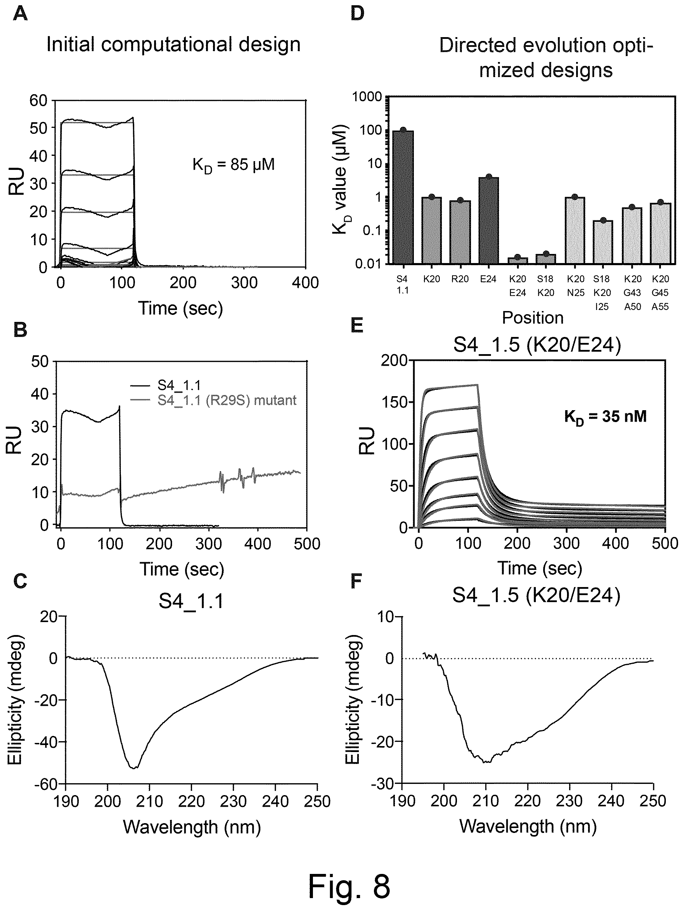

Fig 8 - **Experimental characterization of S4_1 design series.** A) Top: Surface plasmon resonance measurement for the initial computational design S4_1.1 against 101F antibody revealed a dissociation constant of > 85 µM. Middle: Despite low affinity, an R29S mutant revealed that binding was specific to the epitope of interest. Bottom: Circular dichroism spectrum of S4_1.1. B) Top: Dissociation constants for single and combined mutations of S4_1.1 that were identified in the deep mutational scanning screen. K20/E24 double mutant (named S4_1.5) showed a binding affinity of 35 nM (middle). Bottom: Circular dichroism spectrum of S4_1.5.

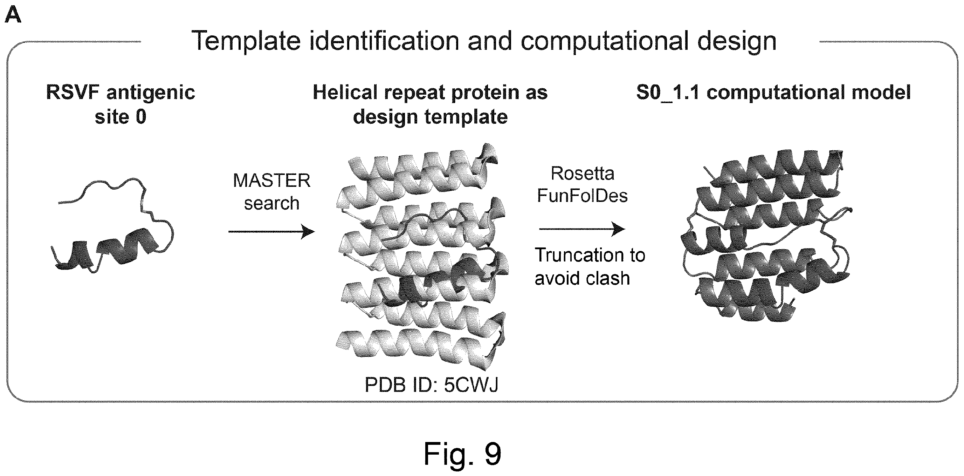

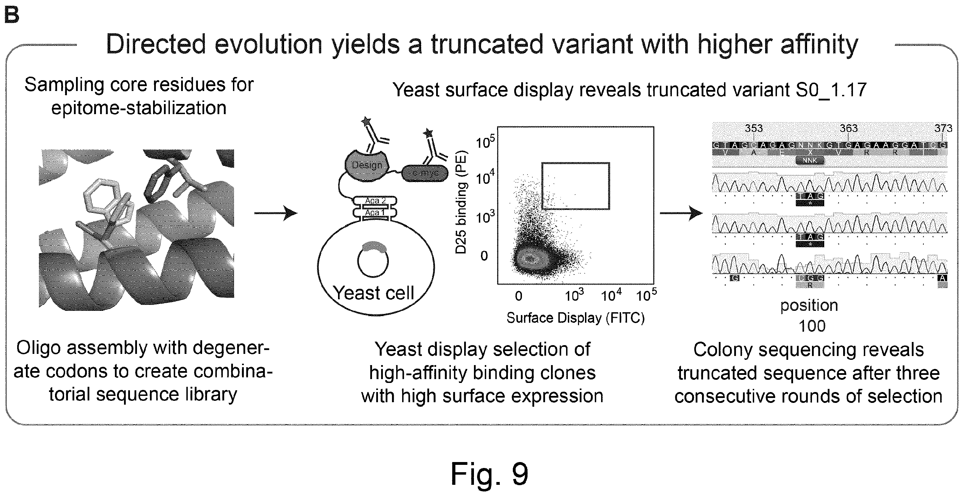

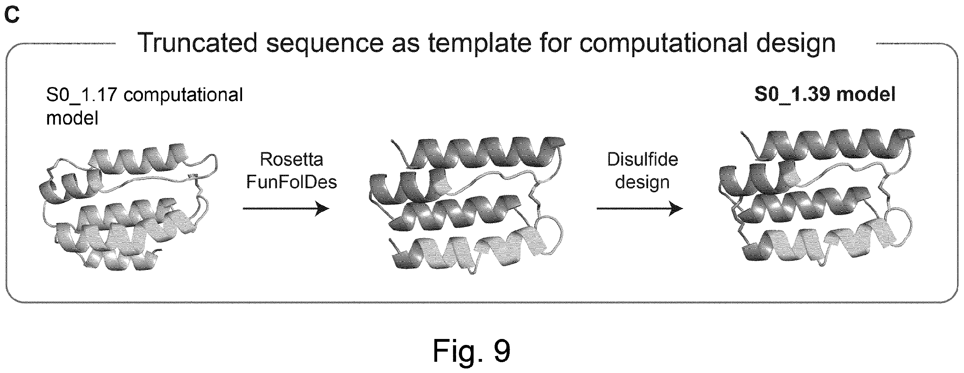

Fig 9 - **Computational design and experimental optimization of S0_1 design series.** A) Template identification and design. Using MASTER, we identified a designed helical repeat protein (PDB ID: 5CWJ) to serve as design template to present and stabilize antigenic site 0 (see methods for details). The Nterminal 29 residues were truncated to avoid clashing with the D25 antibody, and Rosetta FunFolDes was used to design S0_1.1. See methods for details on the design process. B) Based on S0_1.1, a combinatorial sequence library was constructed and screened using yeast surface display. After three consecutive sorts of high-affinity binding clones, individual colonies were sequenced. Position 100 was frequently found to be mutated to a stop codon, leading to a truncated variant with increased expression yield. C) A model of the truncated variant served as template for a second round of in silico folding and design. We truncated the template further by the N terminal 14 residues, and introduced a disulfide bond between residues 1 and 43, leading to S0_1.39. See methods for full details on the design selection process.

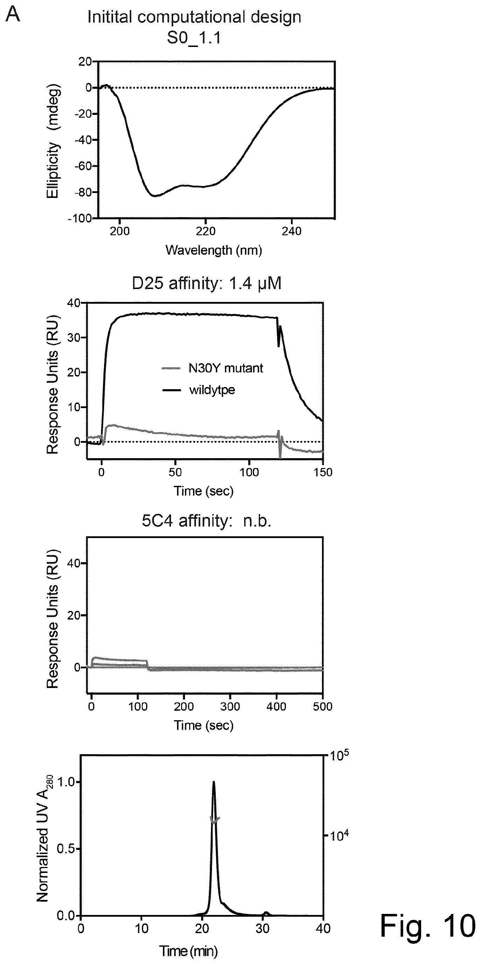

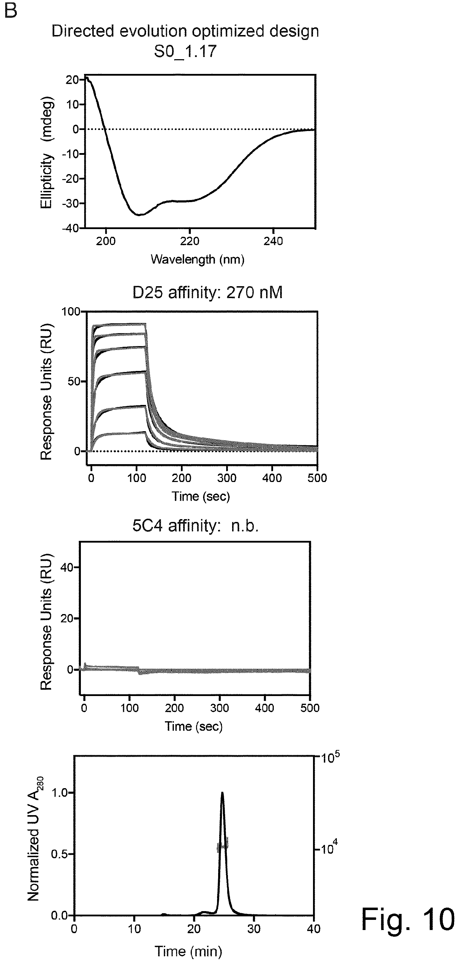

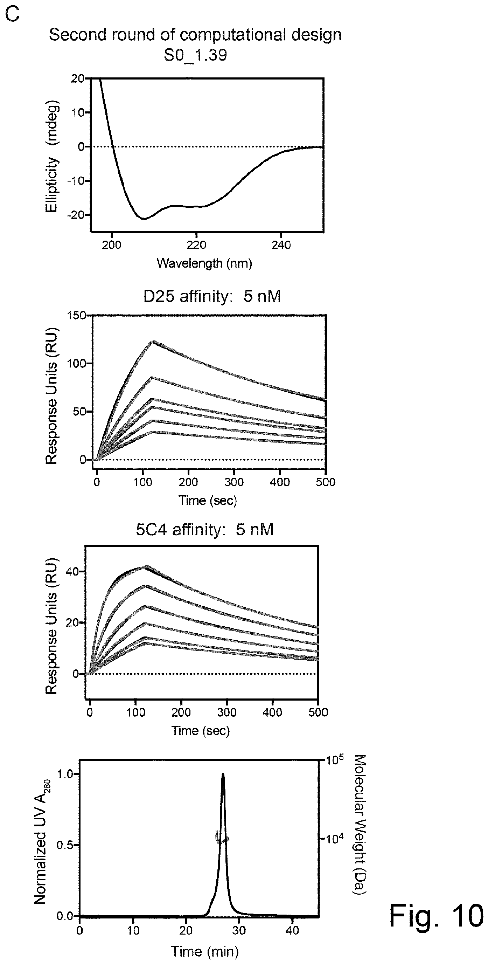

Fig 10 - **Biophysical characterization of the S0_1 design series.** Top: Circular dichroism spectra. Middle: Surface plasmon resonance measurements against D25 and 5C4. Bottom: Multi-angle light scattering coupled to size exclusion chromatography. A) S0_1.1 bound with a KD of 1.4 µM to D25 and no detectable binding to 5C4. To verify that the binding interaction was specific to the epitope we generated a knockout mutant (N30Y) and observed that the binding interaction was absent. B) S0_1.17 showed a KD of 270 nM to D25 and no binding to 5C4. C) S0_1.39 binds with a KD of 5 nM to 5 D24 and 5C4. All designs showed CD spectra typical of helical proteins and behaved as monomers is solution (Top and bottom rows).

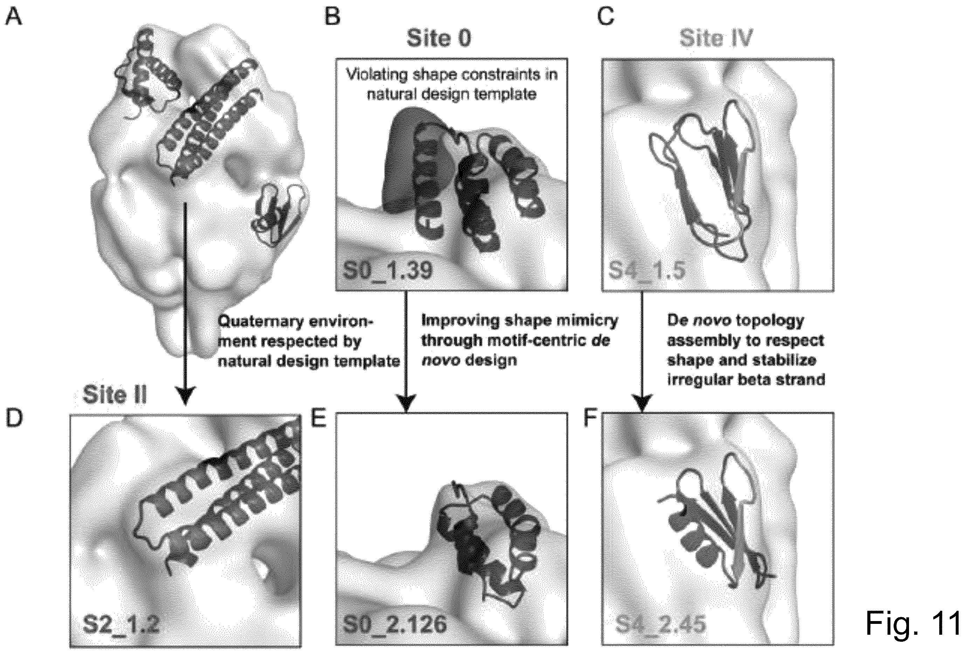

Fig 11 - **Shape mimicry of computationally designed immunogens compared to prefusion RSVF.** A) Designed immunogens (S2_1.2, S0_2.126, S4 2.45) superimposed to prefusion RSVF (PDB 4JHW), shown in surface representation (grey). (B, C) Close-up view of S0_1.39 and S4_1.5 with RSVF shown in surface representation. The design template used for the S0_1.39 design violates the shape constraints of the site 0 epitope in its native environment (preRSVF). While site 0 is freely accessible for antibody binding in preRSVF, the C-terminal helix of S0_1.39 constrains its accessibility (dark grey surface). (D) The design template used for the previously designed site II antigen respected the site II quaternary environment. (E,F) De novo templates were built in order to respect the epitope's shape constraints, and to improve epitope stabilization compared to naturally occurring design templates.
Fig 12 - **De novo computational method to assemble idealized SSEs and motif of interest.**

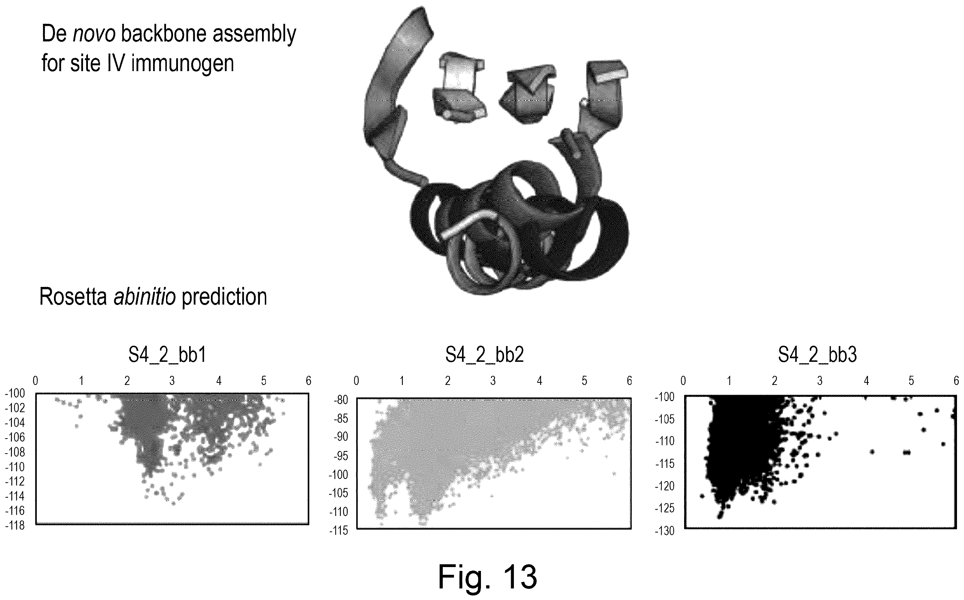

Fig 13 - **De novo backbone assembly for site IV immunogen.** The site IV epitope was stabilized with three antiparallel beta strands built de novo, and a helix packing in various orientations against this beta sheet (bb1-bb3). Each backbone was simulated in Rosetta abinitio simulations for its ability to fold into a low energy state that is close to the design model, indicating that S4_2_bb2 and bb3 have a stronger tendency to fold into the designed fold.

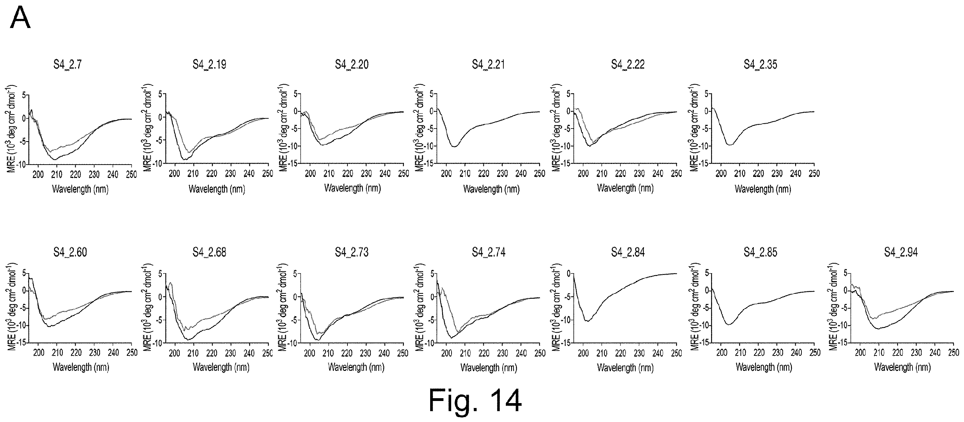

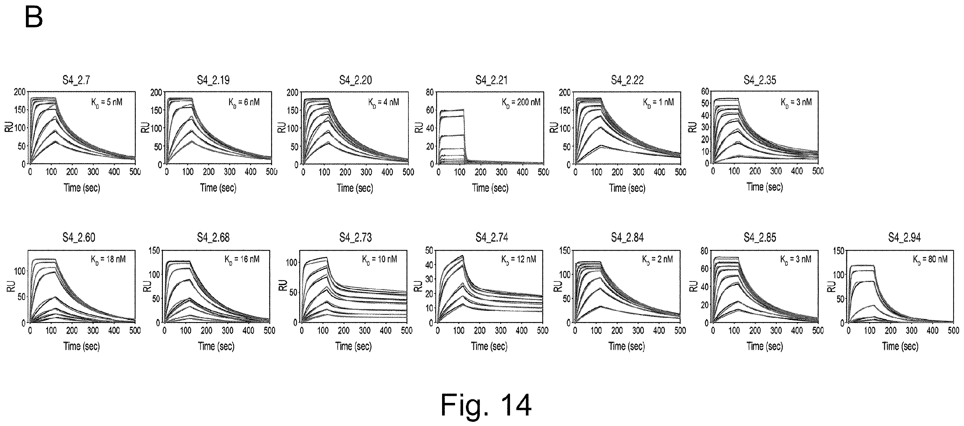

Fig 14 - **Biophysical characterization of de novo site IV designs.** Shown are circular dichroism spectra and SPR sensorgrams against 101F for 13 designs of the S4_2 design series that were enriched for protease resistance and binding to 101F in the yeast display selection assay.

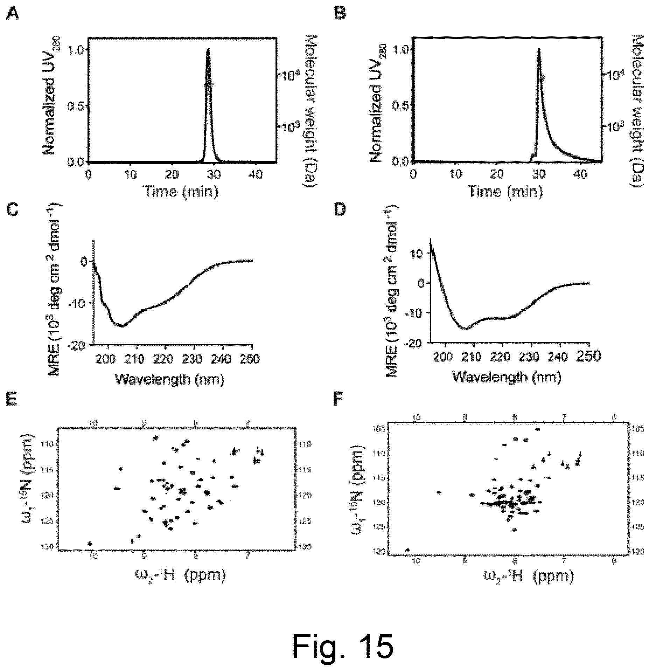

Fig 15 - **Biophysical characterization of S4_2.45 (A,C,E) and S0_2.126 (B,D,F).** A,B: S4_2.45 and S0_2.126 are monomeric in solution as shown by SEC-MALS profile. C,D: Circular dichroism spectra at 25 ºC. E,F: 2D NMR of 15N HSQC spectra for S4_2.45 (E) and S0_2.126 (F) are well dispersed, confirming that the designs are well folded in solution.

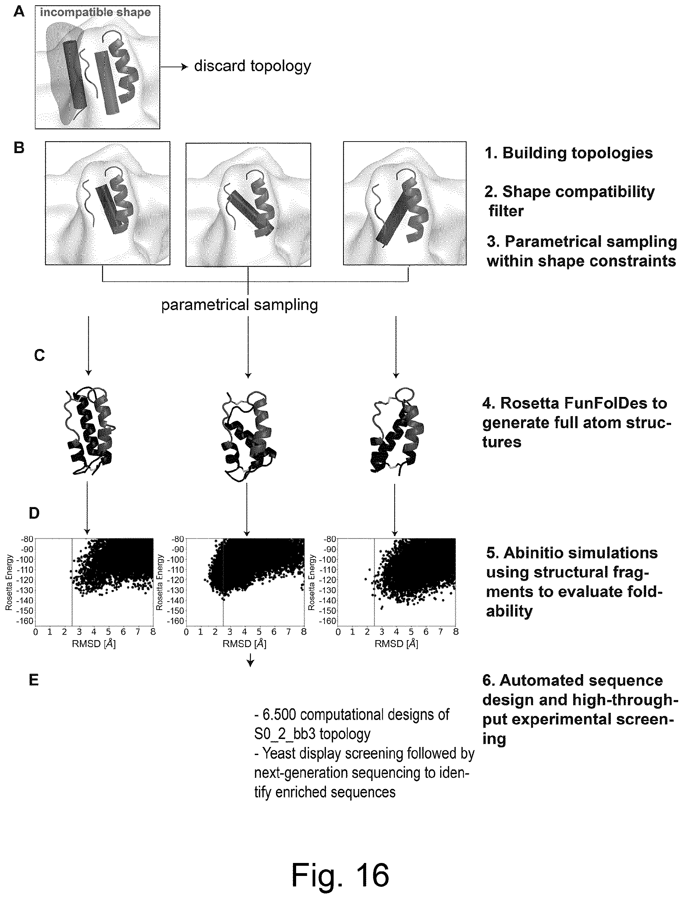

Fig 16 - **De novo topology assembly to stabilize site 0**. Three customized helical orientations were assembled (S0_2_bb1-bb3) to support site 0 epitope, and evaluated for their ability to fold into the designed topology in Rosetta abinitio simulations. S0_2_bb3 showed a funnel-shaped energy landscape, and was selected for subsequent sequence design.

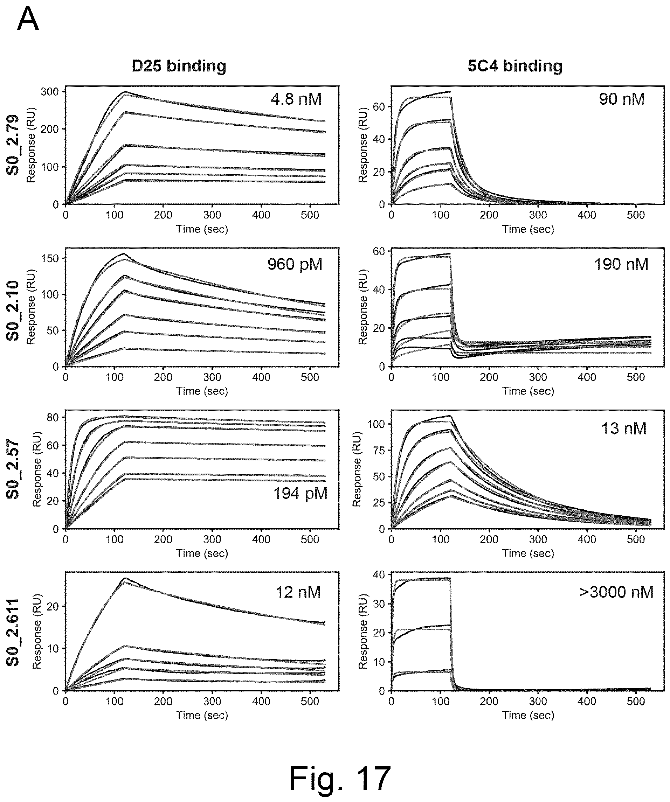

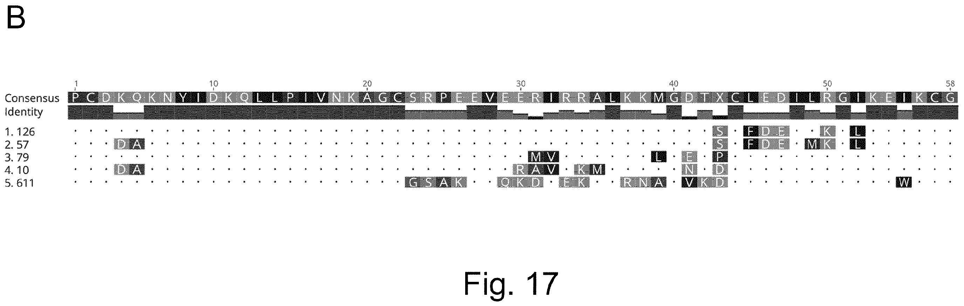

Fig 17 - (A) Binding affinity measurement for D25 and 5C4 binding of de novo site 0 scaffolds. Shown are the SPR sensorgrams of enriched designs that were successfully expressed and purified after the yeast display selection. (B) Sequence alignment of experimentally characterized sequences.

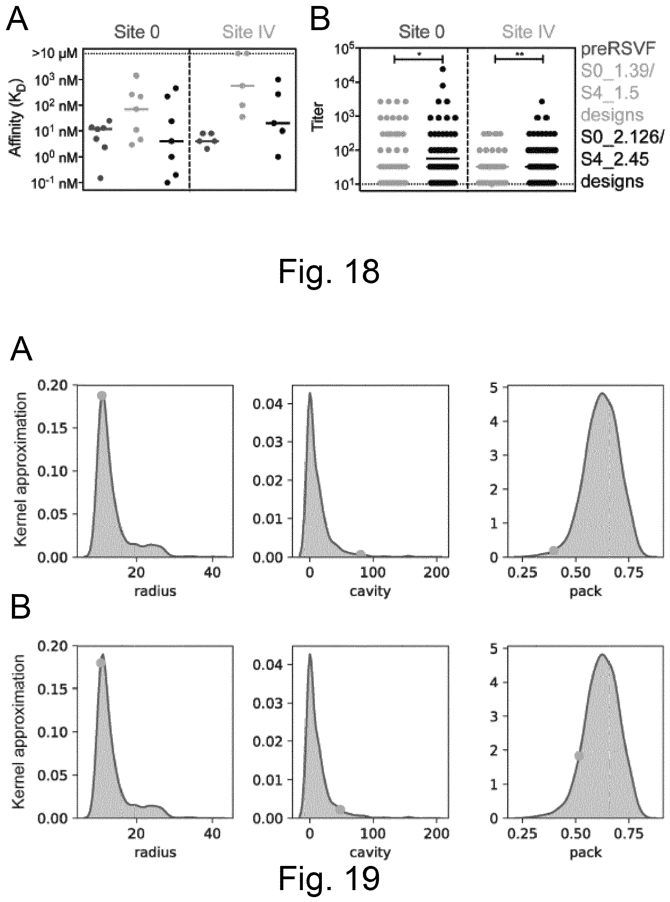

Fig 18 - **Binding affinity of designed immunogens towards panels of site-specific, human neutralizing antibodies and human sera.** A) Binding affinity (KD, determined by SPR flowing Fabs as analyte) of S0_1.39 (grey) and S0_2.126 (black) towards a diverse panel of site-specific neutralizing antibodies, in comparison to prefusion RSVF (blue). Antibodies shown for site 0 are 5C4, D25 (1), ADI-14496, ADI-18916, ADI-15602, ADI-18900 and ADI-19009 (2). For site IV, the binding affinity was tested against 101F (3), ADI-15600 (2), 17E10, 6F18 and 2N6 (4), comparing S4_1.5 (grey) and S4_2.45 (black) to prefusion RSVF. The higher binding affinity of the second-generation designs (S0_2.126 and S4_2.45) compared to the first-generation and to prefusion RSVF indicates a greatly improved, near-native epitope mimicry of the respective antigenic sites in the designed immunogens. B) ELISA reactivity of designed immunogens with sera obtained from 50 healthy human adults that were seropositive for prefusion RSVF. Both S0_2.126 and S4_2.45 showed significantly increased reactivity compared to the first-generation designs, confirming an improved epitope-mimicry on the serum level (* p< 0.05 and ** p< 0.01, Wilcoxon test).
Fig 19 - **Comparison of S0_2.126 Rosetta scores against natural proteins.** Protein structures within the same size as S0_2.126 (57 +/- 5 residues) were downloaded from the CATH database and filtered by 70 % sequence homology, yielding a representative database of natural proteins with similar size as S0_2.126 (n = 1,013 structures). Proteins were then minimized and scored by Rosetta to compute their radius of gyration, intra-protein cavities (cavity) and core packing (packstat). Plotted is the distribution for these score terms in 1,013 natural proteins (blue histogram), and the same scores for S0_2.126 are shown in orange. The NMR structure of S0_2.126 is shown in (A), the computational model of S0_2.126 is shown in (B), indicating that, despite similar radius of gyration, S0_2.126 shows a substantial cavity volume as well as a very low core packing compared to natural proteins of similar size.

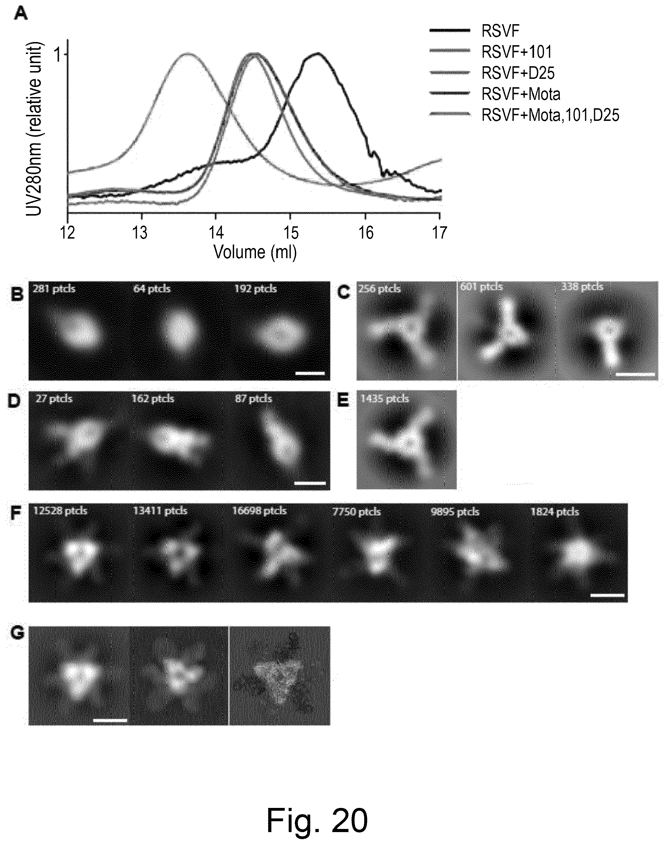

Fig 20 - **Electron microscopy analysis of site-specific antibodies in complex with RSVF trimer.** (A) Superposed size-exclusion profiles of unliganded RSVF (black line) and RSVF in complex with 101F (green line), D25 (blue line), Mota (purple line) and all three (101F, D25, Mota - red line) Fabs. (B-F) Representative reference-free 2D class averages of the unliganded RSVF trimer (B) and RSVF in complex with 101F (C), D25 (D), Mota (E) or all three (101F, D25, Mota (F)) Fabs. Fully-saturated RSVF trimers bound by Fabs are observed, as well as sub-stoichiometric classes. (G) Left panel: referencefree 2D class average of RSVF trimer with three copies of 101F, D25 and Mota Fabs visibly bound. Right panel: predicted structure of RSVF trimer with bound 101F, D25 and Mota Fabs based on the existing structures of RSVF with individual Fabs (PDB ID 4JHW, 3QWO and 3O45). The predicted structure of RSVF in complex with 101F, D25 and Mota was used to simulate 2D class averages in Cryosparc2, and simulated 2D class average with all three types of Fabs is shown in the middle panel. Fabs are colored as follow: red - 101F; blue - Mota; green - D25. Scale bar - 100 Å.

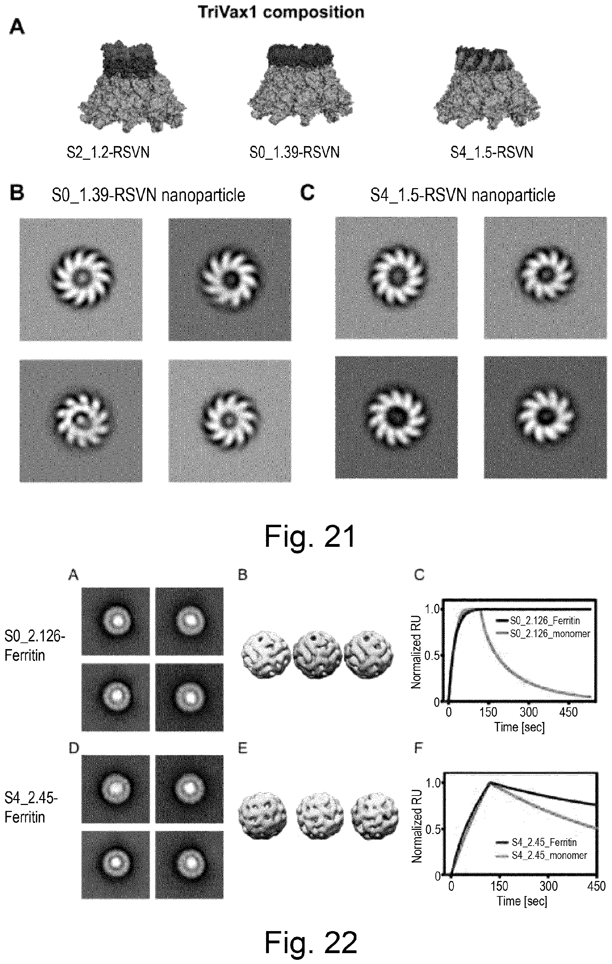

Fig 21 - **Composition and EM analysis of Trivax1 RSVN nanoparticles.** A) Trivax1 contains equimolar amounts of site II, 0 and IV epitope focused immunogens fused to the self-assembling RSVN nanoparticle with a ring-like structure (n = 10-11 subunits). The site II-RSVN nanoparticle has been described previously (5). Shown are the computational models for the nanoparticles-immunogen fusion proteins. B,C) Negative stain electron microscopy for S0_1.39-RSVN and S4_1.5-RSVN nanoparticles confirms that the ring-like structure is maintained upon fusion of the designed immunogens.
Fig 22 - **EM analysis of Trivax2 ferritin nanoparticles.** A,B,D,E) Negative stain electron microscopy (A,D) and 3D reconstruction (B,E) for S0_2.126 and S4_2.45 fused to ferritin nanoparticles. C) Binding affinity of S0_2.126 nanoparticle (blue) to 5C4 antibody in comparison to S0_2.126 monomer (red), showing that S0_2.126 has been successfully multimerized and antibody binding sites are accessible. F) Binding of S4_2.45 to 101F antibody when multimerized on ferritin nanoparticle (blue) compared to monomeric S4_2.45 (red), indicating that the scaffold is multimerized and the epitope is accessible for antibody binding.

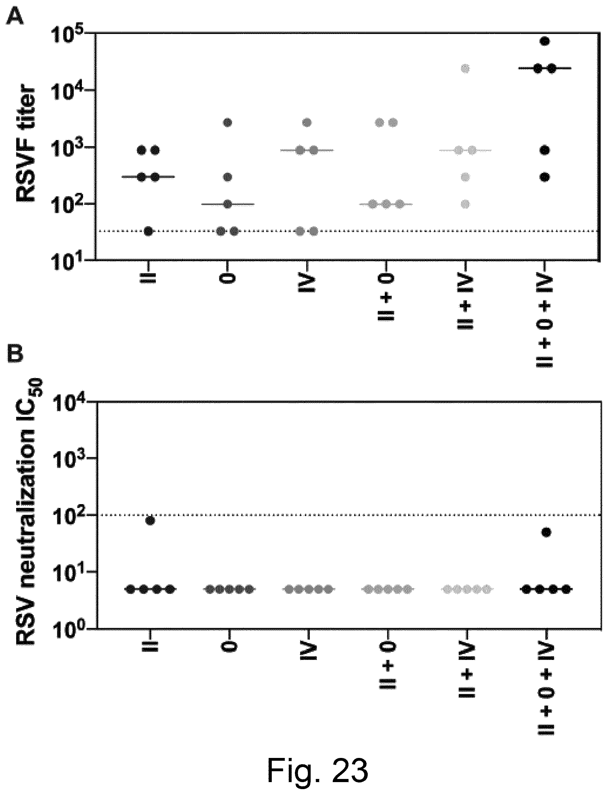

Fig 23 - **Mouse immunization studies with Trivax1.** A) RSVF cross-reactivity of epitope-focused immunogens formulated individually, as cocktail of two, and three (Trivax1). B) RSV neutralizing serum titer of mice immunized with designed immunogens and combinations thereof.

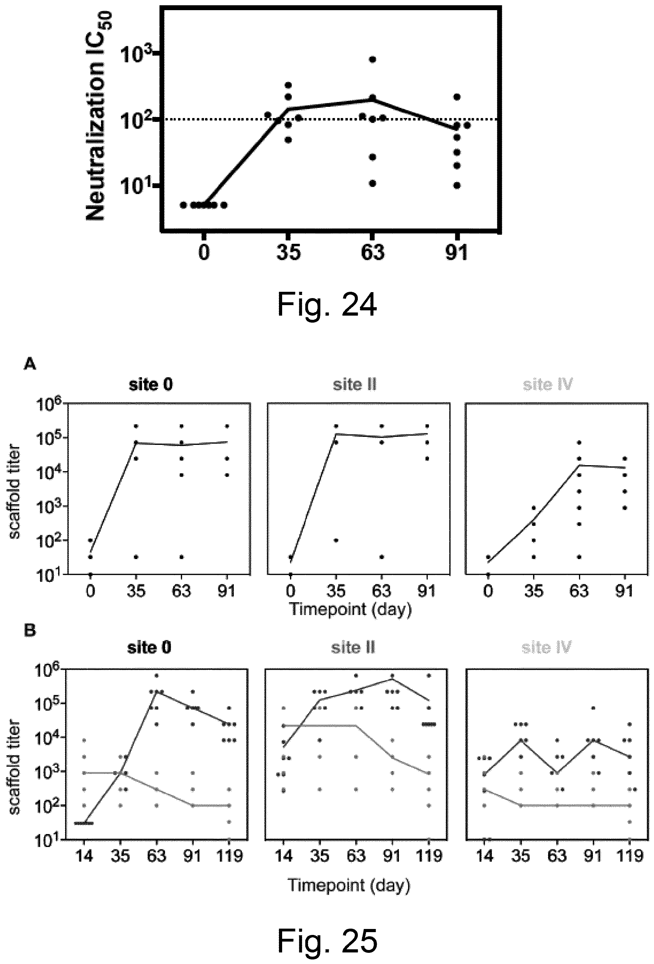

Fig 24 - **Confirmation of NHP neutralization titer by an independent laboratory.** Sera from indicated timepoints were tested for RSV neutralization by an independent laboratory in a different RSV neutralization assay, using a Vero-118 cell line and a GFP readout. See (6) for method details.
Fig 25 - **NHP serum reactivity with designed immunogens.** A) ELISA titer of NHP group 1 (immunized with Trivax1) measured at different timepoints. All animals responded to Trivax1 immunogens at day 91, with site IV immunogen reactivity lower compared to site 0 and site II reactivity. B) ELISA titer of NHP group 2 (grey, RSVF prime) and 3 (blue, RSVF prime, Trivax1 boost) (see Fig 5 for immunization schedule). Following the priming immunization, all animals developed detectable cross-reactivity with the designed immunogens, indicating that the designed scaffolds recognized relevant antibodies primed by RSVF.

### References for legends to Figs 6-25 only:

1. J. S. McLellan et al., Structure of RSV fusion glycoprotein trimer bound to a prefusion specific neutralizing antibody. Science 340, 1113-1117 (2013).
2. M. S. Gilman et al., Rapid profiling of RSV antibody repertoires from the memory B cells of naturally infected adult donors. Sci Immunol 1, (2016).
3. J. S. McLellan et al., Structure of a major antigenic site on the respiratory syncytial virus fusion glycoprotein in complex with neutralizing antibody 101F. J Virol 84, 12236-12244 (2010).
4. J. J. Mousa et al., Human antibody recognition of antigenic site IV on Pneumovirus fusion proteins. PLoS Pathog 14, e1006837 (2018).
5. F. Sesterhenn et al., Boosting subdominant neutralizing antibody responses with a computationally designed epitope-focused immunogen. PLoS Biol 17, e3000164 (2019).
6. E. Olmedillas et al., Chimeric Pneumoviridae fusion proteins as immunogens to induce cross-neutralizing antibody responses. EMBO Mol Med 10, 175-187 (2018).

### DETAILED DESCRIPTION OF THE INVENTION

### De novo design of immunogens with structurally complex epitopes

Designing proteins with structurally complex functional sites has remained a largely unmet challenge on the field of computational protein design. We sought to design accurate mimetics of RSV neutralization epitopes, which have been particularly well studied structurally, and evaluate their functionality in immunization studies. We chose antigenic sites 0 and IV (Fig. 1), which are both targeted by potent nAbs, and are structurally distinct from functional motifs that have previously been handled by computational protein design algorithms. The antigenic site 0 presents a structurally complex and discontinuous epitope consisting of a kinked 17-residue alpha helix and a disordered loop of 7 residues, targeted by nAbs D25 and 5C4 (*12, 14*), while site IV presents an irregular 6-residue bulged beta-strand and is targeted by nAb 101F (*13*).

The computational design of proteins mimicking structural motifs has previously been performed by first identifying compatible protein scaffolds, either from naturally occurring structures or built *de novo,* which then serve as design templates to graft the motif (*5*, *6, 8, 15, 16).* Given the structural complexity of sites 0 and IV, this approach did not yield any promising matches even with loose structural criteria (Fig 6).

Thus, for site IV, we noticed that a small structural domain that resembles an immunoglobulin fold containing the epitope could be excised from the preRSVF structure, hypothesizing this would be a conservative approach to maintain its native, distorted epitope structure (Fig 7). The excised domain did not show a folding funnel in Rosetta *abinitio* simulations, and could not be expressed in *E. coli,* prompting us to perform *in silico* folding and design with Rosetta FunFolDes (*17*) to optimize the sequence for stability and epitope mimicry (Fig 2a). The best computational designs for site IV (S4_1.1) bound with a K_{D} >85 µM to the 101F target antibody. To improve binding affinity, we generated a deep mutational scanning library for selected positions, sorted clones with higher affinity and used next-generation sequencing to identify positions and amino acids that were enriched for high-affinity binding (Fig 7). We tested combinations of enriched positions in recombinantly expressed proteins for antibody binding, obtaining a double mutant (S4_1.5) that bound with a K_{D} of 35 nM to the target antibody, showed a circular dichroism (CD) spectrum corresponding to the secondary structure content designed, and that was thermostable up to 65 °C (Fig 2b-d and Fig 8).

The discontinuous structure of site 0 was not amenable for a domain excision and stabilization approach. We searched for template structures that mimicked the helical segment of the epitope, and simultaneously allowed to graft the loop segment, and selected a designed helical repeat protein as design template (PDB 5cwj) (Fig 2a and Fig 9) (*18*). In order to avoid clashing with the target antibody D25, we truncated the N-terminal 29 residues of the 5cwj template, and performed *in silico* folding and design simulations to perform local and global changes on the scaffold to allow the presentation of the site 0 epitope (Fig 2a). Out of 9 sequences tested, 2 were successfully expressed in *E. coli* and behaved as monomers in solution (Fig 10). The best design, named S0_1.1, bound with a K_{D} of 1.4 µM to the D25 target antibody (Fig 10), which is four orders of magnitude lower than the target affinity (*19*). Following multiple rounds of directed evolution using yeast display, we found a sequence that was C-terminally truncated by 29 residues (S0_1.17), which was enriched and showed greatly increased expression yield, as well as a ∼5-fold increased affinity towards D25 (Fig 9-10). We used the truncated structure as a new template for *in silico* folding and design. Ultimately, this multi-stage process yielded S0_1.39, a design truncated by another 13 residues, which bound with 5 nM to D25 (Fig 2d). S0_1.39 also gained binding to the 5C4 antibody (Fig 10), which was shown to engage site 0 from a different orientation, with an affinity of 5 nM, identical to that of the 5C4-preRSVF interaction *(19).*

The primary goals for the designs were achieved in terms of the stabilization of irregular and complex binding motifs in a conformation relevant for antibody binding, however, the overall strategy presented important limitations with respect to its general utility. Despite the large number of structures available to serve as design templates, the number of those that are practically useful for the design of functional proteins becomes increasingly limited with the structural complexity of the motif. As described above, suboptimal design templates require extensive backbone flexibility on the design process and multiple rounds of directed evolution until a sequence with high-affinity binding is identified. Additionally, the starting topology determines the overall shape of the designed protein, which may be suboptimal for the accurate stabilization of the motif, and may oppose unwanted tertiary steric constraints that interfere with the designed function. In particular, for immunogen design it would be advantageous to preserve native-like accessibility of the epitope to maximize the induction of functional antibodies that can cross-react with the proteins presented by the pathogen. An illustrative example on how a template-based design approach can fail to fulfil these criteria is the comparison between the quaternary environment of the site 0 epitope in preRSVF and S0_1.39 showing that this topology does not mimic such environment, albeit allowing the binding of several monoclonal antibodies (Fig 11).

To overcome these limitations, we developed a template-free design protocol - the TopoBuilder - that generates tailor-made topologies to stabilize complex functional motifs. Within the TopoBuilder, we sample parametrically the placement of idealized secondary structure elements which are then connected by loop segments, to assemble topologies that can stabilize the desired conformation of the structural motif. These topologies are then diversified to enhance structural and sequence diversity with a folding and design stage using Rosetta FunFoldDes (see Fig 12 and methods for full details). For this approach, we defined two new design objectives which were not met by our previous designs using available structural templates: (1) building stable topologies *de novo* that stabilize the epitope, while mimicking its native quaternary environment; (2) Fine-tuning the topology's secondary structure arrangement to maximize the fold stability and optimize epitope presentation for high affinity antibody binding.

To present antigenic site IV, we designed a fold composed of a β-sheet with 4 antiparallel strands and one helix (Fig 3a), referred to as S4_2 fold. Within the S4_2 topology, we generated three distinct structural variants (S4_2_bb1-3), by sampling parametrically three distinct helical secondary structural elements, varying orientations and lengths to maximize the packing interaction against the β-sheet. Sequences generated from 2 out of the 3 structural variants (S4_2_bb2 and S4_2_bb3) showed a strong tendency to recover the designed structures in Rosetta *abinitio* simulations (Fig 3a and Fig 13).

We screened a defined set of computationally designed sequences using yeast display and applied two selective pressures - binding to 101F and resistance to the unspecific protease chymotrypsin, an effective method to digest partially unfolded proteins (*5, 20, 21*). Deep sequencing of populations sorted under different conditions revealed that S4_2_bb2-based designs were strongly enriched under stringent selection conditions for folding and 101F binding, showing that subtle topological differences in the design template can have substantial impact on function and stability. We expressed 15 S4_2_bb2 design variants and successfully purified and biochemically characterized 14. The designs showed mixed alpha/beta CD spectra and bound to 101F with affinities ranging from 1 nM to 1 µM (Fig 14). The best variant, S4_2.45 (K_{D} = 1 nM), was well folded and thermostable according to CD and NMR with a Tₘ of 75 °C (Fig 3d and Fig 20).

Similarly, we built a minimal *de novo* topology to present the tertiary structure of the site 0 epitope. The choice for this topology was motivated by the fact that site 0, in its native environment preRSVF, is accessible for antibody binding from diverse angles (14), in contrast to the S0_39 natural template which topologically constrained site 0 accessibility (Fig 11). By building a template *de novo,* we attempted to mimic the native quaternary constraints and improve the binding affinity to the site 0 specific monoclonal antibodies.

We explored the topological space within the shape constraints of preRSVF and built three different helical orientations that support both epitope segments. Evaluation of the designed sequences with Rosetta *abinitio* showed that only sequences generated based on one of the three topologies (S0_2_bb3) presented a funnel-shaped energy landscape (Fig 16). A set of computationally designed sequences based on S0_2_bb3 was screened in yeast under the selective pressure of two site 0-specific antibodies (D25 and 5C4) to ensure the integrity of the epitope. Deep sequencing of the double-enriched clones and subsequent sequence analysis revealed that a valine at position 28 is critical to retain a cavity formed between the two epitope segments, ensuring binding to both antibodies (Fig 3b).

We selected 5 sequences, differing in 3-21 positions, for further biochemical characterization (Fig 17). The design with best solution behaviour (S0_02.126) showed a CD spectrum of a mostly helical protein, with extremely high thermostability even under reducing conditions (Tₘ = 81 °C, Fig 3d) and a well-dispersed HSQC NMR spectrum (Fig 15). Strikingly, S0_02.126 bound with ∼50 pM affinity to D25, similar to that of the preRSVF-D25 interaction (150 pM), and with a K_{D} = 4 nM to 5C4 (Fig 3e and Fig 18).

Overall, the properties of the designs generated by topological assembly with the TopoBuilder showed improved binding affinities and thermal stabilities as compared to those using available structural templates. To investigate whether this design and screening procedure yielded scaffolds that better mimicked the viral epitope presented, or rather revealed sequences with a highly optimized interface towards the antibodies used during the selection, we determined the affinity of S4_2.45 and S0_2.126 against a panel of site-specific antibodies. Compared to the first-generation designs, S4_2.45 and S0_2.126 showed large affinity improvements to diverse panels of site-specific antibodies, exhibiting a geometric mean affinity closely resembling that of the antibodies to preRSVF (Fig 18). In the light of such results, we concluded that the topologically designed immunogens were improved mimetics of site IV and 0 as compared to template-based designs.

### De novo designed topologies adopt the predicted structures

To evaluate the structural accuracy of the computational design approach, we solved the crystal structure of S4_2.45 in complex with 101F at 2.6 Å resolution. The structure closely matched our design model, with a full-atom RMSD of 1.5 Å. The epitope was mimicked with an RMSD of 0.135 Å, and retained all essential interactions with 101F (Fig 4a). Importantly, the structural data confirmed that we presented an irregular beta strand, a common motif found in many protein-protein interactions (22), in a fully *de novo* designed protein with sub-angstrom accuracy.

Next, we solved an unbound structure of S0_2.126 by NMR, confirming the accuracy of the designed fold with a backbone RMSD between the average structure and the model of 2.8 Å (Fig 4b). Additionally, we solved a crystal structure of S0_2.126 bound to D25 at a resolution of 3.0 Å. The structure showed an overall RMSD of 1.5 Å to the design model, and an RMSD of 0.9 Å over the discontinuous epitope compared to prefusion RSVF (Fig 4c-d). To the best of our knowledge, this is the first computationally *de novo* designed protein that presents a two-segment, structurally irregular, binding motif with atomic-level accuracy. In comparison with native proteins, S0_2.126 showed exceptionally low packing due to a large core cavity (Fig 19), but retained a very high thermal stability. The core cavity was essential for antibody binding and highlights the potential of *de novo* approaches to design small proteins hosting structurally challenging motifs and preserving cavities required for function (2). Notably, due to the level of control and precision of the TopoBuilder, both designed antigens respected the shape constraints of the respective epitope in their native environment preRSVF, a structural feature that may be important for the improved elicitation of functional antibodies (Fig 11).

### Cocktails of designed immunogens elicit neutralizing antibodies in vivo

Lastly, we sought to evaluate the designed antigens for their ability to elicit antibody responses *in vivo.* Our rationale for combining site 0, II and IV immunogens in a cocktail formulation is that all three sites are non-overlapping, as verified by electron microscopy analysis (Fig 20), and thus could induce a broader antibody response *in vivo.* To increase immunogenicity, each immunogen was multimerized on self-assembling protein nanoparticles. We chose the RSV nucleoprotein (RSVN), a self-assembling ring-like structure containing 10-11 subunits, previously been shown to be an effective carrier for the site II immunogen (23), and formulated a trivalent immunogen cocktail containing equimolar amounts of S0_1.39, S4_1.05 and S2_1.2 immunogen nanoparticles ("Trivax1", Fig 21). The fusion of S0_2.126 and S4_2.45 to RSVN yielded poorly soluble nanoparticles, prompting us to use ferritin particles for multimerization, with a 50% occupancy (∼12 copies), creating a second cocktail that contained S2_1.2 in RSVN and the remaining immunogens in ferritin ("Trivax2", Fig 22).

In mice, Trivax1 elicited low levels of RSVF cross-reactive antibodies, and sera did not show RSV neutralizing activity in most animals (Fig 23). In contrast, Trivax2 induced robust levels of RSVF cross-reactive serum levels, and the response was balanced against all three epitopes (Fig 5a,b). Strikingly, Trivax2 immunization yielded RSV neutralizing activity above the protective threshold in 6/10 mice (Fig 5c). Remarkably, these results show that vaccine candidates composed of *de novo* designed proteins mimicking viral neutralization epitopes can induce robust antibody responses *in vivo,* targeting multiple specificities. This is an important finding given that mice have been a traditionally difficult model to induce neutralizing antibodies with scaffold-based design approaches (*11, 15*).

In parallel, we sought to test the potential of a trivalent immunogen cocktail in NHPs. The previously designed site II immunogen showed promise in NHPs, but induced neutralizing titers were low and inconsistent across animals, requiring up to five immunizations to elicit neutralizing antibodies in 2/4 animals (*11*). We immunized seven RSV naïve NHPs with Trivax1, as detailed in Fig 5d. In contrast to mice, NHPs developed robust levels of RSVF cross-reactive serum titer in all animals (Fig 5e), and antibodies induced were directed against all three epitopes (Fig 5f). Strikingly, we found that 6/7 NHPs showed RSV neutralizing serum levels above the protective threshold after a single boosting immunization (mean IC₅₀ = 312) (Fig 5g). Neutralization titers were maximal at day 84 (median IC₅₀ = 408), four-fold above the protective threshold (*19*), and measurements were confirmed by an independent laboratory (Fig 24).

While immunization studies in naïve animals are important to test the designed immunogens, an overarching challenge for vaccine development to target pathogens such as RSV, influenza, dengue and others is to focus or reshape pre-existing immunity of broad specificity on defined neutralizing epitopes that may be of higher-quality and mediate long-term protection (*23*). To mimic a serum response of broad specificity, we immunized 13 NHPs with prefusion RSVF. All animals developed strong preRSVF-specific titers and cross-reactivity with all the epitope-focused immunogens, indicating that epitope-specific antibodies were primed and recognized by the designed immunogens (Fig 25). Group 2 (6 animals) subsequently served as control group to follow the dynamics of epitope-specific antibodies over time, and group 3 (7 animals) was boosted three times with Trivax1 (Fig 5d). PreRSVF-specific antibody and neutralization titers maximized at day 28 and were maintained up to day 119 in both groups (Fig 5h,i). Analysis of the site-specific antibody levels showed that site 0, II and IV responses were dynamic in the control group, with site II dropping from 37% to 13% and site 0 from 17% to 4% at day 28 and 91, respectively (Fig 5j). In contrast, site IV specific responses increased from 13% to 43% over the same time span. Although Trivax1 boosting immunizations did not significantly change the magnitude of the preRSVF-specific serum response, they reshaped the serum specificities in primed animals. Site II specific titers were 6.5-fold higher (day 91) compared to the non-boosted control group (84% vs 13%, p = 0.02, Mann-Whitney), and unlike the rapid drop of site 0-specific antibodies in the non-boosted group, these antibodies were maintained upon Trivax1 boosting (25% vs 4%, p=0.02, Mann-Whitney) (Fig 5j). In contrast, site IV specific responses increased to similar levels in both groups, 43% and 40% in group 2 and 3, respectively. Strikingly, upon depletion of site 0, II and IV specific antibodies from pooled sera, we observed a 60% drop in neutralizing activity in group 3 as compared to only a 7% drop in the non-boosted control group, indicating that Trivax1 boosting reshaped a serum response of broad specificity towards a more focused response that predominantly depends on site 0, II and IV-specific antibodies for RSV neutralization (Fig 5k).

Altogether, we concluded that both design strategies yielded antigens for complex neutralization epitopes that induce neutralizing antibodies upon cocktail formulation, providing a strong rationale for including multiple, ideally non-overlapping epitopes in an epitope-focused vaccination strategy. While the first-generation immunogens were inferior according to biophysical parameters and failed to induce neutralization in mice, but were successful under two different immunological scenarios in NHPs, we show that a second generation with improved biophysical properties and proven accurate mimicry of the epitope can now induce neutralizing antibodies in mice. This is an important step as it now allows to optimize and test the different nanoparticles, formulations and delivery routes in a small animal model, and we foresee that these second-generation immunogens will prove superior in inducing neutralizing serum responses in NHPs.

### Discussion & Conclusions

Here, we have showcased computational protein design strategies to design accurate mimetics of structurally complex epitopes, and validated their functionality to elicit neutralizing antibody responses in cocktail formulations both in mice and NHPs.

We have shown that through computational design of pre-existing templates with full backbone flexibility, irregular and discontinuous epitopes were successfully stabilized in heterologous scaffolds. However, this design strategy required extensive *in vitro* evolution optimization and the resulting scaffolds remained suboptimal regarding their biochemical and biophysical properties. In addition, the lack of precise topological control of the designed proteins is a major limitation for the design of functional proteins that require specific topological similarity on top of the local mimicry of the transplanted site. For instance, the design template of the site 0 immunogen did not mimic the quaternary environment of the epitope of interest, which may have contributed to the low levels of functional antibodies induced in mice. To overcome these limitations, we developed the TopoBuilder, a motif-centric design approach that tailors a protein fold directly to the functional site of interest. Compared to previously employed *de novo* design approaches, in which a stable scaffold topology was constructed first and endowed with binding motifs in a second step (*5*), our method has significant advantages for structurally complex motifs. First, it allows to tailor the topology to the structural requirements of the functional motif from the beginning of the design process, rather than through the adaptation (and often destabilization) of a stable protein to accommodate the functional site. Second, the topological assembly and fine-tuning allowed to select for optimal backbone orientations and sequences that stably folded and bound with high affinity in a single screening round, without requiring further optimization through directed evolution, as often used in computational protein design efforts (*5, 24, 25*). Together, our approach enabled the computational design of *de novo* proteins presenting irregular and discontinuous structural motifs that are typically required to endow proteins with diverse biochemical functions (e.g. binding or catalysis), thus providing a new means for the *de novo* design of functional proteins.

On the functional aspect of our design work, we showed *in vivo* that these immunogens consistently elicited neutralizing serum levels in mice and NHPs as cocktail formulations. The elicitation of focused neutralizing antibody responses by vaccination remains the central goal for vaccines against pathogens that have frustrated conventional vaccine development efforts. Using RSV as a model system, we have shown that cocktails of computationally designed antigens can robustly elicit neutralizing serum levels in naïve animals. These neutralization levels were much superior to any previous report on epitope-focused immunogens (*11*) and provide a strong rationale for an epitope-focused vaccination strategy involving multiple, non-overlapping epitopes. Also, their capability to dramatically reshape the nature of non-naïve repertoires in NHPs, addresses an important challenge for many next-generation vaccines to target pathogens for which efficacious vaccines are needed. An important pathogen from this category is influenza, where the challenge is to overcome established immunodominance hierarchies (*26*) that favour strain-specific antibody specificities, rather than cross-protecting nAbs found in the hemagglutinin stem region (*27*). The ability to selectively boost subdominant nAbs targeting defined, broadly protective epitopes that are surrounded by strain-specific epitopes could overcome a long-standing challenge for vaccine development, given that cross-neutralizing antibodies were shown to persist for years once elicited (*28*). A tantalizing future application for epitope-focused immunogens could marry this technology with engineered components of the immune system and they could be used to stimulate antibody production of adoptively transferred, engineered B-cells that express monoclonal therapeutic antibodies *in vivo* (*29*).

Altogether, this study provides a blueprint for the design of an epitope-focused vaccination strategy against pathogens that have eluded traditional vaccine development approaches. Beyond immunogen design, the design strategy presented opens a door for the *de novo* design of proteins stabilizing complex binding sites, applicable to the design of novel functional proteins with defined structural properties.

### References

1. N. Koga et al., Principles for designing ideal protein structures. Nature 491, 222-227 (2012).
2. E. Marcos et al., Principles for designing proteins with cavities formed by curved beta sheets. Science 355, 201-206 (2017).
3. P. S. Huang et al., De novo design of a four-fold symmetric TIM-barrel protein with atomic-level accuracy. Nat Chem Biol 12, 29-34 (2016).
4. M. Mravic et al., Packing of apolar side chains enables accurate design of highly stable membrane proteins. Science 363, 1418-1423 (2019).
5. A. Chevalier et al., Massively parallel de novo protein design for targeted therapeutics. Nature 550, 74-79 (2017).
6. E. Procko et al., A computationally designed inhibitor of an Epstein-Barr viral Bcl-2 protein induces apoptosis in infected cells. Cell 157, 1644-1656 (2014).
7. S. Berger et al., Computationally designed high specificity inhibitors delineate the roles of BCL2 family proteins in cancer. Elife 5, (2016).
8. M. L. Azoitei et al., Computation-guided backbone grafting of a discontinuous motif onto a protein scaffold. Science 334, 373-376 (2011).
9. S. Jones, J. M. Thornton, Principles of protein-protein interactions. Proc Natl Acad Sci USA 93, 13-20 (1996).
10. N. D. Rubinstein et al., Computational characterization of B-cell epitopes. Mol Immunol 45, 3477-3489 (2008).
11. B. E. Correia et al., Proof of principle for epitope-focused vaccine design. Nature 507, 201-206 (2014).
12. J. S. McLellan et al., Structure of RSV fusion glycoprotein trimer bound to a prefusion-specific neutralizing antibody. Science 340, 1113-1117 (2013).
13. J. S. McLellan et al., Structure of a major antigenic site on the respiratory syncytial virus fusion glycoprotein in complex with neutralizing antibody 101F. J Virol 84, 12236-12244 (2010).
14. D. Tian et al., Structural basis of respiratory syncytial virus subtype-dependent neutralization by an antibody targeting the fusion glycoprotein. Nat Commun 8, 1877 (2017).
15. J. S. McLellan et al., Design and characterization of epitope-scaffold immunogens that present the motavizumab epitope from respiratory syncytial virus. J Mol Biol 409, 853-866 (2011).
16. S. J. Fleishman et al., Computational design of proteins targeting the conserved stem region of influenza hemagglutinin. Science 332, 816-821 (2011).
17. J. Bonet et al., Rosetta FunFolDes - A general framework for the computational design of functional proteins. PLoS Comput Biol 14, el006623 (2018).
18. T. J. Brunette et al., Exploring the repeat protein universe through computational protein design. Nature 528, 580-584 (2015).
19. J. S. McLellan et al., Structure-based design of a fusion glycoprotein vaccine for respiratory syncytial virus. Science 342, 592-598 (2013).
20. P. Kristensen, G. Winter, Proteolytic selection for protein folding using filamentous bacteriophages. Fold Des 3, 321-328 (1998).
21. M. D. Finucane, M. Tuna, J. H. Lees, D. N. Woolfson, Core-directed protein design. I. An experimental method for selecting stable proteins from combinatorial libraries. Biochemistry 38, 11604-11612 (1999).
22. A. M. Watkins, P. S. Arora, Anatomy of beta-strands at protein-protein interfaces. ACS Chem Biol 9, 1747-1754 (2014).
23. F. Sesterhenn et al., Boosting subdominant neutralizing antibody responses with a computationally designed epitope-focused immunogen. PLoS Biol 17, e3000164 (2019).
24. D. A. Silva et al., De novo design of potent and selective mimics of IL-2 and IL-15. Nature 565, 186-191 (2019).
25. E. M. Strauch et al., Computational design of trimeric influenza-neutralizing proteins targeting the hemagglutinin receptor binding site. Nat Biotechnol 35, 667-671 (2017).
26. D. Angeletti et al., Defining B cell immunodominance to viruses. Nat Immunol 18, 456-463 (2017).
27. D. Corti et al., A neutralizing antibody selected from plasma cells that binds to group 1 and group 2 influenza A hemagglutinins. Science 333, 850-856 (2011).
28. J. Lee et al., Persistent Antibody Clonotypes Dominate the Serum Response to Influenza over Multiple Years and Repeated Vaccinations. Cell Host Microbe 25, 367-376 e365 (2019).
29. H. F. Moffett et al., B cells engineered to express pathogen-specific antibodies protect against infection. Sci Immunol 4, (2019).

### METHODS

### Computational design of template-based epitope-focused immunogens

### Site 0

The structural segments entailing the antigenic site 0 were extracted from the prefusion stabilized RSVF Ds-Cav1 crystal structure, bound to the antibody D25 (PDB ID: 4JHW) (1). The epitope consists of two segments: a kinked helical segment (residues 196-212) and a 7-residue loop (residues 63-69).

The MASTER software (2) was used to perform structural searches over the Protein Data Bank (PDB, from August 2018), containing 141,920 protein structures, to select template scaffolds with local structural similarities to the site 0 motif. A first search with a Cα RMSD threshold below 2.5 Å did not produce any usable structural matches both in terms of local mimicry as well as global topology features. A second search was performed, where extra structural elements that support the epitope in its native environment were included as part of the query motif to bias the search towards matches that favoured motif-compatible topologies rather than those with close local similarities. The extra structural elements included were the two buried helices that directly contact the site 0 in the preRSVF structure (4JHW residues 70-88 and 212-229). The search yielded initially 7,600 matches under 5 Å of backbone RMSD, which were subsequently filtered for proteins with a length between 50 and 160 residues, high secondary structure content, as well as for accessibility of the epitope for antibody binding. Remaining matches were manually inspected to select template-scaffolds suitable to present the native conformation of antigenic site 0. Subsequently, we selected a computationally designed, highly stable, helical repeat protein (3) consisting of 8 regular helices (PDB ID: 5CWJ) with an RMSD of 4.4 Å to the query (2.82 Å for site 0 segments only). To avoid steric clashes with the D25 antibody, we truncated the 5CWJ template structure at the N-terminus by 29 residues, resulting in a structural topology composed of 7 helices.

Using Rosetta FunFolDes (4) the truncated 5CWJ topology was folded and designed to stabilize the grafted site 0 epitope recognized by D25. We generated 25,000 designs and selected the top 300 by Rosetta energy score (RE), designed backbones that presented obvious flaws, as low packing scores, distorted secondary structural elements and buried unsatisfied atoms were discarded. From the top 300 designs, 3 were retained for follow-up iterative cycles of structural relaxation and design using Rosetta FastDesign (5), generating a total of 100 designed sequences.

The best 9 designs by Rosetta energy score were recombinantly expressed in *E. coli.* 2 designed sequences derived from the same backbone, were successfully expressed and purified. The best variant was named S0_1.1, and subjected to experimental optimization using yeast surface display (Fig 9-10). In one of the libraries, we found a truncated sequence (S0_1.17) enriched for expression and binding, which served as template for a second round of computational design (Fig 9-10). We performed 25,000 folding and design simulations using Rosetta FunFolDes (4). The best 300 decoys by total Rosetta energy score were extracted, and relaxed using the Rosetta Relax application (6). We computed the mean total RE, and selected designs that showed a lower energy score than the mean of the design population (RE = -155.2), RMSD drift of the epitope after relaxing of less than 0.7 Å, and a cavity volume <60 Å³. We selected one of the best 5 scoring decoys, truncated the N-terminal 14 residues which did not contribute to epitope stabilization, and introduced a disulfide bond between residue 1 and 43. Four sequences were experimentally tested (S0_1.37-40). The best variant according to binding, S0_1.39, bound with 5 nM affinity to antibody D25, and, importantly, also gained binding to the 5C4 antibody (K_{D} = 5 nM).

### Site IV

When the design simulations were carried out, there was no structure available of the full RSVF protein in complex with a site IV-specific nAb, nevertheless a peptide epitope of this site recognized by the 101F nAb had been previously reported (PDB ID: 3041) (7).

The crystallized peptide-epitope corresponds to the residues 429-434 of the RSVF protein. Structurally the 101F-bound peptide-epitope adopts a bulged strand and several studies suggest that 101F recognition extends beyond the linear β-strand, contacting other residues located in antigenic site IV (8). Despite the apparent structural simplicity of the epitope, structural searches for designable scaffolds failed to yield promising starting templates. However, we noticed that the antigenic site IV of RSVF is self-contained within an individual domain that could potentially be excised and designed as a soluble folded protein. To maximize these contacts, we first truncated the seemingly self-contained region from RSVF pre-fusion structure (PDB ID: 4JHW, residue: 402-459) forming a β-sandwich and containing site IV. We used Rosetta FastDesign to optimize the core positions of this minimal topology, obtaining our initial design: S4_wt. However, S4_wt did not show a funnel-shaped energy landscape in Rosetta *ab initio* simulations, and we were unable to obtain expression in *E. coli.*

In an attempt to improve the conformation and stabilization of S4_wt, we used Rosetta FunFolDes to fold and design this topology, while keeping the conformation of the site IV epitope fixed. Out of 25,000 simulations, the top 1 % decoys according to RE score and overall RMSD were selected for manual inspection, and 12 designed sequences were selected for recombinant expression in *E. coli.*

### TopoBuilder - Motif-centric de novo design

Given the limited availability of suitable starting templates to host structurally complex motifs such as site 0 and site IV, we developed a template-free design protocol, which we named TopoBuilder. In contrast to adapting an existing topology to accommodate the epitope, the design goal is to build protein scaffolds around the epitope from scratch, using idealized secondary structures (beta strands and alpha helices). The length, orientation and 3D-positioning are defined by the user for each secondary structure with respect to the epitope, which is extracted from its native environment. The topologies built were designed to meet the following criteria: (1) Small, globular proteins with a high contact order between secondary structures and the epitope, to allow for stable folding and accurate stabilization of the epitope in its native conformation (2) Context mimicry, i.e. respecting shape constraints of the epitope in its native context (Fig 12). For assembling the topology, the default distances between alpha helices was set to 11 Å and for adjacent beta-strands was 5 Å. For each discontinuous structural sketch, a connectivity between the secondary structural elements was defined and loop lengths were selected to connect the secondary structure elements with the minimal number of residues that can cover a given distance, while maintaining proper backbone geometries.

For site 0, the short helix of S0_1.39 preceding the epitope loop segment was kept, and a third helix was placed on the backside of the epitope to: (1) provide a core to the protein and (2) allow for the proper connectivity between the secondary structures.

A total of three different orientations (45°, 0° and -45° degrees to the plane formed by site 0) were tested for the designed supporting alpha helix (Fig. 3 and Fig 16).

In the case of site IV, the known binding region to 101F (residues 428F-434F) was extracted from prefusion RSVF (PDB 4JWH). Three antiparallel beta strands, pairing with the epitope, plus an alpha helix on the buried side, were assembled around the 101F epitope. Three different configurations (45°, (-45°,0°,10°) and -45° degrees with respect to the β-sheet) were sampled parametrically for the alpha helix (Fig. 3).

The structural sketches were used to generate C! distance constraints to guide Rosetta FunFolDes (4) folding trajectories. Around 25,000 trajectories were generated for each sketch. The newly generated backbones were further subjected to layer-based FastDesign (5), meaning that each amino acid position was assigned a layer (combining 'core', 'boundary', 'surface' and 'sheet' or 'helix') on the basis of its exposure and secondary structure type, that dictated the allowed amino acid types at that position.

After iterative cycles of sequence design, unconstraint FastRelax (9) (i.e sidechain repacking and backbone minimization) was applied over the designs to evaluate their conformational stability of the epitope region. After each relax cycle, structural changes of the epitope region were evaluated (epitope RMSD drift). Designs with epitope RMSD drifts higher than 1.2 Å were discarded. Designs were also ranked and selected according hydrophobic core packing (packstat score), with a cutoff of 0.5 for site 0 and 0.6 for the site IV design series, and a cavity volume of < 50 Å³. Between 1,000 and 10,000 of the designed sequences were generated from this computational protocol. We evaluated sequence profiles for the designs, and encoded the critical positions combinatorially by assembling overlapping oligos. Upon PCR assembly, libraries were transformed in yeast and screened for antibody binding and stability as assessed by protease digestion assays (10-12).

### Mouse immunizations

All animal experiments were approved by the Veterinary Authority of the Canton of Vaud (Switzerland) according to Swiss regulations of animal welfare (animal protocol number 3074). Female Balb/c mice (6-week old) were purchased from Janvier labs. Immunogens were thawed on ice, mixed with equal volumes of adjuvant (2% Alhydrogel, Invivogen or Sigma Adjuvant System, Sigma) and incubated for 30 minutes. Mice were injected subcutaneously with 100 µl vaccine formulation, containing in total 10 µg of immunogen (equimolar ratios of each immunogen for Trivax immunizations). Immunizations were performed on day 0, 21 and 42. 100-200 µl blood were drawn on day 0, 14 and 35. Mice were euthanized at day 56 and blood was taken by cardiac puncture.

### NHP immunizations

Twenty-one african green monkeys (AGM, 3-4 years) were divided into three experimental groups with at least two animals of each sex. AGMs were pre-screened as seronegative against prefusion RSVF (preRSVF) by ELISA. Vaccines were prepared 1 hour before injection, containing 50 µg preRSVF or 300 µg Trivax1 in 0.5 ml PBS, mixed with 0.5 ml alum adjuvant (Alhydrogel, Invivogen) for each animal. AGMs were immunized intramuscularly at day 0, 28, 56, and 84. Blood was drawn at days 14, 28, 35, 56, 63, 84, 91, 105 and 119.

### RSV neutralization assay

The RSV neutralization assay was performed as described previously (13). Briefly, Hep2 cells were seeded in Corning 96-well tissue culture plates (Sigma) at a density of 40,000 cells/well in 100 µl of Minimum Essential Medium (MEM, Gibco) supplemented with 10% FBS (Gibco), L-glutamine 2 mM (Gibco) and penicillin-streptomycin (Gibco), and grown overnight at 37 °C with 5% CO2. Serial dilutions of heat-inactivated sera were prepared in MEM without phenol red (M0, Life Technologies, supplemented with 2 mM L-glutamine and penicillin/streptomycin) and were incubated with 800 pfu/well (final MOI 0.01) RSV-Luc (A2 strain carrying a luciferase gene) for 1 hour at 37 °C. Serum-virus mixture was added to Hep2 cell layer, and incubated for 48 hours. Cells were lysed in lysis buffer supplemented with 1 µg/ml luciferin (Sigma) and 2 mM ATP (Sigma), and luminescence signal was read on a Tecan Infinite 500 plate reader. The neutralization curve was plotted and fitted using the GraphPad variable slope fitting model, weighted by 1/Y².

### Serum fractionation

Monomeric Trivax1 immunogens (S2_1, S0_1.39 and S4_1.5) were used to deplete the site 0, II and IV specific antibodies in immunized sera. HisPurTM Ni-NTA resin slurry (Thermo Scientific) was washed with PBS containing 10 mM imidazole. Approximately 1 mg of each immunogen was immobilized on Ni-NTA resin, followed by two wash steps to remove unbound scaffold. 60 µl of sera pooled from all animals within the same group were diluted to a final volume of 600 µl in wash buffer, and incubated overnight at 4 °C with 500 µl Ni-NTA resin slurry. As control, the same amount of sera was incubated with Ni-NTA resin that did not contain scaffolds. Resin was pelleted down at 13,000 rpm for 5 minutes, and the supernatant (depleted sera) was collected and then used for neutralization assays.

### Site saturation mutagenesis library (SSM)

A SSM library was assembled by overhang PCR, in which 11 selected positions surrounding the epitope in the S4_1.1 design model were allowed to mutate to all 20 amino acids, with one mutation allowed at a time. Each of the 11 libraries was assembled by primers (Table 1) containing the degenerate codon 'NNK' at the selected position. All 11 libraries were pooled, and transformed into EBY-100 yeast strain with a transformation efficiency of 1x10⁶ transformants.

### Combinatorial library

Combinatorial sequence libraries were constructed by assembling multiple overlapping primers (Table 2) containing degenerate codons at selected positions for combinatorial sampling of hydrophobic amino acids in the protein core. The theoretical diversity was between 1x10⁶ and 5x10⁶. Primers were mixed (10 µM each), and assembled in a PCR reaction (55 °C annealing for 30 sec, 72 °C extension time for 1 min, 25 cycles). To amplify full-length assembled products, a second PCR reaction was performed, with forward and reverse primers specific for the full-length product. The PCR product was desalted, and transformed into EBY-100 yeast strain with a transformation efficiency of at least 1x10⁷ transformants (14).

### Protein expression and purification

### Designed scaffolds

All genes of designed proteins were purchased as DNA fragments from Twist Bioscience, and cloned via Gibson assembly into either pET11b or pET21b bacterial expression vectors. Plasmids were transformed into E.coli BL21 (DE3) (Merck) and grown overnight in LB media. For protein expression, precultures were diluted 1:100 and grown at 37 °C until the OD₆₀₀ reached 0.6, followed by the addition of 1 mM IPTG to induce expression. Cultures were harvested after 12-16 hours at 22 °C. Pellets were resuspended in lysis buffer (50 mM Tris, pH 7.5, 500 mM NaCl, 5% Glycerol, 1 mg/ml lysozyme, 1 mM PMSF, 1 µg/ml DNase) and sonicated on ice for a total of 12 minutes, in intervals of 15 seconds sonication followed by 45 seconds pause. Lysates were clarified by centrifugation (20,000 rpm, 20 minutes) and purified via Ni-NTA affinity chromatography followed by size exclusion on a HiLoad 16/600 Superdex 75 column (GE Healthcare) in PBS buffer.

### Antibodies - IgG and Fab constructs

Plasmids encoding cDNAs for the heavy chain of IgG were purchased from Genscript and cloned into the pFUSE-CHIg-hG1 vector (Invivogen). Heavy and light chain DNA sequences of antibody fragments (Fab) were purchased from Twist Bioscience and cloned separately into the pHLsec mammalian expression vector (Addgene, #99845) via Gibson assembly. HEK293-F cells were transfected in a 1:1 ratio with heavy and light chains, and cultured in FreeStyle medium (Gibco) for 7 days. Supernatants were collected by centrifugation and purified using a 1 ml HiTrap Protein A HP column (GE Healthcare) for IgG expression and 5 ml kappa-select column (GE Healthcare) for Fab purification. Bound antibodies/Fabs were eluted with 0.1 M glycine buffer (pH 2.7), immediately neutralized by 1 M Tris ethylamine buffer (pH 9), and buffer exchanged to PBS.

### Prefusion stabilized RSVF

The construct encoding the thermostabilized the preRSVF protein corresponds to the sc9-10 DS-Cav1 A149C Y458C S46G E92D S215P K465Q variant (referred to as DS2) reported by Joyce and colleagues (15). The sequence was codon-optimized for mammalian cell expression and cloned into the pHCMV-1 vector flanked with two C-terminal Strep-Tag II and one 8x His tag. Expression and purification were performed as described previously (13).

### Nanoring-based immunogens

The full-length N gene derived from the human RSV strain Long, ATCC VR-26 (GenBank accession number AY911262.1) was PCR amplified and cloned into pET28a+ at Ncol-Xhol sites to obtain the pET-N plasmid. Immunogens presenting sites 0, II and IV epitopes were cloned into the pET-N plasmid at Ncol site as pET-S0_1.39-N, pET-S2_1.2-N and pETS4_1.5-N, respectively. Expression and purification of the nanoring fusion proteins was performed as described previously (13).

### Ferritin-based immunogens

The gene encoding *Helicobacter pylori* ferritin (GenBank ID: QAB33511.1) was cloned into the pHLsec vector for mammalian expression, with an N-terminal 6x His Tag. The sequence of the designed immunogens (S0_2.126 and S4_2.45) were cloned upstream of the ferritin gene, spaced by a GGGGS linker. Ferritin particulate immunogens were produced by co-transfecting a 1:1 stochiometric ratio of "naked" ferritin and immunogen-ferritin in HEK-293F cells, as previously described for other immunogen-nanoparticle fusion constructs (16). The supernatant was collected 7-days post transfection and purified via Ni-NTA affinity chromatography and size exclusion on a Superose 6 increase 10/300 GL column (GE).

### Negative-stain transmission electron microscopy

### Sample preparation

RSVN and Ferritin- based nanoparticles were diluted to a concentration of 0.015 mg/ml. The samples were absorbed on carbon-coated copper grid (EMS, Hatfield, PA, United States) for 3 mins, washed with deionized water and stained with freshly prepared 0.75 % uranyl formate.

### Data acquisition

The samples were viewed under an F20 electron microscope (Thermo Fisher) operated at 200 kV. Digital images were collected using a direct detector camera Falcon III (Thermo Fisher) with the set-up of 4098 X 4098 pixels. The homogeneity and coverage of staining samples on the grid was first visualized at low magnification mode before automatic data collection. Automatic data collection was performed using EPU software (Thermo Fisher) at a nominal magnification of 50,000X, corresponding to pixel size of 2 Å, and defocus range from -1 µm to -2 µm.

### Image processing

CTFFIND4 program (17) was used to estimate the contrast transfer function for each collected image. Around 1000 particles were manually selected using the installed package XMIPP within SCIPION framework (18). Manually picked particles were served as input for XMIPP auto-picking utility, resulting in at least 10,000 particles. Selected particles were extracted with the box size of 100 pixels and subjected for three rounds of reference-free 2D classification without CTF correction using RELION-3.0 Beta suite (19).

### RSVF-Fabs complex formation and negative stain EM

20 µg of RSVF trimer was incubated overnight at 4°C with 80 µg of Fabs (Motavizumab, D25 or 101F). For complex formation with all three monoclonal Fabs, 80 µg of each Fab was used. Complexes were purified on a Superose 6 Increase 10/300 column using an Äkta Pure system (GE Healthcare) in TBS buffer. The main fraction containing the complex was directly used for negative stain EM. Purified complexes of RSVF and Fabs were deposited at approximately 0.02 mg/ml onto carbon-coated copper grids and stained with 2% uranyl formate. Images were collected with a field-emission FEI Tecnai F20 electron microscope operating at 200 kV. Images were acquired with an Orius charge-coupled device (CCD) camera (Gatan Inc.) at a calibrated magnification of x34,483, resulting in a pixel size of 2.71 Å. For the complexes of RSVF with a single Fab, approximately 2,000 particles were manually selected with Cryosparc2 (20). Two rounds of 2D classification of particle images were performed with 20 classes allowed. For the complexes of RSVF with D25, Motavizumab and 101F Fabs, approximately 330,000 particles were picked using Relion 3.0 (19) and subsequently imported to Cryosparc2 for two rounds of 2D classification with 50 classes allowed.

### Determining binding affinities by Surface plasmon resonance (SPR)

SPR measurements were performed on a Biacore 8K (GE Healthcare) with HBS-EP+ as running buffer (10 mM HEPES pH 7.4, 150 mM NaCl, 3 mM EDTA, 0.005% v/v Surfactant P20, GE Healthcare). Ligands were immobilized on a CM5 chip (GE Healthcare # 29104988) via amine coupling. Approximately 2000 response units (RU) of IgG were immobilized, and designed monomeric proteins were injected as analyte in two-fold serial dilutions. The flow rate was 30 µl/min for a contact time of 120 seconds followed by 400 seconds dissociation time. After each injection, surface was regenerated using 3 M magnesium chloride (101F as immobilized ligand) or 0.1 M Glycine at pH 4.0 (Motavizumab and D25 IgG as an immobilized ligand). Data were fitted using 1:1 Langmuir binding model within the Biacore 8K analysis software (GE Healthcare #29310604).

### Dissection of serum antibody specificities by SPR

To quantify the epitope-specific antibody responses in bulk serum from immunized animals, we performed an SPR competition assay with the monoclonal antibodies (D25, Motavizumab and 101F) as described previously (13). Briefly, approximately 400 RU of prefusion RSVF were immobilized on a CM5 chip via amine coupling, and serum diluted 1:10 in running buffer was injected to measure the total response ((RU_{non-blocked surface}). After chip regeneration using 50 mM NaOH, the site 0/II/IV epitopes were blocked by injecting saturating amounts of either D25, Motavizumab, or 101F IgG, and serum was injected again to quantify residual response (RU_{blocked surface}). The delta serum response (Δ *SR*) was calculated as follows: $Δ SR = {RU}_{\left(non-\right) blocked surface} - {RU}_{Baseline}$

Percent blocking was calculated for each site as: $% blocking = \left(1-\left(\frac{{Δ SR}_{blocked surface}}{{Δ SR}_{non - blocked surface}}\right)\right) ∗ 100$

### SEC-MALS

Size exclusion chromatography with an online multi-angle light scattering (MALS) device (miniDAWN TREOS, Wyatt) was used to determine the oligomeric state and molecular weight for the protein in solution. Purified proteins were concentrated to 1 mg/ml in PBS (pH 7.4), and 100 µl of sample was injected into a Superdex 75 300/10 GL column (GE Healthcare) with a flow rate of 0.5 ml/min, and UV280 and light scattering signals were recorded. Molecular weight was determined using the ASTRA software (version 6.1, Wyatt).

### Circular Dichroism

Far-UV circular dichroism spectra were measured using a Jasco-815 spectrometer in a 1 mm path-length cuvette. The protein samples were prepared in 10 mM sodium phosphate buffer at a protein concentration of 30 µM. Wavelengths between 190 nm and 250 nm were recorded with a scanning speed of 20 nm min⁻¹ and a response time of 0.125 sec. All spectra were averaged 2 times and corrected for buffer absorption. Temperature ramping melts were performed from 25 to 90 °C with an increment of 2 °C/min in presence or absence of 2.5 mM TCEP reducing agent. Thermal denaturation curves were plotted by the change of ellipticity at the global curve minimum to calculate the melting temperature (Tₘ).

### Yeast surface display

Libraries of linear DNA fragments encoding variants of the designed proteins were transformed together with linearized pCTcon2 vector (Addgene #41843) based on the protocol previously described by Chao and colleagues (14). Transformation procedures generally yielded ∼10⁷ transformants. The transformed cells were passaged twice in SDCAA medium before induction. To induce cell surface expression, cells were centrifuged at 7,000 r.p.m. for 1 min, washed with induction media (SGCAA) and resuspended in 100 ml SGCAA with a cell density of 1 x 10⁷ cells/ml SGCAA. Cells were grown overnight at 30 °C in SGCAA medium. Induced cells were washed in cold wash buffer (PBS + 0.05% BSA) and labelled with various concentration of target IgG or Fab (101F, D25, and 5C4) at 4°C. After one hour of incubation, cells were washed twice with wash buffer and then incubated with FITC-conjugated anti-cMyc antibody and PE-conjugated anti-human Fc (BioLegend, #342303) or PE-conjugated anti-Fab (Thermo Scientific, #MA1-10377) for an additional 30 min. Cells were washed and sorted using a SONY SH800 flow cytometer in 'ultra-purity' mode. The sorted cells were recovered in SDCAA medium, and grown for 1-2 days at 30 °C. In order to select stably folded proteins, we washed the induced cells with TBS buffer (20 mM Tris, 100 mM NaCl, pH 8.0) three times and resuspended in 0.5 ml of TBS buffer containing 1 µM of chymotrypsin. After incubating five-minutes at 30°C, the reaction was quenched by adding 1 ml of wash buffer, followed by five wash steps. Cells were then labelled with primary and secondary antibodies as described above.

### ELISA

96-well plates (Nunc MediSorp platesf Thermo Scientific) were coated overnight at 4°C with 50 ng/well of purified antigen (recombinant RSVF or designed immunogen) in coating buffer (100 mM sodium bicarbonate, pH 9) in 100 µl total volume. Following overnight incubation, wells were blocked with blocking buffer (PBS + 0.05% Tween 20 (PBST) containing 5% skim milk (Sigma)) for 2 hours at room temperature. Plates were washed five times with PBST. 3-fold serial dilutions were prepared and added to the plates in duplicates, and incubated at room temperature for 1 hour. After washing, anti-mouse (abcam, #99617) or anti-monkey (abcam, #112767) HRP-conjugated secondary antibody were diluted 1:1,500 or 1:10,000, respectively, in blocking buffer and incubated for 1 hour. An additional five wash steps were performed before adding 100 µl/well Pierce TMB substrate (Thermo Scientific). The reaction was terminated by adding an equal volume of 2 M sulfuric acid. The absorbance at 450 nm was measured on a Tecan Safire 2 plate reader, and the antigen specific titers were determined as the reciprocal of the serum dilution yielding a signal two-fold above the background.

### NMR

Protein samples for NMR were prepared in 10 mM sodium phosphate buffer, 50 mM sodium chloride at pH 7.4 with the protein concentration of 500 µM. All NMR experiments were carried out in a 18.8T (800 MHz proton Larmor frequency) Bruker spectrometer equipped with a CPTC 1H,¹³C,¹⁵N 5 mm cryoprobe and an Avance III console. Experiments for backbone resonance assignment consisted in standard triple resonance spectra HNCA, HN(CO)CA, HNCO, HN(CO)CA, CBCA(CO)NH and HNCACB acquired on a 0.5 mM sample doubly labelled with ¹³C and ¹⁵N (21). Sidechain assignments were obtained from HCCH-TOCSY experiments acquired on the same sample plus HNHA, NOESY-¹⁵N-HSQC and TOCSY-¹⁵N-HSQC acquired on a ¹⁵N-labeled sample. The NOESY-¹⁵N-HSQC was used together with a 2D NOESY collected on an unlabelled sample for structure calculations. Spectra for backbone assignments were acquired with 40 increments in the ¹⁵N dimension and 128 increments in the ¹³C dimension, and processed with 128 and 256 points by using linear prediction. HCCH-TOCSY were recorded with 64-128 increments in the ¹³C dimensions and processed with twice the number of points. ¹⁵N-resolved NOESY and TOCSY spectra were acquired with 64 increments in ¹⁵N dimension and 128 in the indirect ¹H dimension, and processed with twice the number of points. ¹H-¹H 2D-NOESY and 2D TOCSY spectra were acquired with 256 increments in the indirect dimension, processed with 512 points. Mixing times for NOESY spectra were 100 ms and TOCSY spin locks were 60 ms. Heteronuclear ¹H-¹⁵N NOE was measured with 128 ¹⁵N increments processed with 256 points, using 64 scans and a saturation time of 6 seconds. All samples were prepared in 20 mM phosphate buffer pH 7, with 10% ²H₂O and 0.2% sodium azide to prevent sample degradation.

All spectra were acquired and processed with Bruker's TopSpin 3.0 (acquisition with standard pulse programs) and analyzed manually with the program CARA (http://cara.nmr.ch/doku.php/home) to obtain backbone and sidechain resonance assignments. Peak picking and assignment of NOESY spectra (a ¹⁵N-resolved NOESY and a 2D NOESY) were performed automatically with the program UNIO-ATNOS/CANDID (22, 23) coupled to Cyana 2.1 (24), using standard settings in both programs. The run was complemented with dihedral angles derived from chemical shifts with Talos-n (25).

### X-ray crystallization and structural determination

### Co-crystallization of complex D25 Fab with S0 2.126

After overnight incubation at 4°C, the S0_2.126/D25 Fab complex was purified by size exclusion chromatography using a Superdex200 26 600 (GE Healthcare) equilibrated in 10 mM Tris pH 8, 100 mM NaCl and subsequently concentrated to ∼10 mg/ml (Amicon Ultra-15, MWCO 3,000). Crystals were grown at 291K using the sitting-drop vapor-diffusion method in drops containing 1 µl purified protein mixed with 1 µl reservoir solution containing 10% PEG 8000, 100 mM HEPES pH 7.5, and 200 mM calcium acetate.

For cryo protection, crystals were briefly swished through mother liquor containing 20% ethylene glycol.

### Data collection and structural determination of the S0 2.126/D25 Fab complex

Diffraction data was recorded at ESRF beamline ID30B. Data integration was performed by XDS (26) and a high-resolution cut at l/σ=1 was applied. The dataset contained a strong off-origin peak in the Patterson function (88% height rel. to origin) corresponding to a pseudo translational symmetry of 1/2, 0, 1/2. The structure was determined by the molecular replacement method using PHASER (27) using the D25 structure (1) (PDB ID 4JHW) as a search model. Manual model building was performed using Coot (28), and automated refinement in Phenix (29). After several rounds of automated refinement and manual building, paired refinement (30) determined the resolution cut-off for final refinement.

### Co-crystallization of complex 101F Fab with S4 2.45

The complex of S4_2.45 with the F101 Fab was prepared by mixing two proteins in 2:1 molar ratio for 1 hour at 4 °C, followed by size exclusion chromatography using a Superdex-75 column. Complexes of S4_2.45 with the 101F Fab were verified by SDS-PAGE. Complexes were subsequently concentrated to 6-8 mg/ml. Crystals were grown using hanging drops vapor-diffusion method at 20 °C. The S4_2.45/101F protein complex was mixed with equal volume of a well solution containing 0.2 M Magnesium acetate, 0.1 M Sodium cacodylate pH 6.5, 20 %(w/v) PEG 8000. Native crystals were transferred to a cryoprotectant solution of 0.2 M Magnesium acetate, 0.1 M Sodium cacodylate pH 6.5, 20 % (w/v) PEG 8000 and 15% glycerol, followed by flash-cooling in liquid nitrogen.

### Data collection and structural determination of the S4 2.45/101F Fab complex

Diffraction data were collected at SSRL facility, BL9-2 beamline at the SLAC National Accelerator Laboratory. The crystals belonged to space group P3221. The diffraction data were initially processed to 2.6 Å with X-ray Detector Software (XDS) (Table 9). Molecular replacement searches were conducted with the program PHENIX PHASER using 101F Fab model (PDB ID: 3041) and S4_2.45/101F Fab computational model generated from superimposing epitope region of S4_2.45 with the peptide-bound structure (PDB ID: 3041), and yielded clear molecular replacement solutions. Initial refinement provided a R_{free} of 42.43% and R_{work} of 32.25% and a complex structure was refined using Phenix Refine, followed by manual rebuilding with the program COOT. The final refinement statistics, native data and phasing statistics are summarized in Table 9.

### Next-generation sequencing of design pools

After sorting, yeast cells were grown overnight, pelleted and plasmid DNA was extracted using Zymoprep Yeast Plasmid Miniprep II (Zymo Research) following the manufacturer's instructions. The coding sequence of the designed variants was amplified using vector-specific primer pairs, Illumina sequencing adapters were attached using overhang PCR, and PCR products were desalted (Qiaquick PCR purification kit, Qiagen). Next generation sequencing was performed using an Illumina MiSeq 2x150bp paired end sequencing (300 cycles), yielding between 0.45-0.58 million reads/sample.

For bioinformatic analysis, sequences were translated in the correct reading frame, and enrichment values were computed for each sequence. We defined the enrichment value E as follows: ${E}_{Seq} = \frac{{count}_{Seq} \left(high selective pressure\right)}{{count}_{Seq} \left(low selective pressure\right)}$

The high selective pressure corresponds to low labelling concentration of the respective target antibodies (100 pM D25, 10 nM 5C4 or 20 pM 101F, as shown in Fig. 3), or a higher concentration of chymotrypsin protease (0.5 µM). The low selective pressure corresponds to a high labelling concentration with antibodies (10 nM D25, 1 µM 5C4 or 2 nM 101F), or no protease digestion, as indicated in Fig. 3. Only sequences that had at least one count in both sorting conditions were included in the analysis.

### Tables.

**Table 1 - Primers used for constructing single>site saturation mutagenesis library for S4_1 design.**

| | |
|---|---|
| S4_1_SSM_fw (SEQ ID NO : 2) | CAGGCTAGTGGTGGAGGAGGCTCTGGTGGAGGCGGTAGCGGAGGC GGAGGGTCGGCTAGC |
| S4_1_SSM_rw (SEQ ID NO : 3) | CTGTTGTTATCAGATCTCGAGCTATTACAAGTCCTCTTCAGAAATA AGCTTTTGTTCGGATCC |
| S4_1_#18_rw (SEQ ID NO : 4) | TTTCGGGCATTTGACTTTGATACCATTGCTGT |
| S4_1_#18_fw (SEQ ID NO : 5) | CAATGGTATCAAAGTCAAATGCCCGAAANNKGGTGAATGTACGAT TAAAGACAGTCAACG |
| S4_1_#20_rw (SEQ ID NO : 6) | CTTTCGGGCATTTGACTTTGATACCATTGCTGT |
| S4_1_#20_fw (SEQ ID NO : 7) | GCAATGGTATCAAAGTCAAATGCCCGAAAGGCGGTNNKTGTACGA TTAAAGACAGTCAACGTGG |
| S4_1_#22_rw (SEQ ID NO : 8) | CCTTTCGGGCATTTGACTTTGATACCATTGCTGT |
| S4_1_#22_fw (SEQ ID NO : 9) | GCAATGGTATCAAAGTCAAATGCCCGAAAGGCGGTGAATGTNNKA TTAAAGACAGTCAACGTGGCATTATC |
| S4 1 #25 rw (SEQ ID NO : 10) | TTTAATCGTACATTCACCGCCTTTCG |
| S4_1_#25_fw (SEQ ID NO : 11) | CGAAAGGCGGTGAATGTACGATTAAANNKAGTCAACGTGGCATTA TCAAAACC |
| S4_1_#36_rw (SEQ ID NO : 12) | GCTAAAGGTTTTGATAATGCCACGTTGAC |
| S4_1_#36_fw (SEQ ID NO : 13) | CAACGTGGCATTATCAAAACCTTTAGCNNKGGTACGGAAGAAGTT CGCAGTC |
| S4 1 #39 rw (SEQ ID NO : 14) | CGTACCAGAGCTAAAGGTTTTGATAATGCCA |
| S4_1_#39_fw (SEQ ID NO : 15) | GCATTATCAAAACCTTTAGCTCTGGTACGNNKGAAGTTCGCAGTCC GTCCCTG |
| S4 1 #43 rw (SEQ ID NO : 16) | GCGAACTTCTTCCGTACCAGAGCTAAAG |
| S4_1_#43_fw (SEQ ID NO : 17) | GCTCTGGTACGGAAGAAGTTCGCNNKCCGTCCCTGGGCAAAGTGA CCGT |
| S4_1_#45_fw (SEQ ID NO : 18) | GCTCTGGTACGGAAGAAGTTCGCAGTCCGNNKCTGGGCAAAGTGA CCGTTGGTGATAAC |
| S4 1 #48 rw (SEQ ID NO : 19) | GCCCAGGGACGGACTGCGAACTTC |
| S4_1_#48_fw (SEQ ID NO : 20) | GTTCGCAGTCCGTCCCTGGGCNNKGTGACCGTTGGTGATAACACGT TC |
| S4_1_#50_fw (SEQ ID NO : 21) | GTTCGCAGTCCGTCCCTGGGCAAAGTGNNKGTTGGTGATAACACGT TCGAAGCG |

**Table 2 - Primers used for encoding computationally designed sequences of S4_2 design series.**

| | |
|---|---|
| S4_2_uni_O1 (SEQ ID NO : 22) | GACAATAGCTCGACGATTGAAGGTAGATACCCATACGACGTTCCA GACTACGCTCTGCAGGCTAGTGGTGGAGGAGG |
| S4_2_uni_O2 (SEQ ID NO : 23) | CCCTCCGCCTCCGCTACCGCCTCCACCAGAGCCTCCTCCACCACTA GCCTG |
| S4_2_bb1_O3.1 (SEQ ID NO : 24) | GTAGCGGAGGCGGAGGGTCGGCTAGCCATATGCCGTCCATCYACT CAKWCGTTSYTGGTGGGAACATCAAGGTGAAGTGC |
| S4_2_bb1_O3.2 (SEQ ID NO : 25) | GTAGCGGAGGCGGAGGGTCGGCTAGCCATATGCCGTCCATCYACT CAKWCGTTSYTGGGAACATCAAGGTGAAGTGC |
| S4_2_bb1_O3.3 (SEQ ID NO : 26) | GTAGCGGAGGCGGAGGGTCGGCTAGCCATATGCCGTCCATCYACT CAKWCGTTSYTAACGGGAACATCAAGGTGAAGTGC |
| S4_2_bb1_O4.1 (SEQ ID NO : 27) | GGTCTTGATGATGCCACGCTGGCTATCCTCGATGGTACATTTGTCA CCAGTGCACTTCACCTTGATGTTCCC |
| S4_2_bb1_O4.2 (SEQ ID NO : 28) | GGTCTTGATGATGCCACGATTCTTGTTCTCGATGGTACATTTGTCAC CAGTGCACTTCACCTTGATGTTCCC |
| S4_2_bb1_O4.3 (SEQ ID NO : 29) | GGTCTTGATGATGCCACGCTGGCTATCCTCGATGGTACACTTGCCC TCGTGGCACTTCACCTTGATGTTCCC |
| S4_2_bb1_O5.1 (SEQ ID NO : 30) | GCGTGGCATCATCAAGACCACGAATGTTGATATTGCTGAGGAGRY GYRGAAGCAGSYTCAAGAGBYTBWGGAAGMGAAACGTAAGGGCT CGTGGGGCTCG |
| S4_2_bb1_O5.2 (SEQ ID NO : 31) | GCGTGGCATCATCAAGACCTTCACGGGGTTCGAGCCCGAGGAGRY GYRGAAGCAGSYTCAAGAGBYTBWGGAAGMGAAACGTAAGGGCT CGTGGGGCTCG |
| S4_2_bb1_O5.3 (SEQ ID NO : 32) | GCGTGGCATCATCAAGACCGTCCCGATGATCGAGACAGGGGAGGA GRYGYRGAAGCAGSYTCAAGAGBYTBWGGAAGMGAAACGTGGCT CGTGGGGCTCG |
| S4_2_uni_O6 (SEQ ID NO : 33) | CAGAAATAAGCTTTTGTTCGGATCCGGGCTCAGCCTATTAGTGGTG GTGGTGGTGGTGCGAGCCCCACGAGCC |
| S4_2_uni_O7 (SEQ ID NO : 34) | GGATCCGAACAAAAGCTTATTTCTGAAGAGGACTTGTAATAGCTCG AGATCTGATAAC |
| S4_2_uni_O8 (SEQ ID NO : 35) | GTACGAGCTAAAAGTACAGTGGGAACAAAGTCGATTTTGTTACAT CTACACTGTTGTTATCAGATCTCGAGCTATTACAAGTCC |
| S4_2_bb2_O3.1 (SEQ ID NO : 36) | TAGCGGAGGCGGAGGGTCGGCTAGCCATATGCCAAAWACCHACGT AWTTGAAGCAGGCDTCAGCTTCACCTGCTTAGGTGAGAAGTGCAC CATCGAGGAC |
| S4_2_bb2_O3.2 (SEQ ID NO : 37) | TAGCGGAGGCGGAGGGTCGGCTAGCCATATGCCAAAWACCHACGT AWTTCCCTCGDTCAGCTTCACCTGCTTAGGTGAGAAGTGCACCATC GAGGAC |
| S4_2_bb2_O3.3 (SEQ ID NO : 38) | TAGCGGAGGCGGAGGGTCGGCTAGCCATATGCCAAAWACCHACGT AWTTCCCTCGDTCAGCTTCACCTGCCCTAAGGGGGGAAGTGCAC CATCGAGGAC |
| S4_2_bb2_O4.1 (SEQ ID NO : 39) | CGGTCTTGATGATCCCACGTTGTGAGTCCTCGATGGTGCACTTC |
| S4_2_bb2_O4.2 (SEQ ID NO : 40) | CGGTCTTGATGATCCCACGATCGTCCTCGATGGTGCACTTC |
| S4_2_bb2_O4.3 (SEQ ID NO : 41) | CGGTCTTGATGATCCCACGCGAGCGGTCCTCGATGGTGCACTTC |
| S4_2_bb2_O5.1 (SEQ ID NO : 42) | CGTGGGATCATCAAGACCGGCAAAAATGCCGAGGAGKYCDKGGA AGATBTCGAGAAGVRGGHGCGTGCCCAGGGCTCGTGGGGCTCGCA C |
| S4_2_bb2_O5.2 (SEQ ID NO : 43) | CGTGGGATCATCAAGACCGGCACACATCCAGAGGAGKYCDKGGAA GATBTCGAGAAGVRGGHGCGTGCCCAGGGCTCGTGGGGCTCGCAC |
| S4_2_bb2_O5.3 (SEQ ID NO : 44) | CGTGGGATCATCAAGACCGGCAAAAATAAGGAGGAGKYCDKGGA AGATBTCGAGAAGVRGGHGCGTGCCCAGGGCTCGTGGGGCTCGCA C |
| S4_2_bb3_O3.1 (SEQ ID NO : 45) | TAGCGGAGGCGGAGGGTCGGCTAGCCATATGGTCTKSAGTKKTGT AGYTGGGGAGAACTATTCARYTAAGTGTACTGGCGACAAGTGCAC CATCGAGGAC |
| S4_2_bb3_O3.2 (SEQ ID NO : 46) | TAGCGGAGGCGGAGGGTCGGCTAGCCATATGGTCTKSAGTKKTGT AGYTACCCCGACATTTTCARYTAAGTGTACTGGCGACAAGTGCACC ATCGAGGAC |
| S4_2_bb3_O3.3 (SEQ ID NO : 47) | TAGCGGAGGCGGAGGGTCGGCTAGCCATATGTKSAGTKKTGTAGY TGGGGAGAACTATTCARYTAAGTGTCCTAAGGGGGCAAGTGCAC CATCGAGGAC |
| S4 2 bb3 04.1 (SEQ ID NO : 48) | GGTCTTGATGATCCCGCGCTGTGAGTCCTCGATGGTGCACTTG |
| S4 2 bb3 O4.2 (SEQ ID NO : 49) | GGTCTTGATGATCCCGCGATTCTTGTCCTCGATGGTGCACTTG |
| S4 2 bb3 O4.3 (SEQ ID NO : 50) | GGTCTTGATGATCCCGCGCCCGCCATAGTCCTCGATGGTGCACTTG |
| S4_2_bb3_O5.1 (SEQ ID NO : 51) | CGCGGGATCATCAAGACCACGATTGGAGATACATGTGAGSHGKYG KMTAAGGCGGYTCAAAAGGCTSVGAAAGGCTCGTGGGGCTCG |
| S4_2_bb3_O5.2 (SEQ ID NO : 52) | CGCGGGATCATCAAGACCGTTACTGGCAGTCGCTGTGAGSHGKYG KMTAAGGCGGYTCAAAAGGCTSVGAAAGGCTCGTGGGGCTCG |

**Table 3 - Computationally designed protein sequences for S4_1 design series.**

| Design name | Sequence | Expression vector |
|---|---|---|
| S4_1.1 (SEQ ID NO : 53) | MDGTLQINSNGIKVKCPKGGECTIKDSQRGIIKTFSSGTEEVRSPS LGKVTVGDNTFEASNGSWLEHHHHHH | pET21b |
| S4_1.2 (SEQ ID NO : 54) | MHHHHHHWGSPGTVTLNSNGLTVTGNDNYNLTVTGNDRGIIKT FSPSTETTNDDGMSTITVGNLTVTLGN | pET11b |
| S4_1.3 (SEQ ID NO : 55) | MHHHHHHWGSQSTVNVQDKNIRIEVDDKNSVQVNGSNRGIIKTF SPGTVQISSKNGDTVTGNVRVNMGG | pET11b |
| S4_1.4 (SEQ ID NO : 56) | MHHHHHHWGSQSTVNVQDKNIRIECDDNCGVQVNGSNRGIIKT FSPGTVQISSKNGDTVTGNVRVNMGG | pET11b |
| S4_1.5 (SEQ ID NO : 57) | MHHHHHHWGSDGTLQINSHGVKVKAPPGSGATVKDSQRGIIKTF SSGYEEVRSPSLGKVTVGDNTFEVSN | pET11b |
| S4_1.6 (SEQ ID NO : 58) | MHHHHHHWGSDGTLQINSHGVKVKCPKGSECTVKDSQRGIIKTF SSGYEEVRSPSLGKVTVGDNTFEVSN | pET11b |
| S4_1.7 (SEQ ID NO : 59) | MHHHHHHGSKVTFRQDKNGIKIRVNGNKGLVIRTNDRGIIKTFS NGTYDIPNSGYNRFTVGGTQFDWNE | pET11b |
| S4_1.8 (SEQ ID NO : 60) | MHHHHHHGSKVTFRQDKNGIKFRVNGNKGAVIRTNDRGIIKTFS NGTYDIPNSGYNRFTVGGNTFDWNE | pET11b |
| S4_1.9 (SEQ ID NO : 61) | MDGTLQINSNGVKVKCPKGVECTVKDSQRGIIKTFSSGTEEVRSP SLGKVTVGDNTFEVSNGSWLEHHHHHH | pET21b |
| S4_1.10 (SEQ ID NO : 62) | MDGTLQINSNGVKVKCPKGAECTVKVSQRGIIKTFSSGTEEVRSP SLGKVTVGDNTTEVSNGSWLEHHHHHH | pET21b |
| S4_1.11 (SEQ ID NO : 63) | MHHHHHHWGSPGTVKLNSNGLTVRGNDSYGLTVRGNDRGIIKT FSPSTEVVQSKGMSTITVGNLDVRLGE | pET11b |

**Table 4 - Computationally designed protein sequences for S0_1 design series.**

| Design name | Sequence | Expression vector |
|---|---|---|
| S0_1.1 (SEQ ID NO : 64) | PEDAQKEASKGSEVRELKNIIDKQLLPIVNKTSCSGAEQAAEAL REALEGAGSCDAVEQLLGNIKEIKCGTDAGRALIRILAEVAREI GCPRAIDQVAEWVRRIAKAVGGEEAKKQVKEVEKEIRREKG | pET21 |
| S0_1.17 (SEQ ID NO : 65) | PEDAQKEASKGSEVRELKNIIDKQLLPILNKASCSGAEQLLEAL REALEGAGSCDAVEQLLGNIKEIKCGTDAGRALKRILEEVQREI GCGSW | pET21 |
| S0_1.37 (SEQ ID NO : 66) | CDQLKNYIDKQLLPIVNKQSCANGEEALKDIEKALRGAGSKDC WKELLSNIKEIKCGKEFAEKLKKEWERIKKEAGD | pET21 |
| S0_1.38 (SEQ ID NO : 67) | CDQIKNYIDKQLLPIVNKAGCGSAEEALKDIEKALRLAGSKDCL KEIFSNIKEIKCGKEFAEKLKKEWERIKKEAGD | pET21 |
| S0_1.39 (SEQ ID NO : 68) | CDQIKNYIDKQLLPIVNKAGCGSAEEVLKDIEKALRNAGSKDCL KEIFSGIKEIKCGKEQAEKLKKEWERIKKEAGDG | pET21 |
| S0_1.40 (SEQ ID NO : 69) | ADQIKNYIDKQLLPIVNKAGCGSAEEVLKDIEKALRNAGSKDA LKEIFSGIKEIKCGKEQAEKLKKEWERIKKEAGDG | pET21 |

**Table 5 - Protein sequences for S4_2 design series.**

| Design name | Sequence | Successful expression |
|---|---|---|
| S4_2.07 (SEQ ID NO : 70) | MCSVVVGENYSIKCNPDGKCTIEDKNRGIIKTVTGSRCEL LYKAVQ KAQKGSWGSHHHHHH | yes |
| S4_2.19 (SEQ ID NO : 71) | MPNTNVFPSFSFTCLPDGKCIIEDSQRGIIKTGKNKEEFM EDFEKQV RAQGSWGSHHHHHH | yes |
| S4_2.20 (SEQ ID NO : 72) | MPSIYSDVPGGNIKVKCHEGKCTIEDSQRGIIKTVPMIET GEEMWK QVQEVLEEKRGSWGSHHHHHH | yes |
| S4_2.21 (SEQ ID NO : 73) | MPKTNVIPSFSFTCLGEKCTIEDSQRGIIKTGKNKEEVLE DFEKEER AQGSWSHHHHHH | yes |
| S4_2.22 (SEQ ID NO : 74) | MPSIYSDVPGNIKVKCHEGKCTIEDSQRGIIKTVPMIETG EEMWKQ PQELLEEKRGSWGSHHHHHH | yes |
| S4_2.35 (SEQ ID NO : 75) | MPNTNVFPSFSFTCLPDGKCIIEDSQRGIIKTGKNKEEFM EDFEKKV RAQGSWGSHHHHHH | yes |
| S4_2.45 (SEQ ID NO : 76) | MVCSVVVGENYSIKCDATKCTIEDKNRGIIKTVTGSRCEE LAKAV QKAQKGSWGSHHHHHH | yes |
| S4_2.60 (SEQ ID NO : 77) | MPSIYSDVPGGNVKVKCHEGKCTIEDSQRGIIKTVPMIET GEEMWK QVQEVVEEKRGSWGSHHHHHH | yes |
| S4_2.68 (SEQ ID NO : 78) | MPSIHSVVVGGNIKVKCHEGKCTIEDSQRGIIKTVPMIET GEEMQK QVQEFLEAKRGSWGSHHHHHH | yes |
| S4_2.73 (SEQ ID NO : 79) | MVCSVVVGENYSIKCDATKCTIEDSQRGIIKTGTHPEEFL EDLEKK ARAQGSWGSHHHHHH | yes |
| S4_2.74 (SEQ ID NO : 80) | MVFSCVVGENYSIKCDATKCTIEDSQRGIIKTGTHPEEFL EDLEKK ARAQGSWGSHHHHHH | yes |
| S4_2.84 (SEQ ID NO : 81) | MPSIHSVVPGGNIKVKCHEGKCTIEDSQRGIIKTVPMIET GEEMWK QPQELLEEKRGSWGSHHHHHH | yes |
| S4_2.85 (SEQ ID NO : 82) | MPNTNVFPSFSFTCLPDGKCIIEDSQRGIIKTGKNKEEFM EDFEKQV RAQGSWGSHHHHHH | yes |
| S4_2.94 (SEQ ID NO : 83) | HMPSIHSVVAGGNIKVKCHEGKCTIEDSQRGIIKTFTGFE PEEVWK QAQEFLEEKRGSWGSHHHHH | yes |

**Table 6 - Protein sequences for S0_2 design series.**

| Design name | Sequence | Successful expression |
|---|---|---|
| S0_2.37 (SEQ ID NO : 84) | MSCDQIKNYIDKQLLPIVNKAGCSRPEELEERIRRALKKF GDT DCLKDILLGIKEWKCGGSLEHHHHHH | no |
| S0_2.79 (SEQ ID NO : 85) | MPCDKQKNYIDKQLLPIVNKAGCSRPEEVEEMVRRALKKL GE TPCLEDILRGIKEIKCGGSLEHHHHHH | yes |
| S0_2.10 (SEQ ID NO : 86) | MPCDDAKNYIDKQLLPIVNKAGCSRPEEVERAVRKMLKKM G NTDCLEDILRGIKEIKCGGSLEHHHHHH | yes |
| S0_2.102 (SEQ ID NO : 87) | MSCDQIKNYIDKQLLPIVNKAGCGSAKEVQKDIEKALRNA GV KDCLEDILRGIKEWKCGGSLEHHHHHH | no |
| S0_2.31 (SEQ ID NO : 88) | MSCDESKNYIDKQLLPIVNKAGCDRPEDVERWIRKALKKM G DTSCFDEILKGLKEIKCGGSLEHHHHHH | no |
| S0_2.197 (SEQ ID NO : 89) | MSCDQIKNYIDKQLLPIVNKAGCSRPEEVEERIRRALKKM GDT SCFDEIMKGLKEIKCGGSLEHHHHHH | no |
| S0_2.57516 (SEQ ID NO : 90) | MSCDQIKNYIDKQLLPIVNKAGCNRPEEFEEWIKRALKKL GDT SCLEDILRGIKEIKCGGSLEHHHHHH | no |
| S0_2.57575 (SEQ ID NO : 91) | MSCDQIKNYIDKQLLPIVNKAGCSRPEEVEEMVRRALKKL GE TPCLEDILRGIKEWKCGGSLEHHHHHH | no |
| S0_2.57588 (SEQ ID NO : 92) | MSCDQIKNYIDKQLLPIVNKAGCSRPEEVERAVRKMLKKM G NTDCLEDILRGIKEIKCGGSLEHHHHHH | no |
| S0_2.57855 (SEQ ID NO : 93) | MSCDQIKNYIDKQLLPIVNKAGCGSAKEVQKDIEKALRNA GV KDCLKEIFSGIKEIKCGGSLEHHHHHH | no |
| S0_2.57910 (SEQ ID NO : 94) | MSCDQIKNYIDKQLLPIVNKAGCGSAKEVQKDIEKALRNA GV KDCLEDILRGIKEIKCGGSLEHHHHHH | no |
| S0_2.57911 | MSCEEAKNYIDKQLLPIVNKAGCGSAEEVQKDIEKALRNA | no |
| (SEQ ID NO : 95) | GV KDCLEDILRGIKEWKCGGSLEHHHHHH | |
| S0_2.57 (SEQ ID NO : 96) | MPCDDAKNYIDKQLLPIVNKAGCSRPEEVEERIRRALKKM GD TSCFDEIMKGLKEIKCGGSLEHHHHHH | yes |
| S0_2.58980 (SEQ ID NO : 97) | MSCEEAKNYIDKQLLPIVNKAGCSRPEELEEMIRRALKKM GD TSCFDEIMKGLKEIKCGGSLEHHHHHH | no |
| S0_2.611 (SEQ ID NO : 98) | MPCDKQKNYIDKQLLPIVNKAGCGSAKEVQKDIEKALRNA G VKDCLEDILRGIKEWKCGGSLEHHHHHH | yes |
| S0_2.126 (SEQ ID NO : 99) | MPCDKQKNYIDKQLLPIVNKAGCSRPEEVEERIRRALKKM GD TSCFDEILKGLKEIKCGGSLEHHHHHH | yes |

**Table 7 - Refinement statistics of the S0_2.126 NMR structure.**

| **NMR restraints** | |
|---|---|
| *Total NOEs from Unio^{a}* | *306* |
| Intraresidual | 124 |
| Interresidual | 182 |
| Sequential (i - j = 1) | 112 |
| Medium-range (1 < i - j < 5) | 47 |
| Long-range (i - j ≥ 5) | 23 |
| *Dihedral Angles from Talos-n* ^{b} | *88* |
| φ | 43 |
| ψ | 45 |
| | |

| **Structural statistics** | |
|---|---|
| ***Violations*^{c}** | |
| Distance restraints (A) | 0.0254 ± 0,009 |
| Dihedral angle constraints (°) | 6.8 ± 0.12 |

| *Ramachandran plot (all residues*/*ordered residues)* ^{d} | |
|---|---|
| Most favored (%) | 84.7 / 95.8 |
| Additionally allowed (%) | 14.3 / 4.5 |
| Generously allowed (%) | 0.98 / 0.1 |
| Disallowed (%) | 0 / 0 |

| *Average pairwise RMSD (A)* ^{c} | |
|---|---|
| Heavy | 3.3 / 1.8 |
| Backbone | 2.8 / 1.2 |

| *Structure Quality Factors (raw score*/*z-score)^{e}* | |
|---|---|
| Procheck G-factor (phi/psi) | 0.15 / 0.9 |
| Procheck G-factor (all) | -0.48 / -2.84 |

| | |
|---|---|
| ^{a} From UNIO-ATNOS/CANDID's last cycle (cycle 7) ^{b} Obtained from chemical shifts with Talos-N server ^{c} From Cyana in Unio's last cycle ^{d} All residues from Cyana, un Unio's last cycle; ordered residues (5-22,26-57) from the Protein Structure Validation Suite at http://psvs-1 5-dev.nesg.org/results/testbe/OUTPUT.html ^{e} From the Protein Structure Validation Suite | |

**Table 8 - X-ray data collection and refinement statistics of S0_2.126 crystal structure.**

| **D25 S0_2.126** | |
|---|---|
| Wavelength | 0.9763 |
| Resolution range | 49.09-3.0 (3.107-3.0) |
| Space group | P 21 21 21 |
| Unit cell | 126.3 127.0 156.1 90 90 90 |
| Total reflections | 700184 (72248) |
| Unique reflections | 50740 (5000) |
| Multiplicity | 13.8 (14.4) |
| Completeness (%) | 98.76 (99.22) |
| Mean l/sigma(l) | 12.63 (2.00) |
| Wilson B-factor | 74.78 |
| R-merge | 0.1622 (1.484) |
| R-meas | 0.1684 (1.538) |
| R-pim | 0.04506 (0.4019) |
| CC1/2 | 0.999 (0.893) |
| CC* | 1 (0.971) |
| Reflections used in refinement | 50284 (4971) |
| Reflections R-free | 2519 (249) |
| R-work | 0.2699 (0.3677) |
| R-free | 0.2936 (0.3972) |
| CC(work) | 0.949 (0.817) |
| CC(free) | 0.958 (0.793) |
| Number of non-hydrogen atoms | 14453 |
| macromolecules | 14452 |
| Protein residues | 1921 |
| RMS(bonds) | 0.004 |
| RMS(angles) | 1.02 |
| Ramachandran favored (%) | 94.45 |
| Ramachandran allowed (%) | 5.07 |
| Ramachandran outliers (%) | 0.48 |
| Rotamer outliers (%) | 0.00 |
| Clashscore | 7.35 |
| Average B-factor | 97.74 |
| macromolecules | 97.74 |
| solvent | 59.33 |
| Number of TLS groups | 12 |

**Table 9 - X-ray data collection and refinement statistics of S4_2.45 crystal structure.**

| **101F S4_2.45** | |
|---|---|
| Wavelength | 0.98 |
| Resolution range | 38.49 - 2.6 (2.693 - 2.6) |
| Space group | P 32 2 1 |
| Unit cell | 148.224 148.224 45.046 90 90 120 |
| Total reflections | 113069 (7302) |
| Unique reflections | 17464 (1567) |
| Multiplicity | 6.5 (4.7) |
| Completeness (%) | 98.57 (89.58) |
| Mean l/sigma(l) | 17.03 (1.66) |
| Wilson B-factor | 56.09 |
| R-merge | 0.06712 (0.8361) |
| R-meas | 0.07282 (0.9424) |
| R-pim | 0.02776 (0.4231) |
| CC1/2 | 0.999 (0.635) |
| CC* | 1 (0.881) |
| Reflections used in refinement | 17455 (1565) |
| Reflections used for R-free | 1748 (166) |
| R-work | 0.2298 (0.3682) |
| R-free | 0.2736 (0.3503) |
| CC(work) | 0.462 (0.203) |
| CC(free) | 0.353 (0.190) |
| Number of non-hydrogen atoms | 3794 |
| macromolecules | 3686 |
| solvent | 108 |
| Protein residues | 485 |
| RMS(bonds) | 0.010 |
| RMS(angles) | 1.46 |
| Ramachandran favored (%) | 93.53 |
| Ramachandran allowed (%) | 5.64 |
| Ramachandran outliers (%) | 0.84 |
| Rotamer outliers (%) | 0.96 |
| Clashscore | 2.19 |
| Average B-factor | 38.90 |
| macromolecules | 38.37 |
| solvent | 56.18 |
| Number of TLS groups | 3 |

### References for Methods section:

1. J. S. McLellan et al., Structure of RSV fusion glycoprotein trimer bound to a prefusion-specific neutralizing antibody. Science 340, 1113-1117 (2013).
2. J. Zhou, G. Grigoryan, Rapid search for tertiary fragments reveals protein sequence-structure relationships. Protein Sci 24, 508-524 (2015).
3. T. J. Brunette et al., Exploring the repeat protein universe through computational protein design. Nature 528, 580-584 (2015).
4. J. Bonet et al., Rosetta FunFolDes - A general framework for the computational design of functional proteins. PLoS Comput Biol 14, e1006623 (2018).
5. X. Hu, H. Wang, H. Ke, B. Kuhlman, High-resolution design of a protein loop. Proc Natl Acad Sci U S A 104, 17668-17673 (2007).
6. P. Conway, M. D. Tyka, F. DiMaio, D. E. Konerding, D. Baker, Relaxation of backbone bond geometry improves protein energy landscape modeling. Protein Sci 23, 47-55 (2014).
7. J. S. McLellan et al., Structure of a major antigenic site on the respiratory syncytial virus fusion glycoprotein in complex with neutralizing antibody 101F. J Virol 84, 12236-12244 (2010).
8. V. Mas et al., Engineering, Structure and Immunogenicity of the Human Metapneumovirus F Protein in the Postfusion Conformation. PLoS Pathog 12, e1005859 (2016).
9. M. D. Tyka et al., Alternate states of proteins revealed by detailed energy landscape mapping. J Mol Biol 405, 607-618 (2011).
10. M. D. Finucane, M. Tuna, J. H. Lees, D. N. Woolfson, Core-directed protein design. I. An experimental method for selecting stable proteins from combinatorial libraries. Biochemistry 38, 11604-11612 (1999).
11. P. Kristensen, G. Winter, Proteolytic selection for protein folding using filamentous bacteriophages. Fold Des 3, 321-328 (1998).
12. A. Chevalier et al., Massively parallel de novo protein design for targeted therapeutics. Nature 550, 74-79 (2017).
13. F. Sesterhenn et al., Boosting subdominant neutralizing antibody responses with a computationally designed epitope-focused immunogen. PLoS Biol 17, e3000164 (2019).
14. G. Chao et al., Isolating and engineering human antibodies using yeast surface display. Nat Protoc 1, 755-768 (2006).
15. M. G. Joyce et al., Iterative structure-based improvement of a fusion-glycoprotein vaccine against RSV. Nat Struct Mol Biol 23, 811-820 (2016).
16. B. Briney et al., Tailored Immunogens Direct Affinity Maturation toward HIV Neutralizing Antibodies. Cell 166, 1459-1470 e1411 (2016).
17. A. Rohou, N. Grigorieff, CTFFIND4: Fast and accurate defocus estimation from electron micrographs. J Struct Biol 192, 216-221 (2015).
18. J. M. de la Rosa-Trevin et al., Scipion: A software framework toward integration, reproducibility and validation in 3D electron microscopy. J Struct Biol 195, 93-99 (2016).
19. S. H. Scheres, RELION: implementation of a Bayesian approach to cryo-EM structure determination. J Struct Biol 180, 519-530 (2012).
20. A. Punjani, J. L. Rubinstein, D. J. Fleet, M. A. Brubaker, cryoSPARC: algorithms for rapid unsupervised cryo-EM structure determination. Nat Methods 14, 290-296 (2017).
21. M. Sattler, J. Schleucher, C. Griesinger, Heteronuclear multidimensional NMR experiments for the structure determination of proteins in solution employing pulsed field gradients. Frog Nucl Mag Res Sp 34, 93-158 (1999).
22. T. Herrmann, P. Guntert, K. Wuthrich, Protein NMR structure determination with automated NOE-identification in the NOESY spectra using the new software ATNOS. Journal of Biomolecular Nmr 24, 171-189 (2002).
23. T. Herrmann, P. Guntert, K. Wuthrich, Protein NMR structure determination with automated NOE assignment using the new software CANDID and the torsion angle dynamics algorithm DYANA. Journal of Molecular Biology 319, 209-227 (2002).
24. D. Gottstein, D. K. Kirchner, P. Guntert, Simultaneous single-structure and bundle representation of protein NMR structures in torsion angle space. J Biomol NMR 52, 351-364 (2012).
25. Y. Shen, A. Bax, Protein backbone and sidechain torsion angles predicted from NMR chemical shifts using artificial neural networks. Journal of Biomolecular Nmr 56, 227¬241 (2013).
26. W. Kabsch, Xds. Acta Crystallogr D 66, 125-132 (2010).
27. A. J. Mccoy et al., Phaser crystallographic software. J Appl Crystallogr 40, 658-674 (2007).
28. P. Emsley, B. Lohkamp, W. G. Scott, K. Cowtan, Features and development of Coot. Acta Crystallogr D 66, 486-501 (2010).
29. P. D. Adams et al., PHENIX: a comprehensive Python-based system for macromolecular structure solution. Acta Crystallogr D 66, 213-221 (2010).
30. P. A. Karplus, K. Diederichs, Linking crystallographic model and data quality. Science 336, 1030-1033 (2012).

## Claims

1. A vaccine composition against a target pathogen, the composition comprising a plurality of non-naturally occurring immunogenic polypeptides; at least a first of said immunogenic polypeptides comprising a mimic peptide having an amino acid sequence having a tertiary structure which, when folded, mimics a complex and/or discontinuous neutralisation epitope from said target pathogen.

2. The vaccine composition of claim 1, wherein each of said plurality of non-naturally occurring immunogenic polypeptides comprises a mimic peptide having an amino acid sequence which, when folded, mimics a complex and/or discontinuous neutralisation epitope from said target pathogen.

3. The vaccine composition of claim 2, wherein each of said complex and/or discontinuous neutralisation epitopes are non-overlapping.

4. The vaccine composition of any preceding claim, wherein said target pathogen is RSV.

5. The vaccine composition of claim 4, wherein said complex and/or discontinuous neutralisation epitopes are selected from the group consisting of RSV site 0, site II, and site IV.

6. The vaccine composition of claim 5, wherein said immunogenic peptides are selected from the peptides described in tables 3 to 6, and preferably from tables 5 or 6.

7. The vaccine composition of any preceding claim wherein said immunogenic peptide comprises a scaffold, preferably a peptide scaffold, which presents the mimic peptide so as to assist the mimicking of the complex and/or discontinuous neutralisation epitope.

8. The vaccine composition of claim 7 wherein said scaffold is selected from RSVN and ferritin.

9. The vaccine composition of any preceding claim, in combination with a vaccine composition comprising a native immunogen from the target pathogen.

10. A vaccine composition comprising the S0_2.126 peptide sequence as described herein, and the S4_2.45 peptide sequence as described herein, and optionally further comprising the FFL_001 or FFLM peptides.

11. A vaccine composition of any preceding claim, wherein said target pathogen is RSV, for use in a method for immunising a subject against RSV, the method comprising a) administering said vaccine composition to a subject; and b) prior to said administration, administering a further vaccine composition comprising an RSV-derived protein or glycoprotein, preferably the RSVF glycoprotein, or wherein the vaccine composition of any preceding claim is administered to a subject who has previously been exposed to RSV infection.

12. A method for designing a peptide to mimic a complex and/or discontinuous structural configuration of a target peptide, the method comprising the steps of:
determining a complex and/or discontinuous structural configuration of a target peptide to mimic;
identifying a preliminary mimic peptide having an amino acid sequence;
determining likely structural configuration of said preliminary mimic peptide amino acid sequence by in silico analysis of said sequence;
performing directed evolution on said preliminary mimic peptide to generate a range of variants of said peptide; (preferably wherein directed evolution may be performed by mutagenesis to generate variants and expression of said variants); and
selecting for variants of said peptide which display an improvement in a desired characteristic seen in said target peptide (said characteristic may be, for example, binding affinity to a target such as an antibody; thermal stability; susceptibility or resistance to an enzyme).

13. The method of claim 12 further comprising the steps of identifying a plurality of said variants having improvements, and providing a further peptide having a combination of variations from said plurality of variants.

14. The method of claim 12 or 13 wherein said step of identifying a preliminary mimic peptide comprises selecting a peptide from a peptide database having a structural similarity to the desired target peptide; or wherein said step comprises combining an amino acid sequence from said target peptide with one or more structural peptide elements such that said preliminary mimic peptide sequence has a structural similarity to the desired target peptide.


SEQUENCE LISTING

<110>  ECOLE POLYTECHNIQUE FEDERALE DE LAUSANNE

<120>  Immunogen

<130>  PC928995EP

<160>  99

<170>  PatentIn version 3.5

<210>  1
<211>  112
<212>  PRT
<213>  Artificial Sequence

<220>
<223>  FFLM Peptide

<400>  1

Ala Ser Arg Glu Asp Met Arg Glu Glu Ala Asp Glu Asp Phe Lys Ser
1               5                   10                  15

Phe Val Glu Ala Ala Lys Asp Asn Phe Asn Lys Phe Lys Ala Arg Leu
            20                  25                  30

Arg Lys Gly Lys Ile Thr Arg Glu His Arg Glu Met Met Lys Lys Leu
        35                  40                  45

Ala Lys Gln Asn Ala Asn Lys Ala Lys Glu Ala Val Arg Lys Arg Leu
    50                  55                  60

Ser Glu Leu Leu Ser Lys Ile Asn Asp Met Pro Ile Thr Asn Asp Gln
65                  70                  75                  80

Lys Lys Leu Met Ser Asn Gln Val Leu Gln Phe Ala Asp Asp Ala Glu
                85                  90                  95

Ala Glu Ile Asp Gln Leu Ala Ala Asp Ala Thr Lys Glu Phe Thr Gly
            100                 105                 110

<210>  2
<211>  60
<212>  DNA
<213>  Artificial Sequence

<220>
<223>  S4_1_SSM_fw

<400>  2
caggctagtg gtggaggagg ctctggtgga ggcggtagcg gaggcggagg gtcggctagc        60

<210>  3

<211>  63
<212>  DNA
<213>  Artificial Sequence

<220>
<223>  S4_1_SSM_rw

<400>  3
ctgttgttat cagatctcga gctattacaa gtcctcttca gaaataagct tttgttcgga      60

tcc                                                                    63


<210>  4
<211>  32
<212>  DNA
<213>  Artificial Sequence

<220>
<223>  S4_1_#18_rw

<400>  4
tttcgggcat ttgactttga taccattgct gt                                    32


<210>  5
<211>  60
<212>  DNA
<213>  Artificial Sequence

<220>
<223>  S4_1_#18_fw


<220>
<221>  misc_feature
<222>  (29)..(30)
<223>  n is a, c, g, or t

<400>  5
caatggtatc aaagtcaaat gcccgaaann kggtgaatgt acgattaaag acagtcaacg      60


<210>  6
<211>  33
<212>  DNA
<213>  Artificial Sequence

<220>
<223>  S4_1_#20_rw

<400>  6
ctttcgggca tttgactttg ataccattgc tgt                                   33


<210>  7
<211>  64
<212>  DNA
<213>  Artificial Sequence

<220>
<223>  S4_1_#20_fw

<220>
<221>  misc_feature
<222>  (36)..(37)
<223>  n is a, c, g, or t

<400>  7
gcaatggtat caaagtcaaa tgcccgaaag gcggtnnktg tacgattaaa gacagtcaac      60

gtgg                                                                  64


<210>  8
<211>  34
<212>  DNA
<213>  Artificial Sequence

<220>
<223>  S4_1_#22_rw

<400>  8
cctttcgggc atttgacttt gataccattg ctgt                                 34


<210>  9
<211>  71
<212>  DNA
<213>  Artificial Sequence

<220>
<223>  S4_1_#22_fw


<220>
<221>  misc_feature
<222>  (42)..(43)
<223>  n is a, c, g, or t

<400>  9
gcaatggtat caaagtcaaa tgcccgaaag gcggtgaatg tnnkattaaa gacagtcaac      60

gtggcattat c                                                          71


<210>  10
<211>  26
<212>  DNA
<213>  Artificial Sequence

<220>
<223>  S4 1 #25 rw

<400>  10
tttaatcgta cattcaccgc ctttcg                                          26


<210>  11
<211>  53
<212>  DNA
<213>  Artificial Sequence

<220>
<223>  S4_1_#25_fw

<220>
<221>  misc_feature
<222>  (27)..(28)
<223>  n is a, c, g, or t

<400>  11
cgaaaggcgg tgaatgtacg attaaannka gtcaacgtgg cattatcaaa acc          53


<210>  12
<211>  29
<212>  DNA
<213>  Artificial Sequence

<220>
<223>  S4_1_#36_rw

<400>  12
gctaaaggtt ttgataatgc cacgttgac                                     29


<210>  13
<211>  52
<212>  DNA
<213>  Artificial Sequence

<220>
<223>  S4_1_#36_fw


<220>
<221>  misc_feature
<222>  (28)..(29)
<223>  n is a, c, g, or t

<400>  13
caacgtggca ttatcaaaac ctttagcnnk ggtacggaag aagttcgcag tc           52


<210>  14
<211>  31
<212>  DNA
<213>  Artificial Sequence

<220>
<223>  S4 1 #39 rw

<400>  14
cgtaccagag ctaaaggttt tgataatgcc a                                  31


<210>  15
<211>  53
<212>  DNA
<213>  Artificial Sequence

<220>
<223>  S4_1_#39_fw


<220>

<221>  misc_feature
<222>  (30)..(31)
<223>  n is a, c, g, or t

<400>  15
gcattatcaa aacctttagc tctggtacgn nkgaagttcg cagtccgtcc ctg          53


<210>  16
<211>  28
<212>  DNA
<213>  Artificial Sequence

<220>
<223>  S4 1 #43 rw

<400>  16
gcgaacttct tccgtaccag agctaaag                                      28


<210>  17
<211>  49
<212>  DNA
<213>  Artificial Sequence

<220>
<223>  S4_1_#43_fw


<220>
<221>  misc_feature
<222>  (24)..(25)
<223>  n is a, c, g, or t

<400>  17
gctctggtac ggaagaagtt cgcnnkccgt ccctgggcaa agtgaccgt               49


<210>  18
<211>  59
<212>  DNA
<213>  Artificial Sequence

<220>
<223>  S4_1_#45_fw


<220>
<221>  misc_feature
<222>  (30)..(31)
<223>  n is a, c, g, or t

<400>  18
gctctggtac ggaagaagtt cgcagtccgn nkctgggcaa agtgaccgtt ggtgataac    59


<210>  19
<211>  24
<212>  DNA
<213>  Artificial Sequence

<220>
<223>  S4 1 #48 rw

<400>  19
gcccagggac ggactgcgaa cttc                                              24


<210>  20
<211>  48
<212>  DNA
<213>  Artificial Sequence

<220>
<223>  S4_1_#48_fw


<220>
<221>  misc_feature
<222>  (22)..(23)
<223>  n is a, c, g, or t

<400>  20
gttcgcagtc cgtccctggg cnnkgtgacc gttggtgata acacgttc                    48


<210>  21
<211>  54
<212>  DNA
<213>  Artificial Sequence

<220>
<223>  S4_1_#50_fw


<220>
<221>  misc_feature
<222>  (28)..(29)
<223>  n is a, c, g, or t

<400>  21
gttcgcagtc cgtccctggg caaagtgnnk gttggtgata acacgttcga agcg             54


<210>  22
<211>  77
<212>  DNA
<213>  Artificial Sequence

<220>
<223>  S4_2_uni_O1

<400>  22
gacaatagct cgacgattga aggtagatac ccatacgacg ttccagacta cgctctgcag       60

gctagtggtg gaggagg                                                      77


<210>  23
<211>  51
<212>  DNA
<213>  Artificial Sequence

<220>
<223>  S4_2_uni_O2

<400>  23
ccctccgcct ccgctaccgc ctccaccaga gcctcctcca ccactagcct g          51

<210>  24
<211>  80
<212>  DNA
<213>  Artificial Sequence

<220>
<223>  S4_2_bb1_O3.1

<400>  24
gtagcggagg cggagggtcg gctagccata tgccgtccat cyactcakwc gttsytggtg          60

ggaacatcaa ggtgaagtgc          80

<210>  25
<211>  77
<212>  DNA
<213>  Artificial Sequence

<220>
<223>  S4_2_bb1_O3.2

<400>  25
gtagcggagg cggagggtcg gctagccata tgccgtccat cyactcakwc gttsytggga          60

acatcaaggt gaagtgc          77

<210>  26
<211>  80
<212>  DNA
<213>  Artificial Sequence

<220>
<223>  S4_2_bb1_O3.3

<400>  26
gtagcggagg cggagggtcg gctagccata tgccgtccat cyactcakwc gttsytaacg          60

ggaacatcaa ggtgaagtgc          80

<210>  27
<211>  72
<212>  DNA
<213>  Artificial Sequence

<220>
<223>  S4_2_bb1_O4.1

<400>  27
ggtcttgatg atgccacgct ggctatcctc gatggtacat ttgtcaccag tgcacttcac          60

cttgatgttc cc          72

<210>  28
<211>  72
<212>  DNA

<213>  Artificial Sequence

<220>
<223>  S4_2_bb1_O4.2

<400>  28
ggtcttgatg atgccacgat tcttgttctc gatggtacat ttgtcaccag tgcacttcac      60

cttgatgttc cc                                                          72


<210>  29
<211>  72
<212>  DNA
<213>  Artificial Sequence

<220>
<223>  S4_2_bb1_O4.3

<400>  29
ggtcttgatg atgccacgct ggctatcctc gatggtacac ttgccctcgt ggcacttcac      60

cttgatgttc cc                                                          72


<210>  30
<211>  100
<212>  DNA
<213>  Artificial Sequence

<220>
<223>  S4_2_bb1_O5.1

<400>  30
gcgtggcatc atcaagacca cgaatgttga tattgctgag gagrygyrga agcagsytca      60

agagbytbwg gaagmgaaac gtaagggctc gtggggctcg                           100


<210>  31
<211>  100
<212>  DNA
<213>  Artificial Sequence

<220>
<223>  S4_2_bb1_O5.2

<400>  31
gcgtggcatc atcaagacct tcacggggtt cgagcccgag gagrygyrga agcagsytca      60

agagbytbwg gaagmgaaac gtaagggctc gtggggctcg                           100


<210>  32
<211>  100
<212>  DNA
<213>  Artificial Sequence

<220>
<223>  S4_2_bb1_O5.3

<400>  32
gcgtggcatc atcaagaccg tcccgatgat cgagacaggg gaggagrygy rgaagcagsy      60

tcaagagbyt bwggaagmga aacgtggctc gtggggctcg                          100

<210>  33
<211>  73
<212>  DNA
<213>  Artificial Sequence

<220>
<223>  S4_2_uni_06

<400>  33
cagaaataag cttttgttcg gatccgggct cagcctatta gtggtggtgg tggtggtgcg      60

agccccacga gcc                                                          73

<210>  34
<211>  58
<212>  DNA
<213>  Artificial Sequence

<220>
<223>  S4_2_uni_07

<400>  34
ggatccgaac aaaagcttat ttctgaagag gacttgtaat agctcgagat ctgataac        58

<210>  35
<211>  84
<212>  DNA
<213>  Artificial Sequence

<220>
<223>  S4_2_uni_08

<400>  35
gtacgagcta aaagtacagt gggaacaaag tcgattttgt tacatctaca ctgttgttat      60

cagatctcga gctattacaa gtcc                                              84

<210>  36
<211>  100
<212>  DNA
<213>  Artificial Sequence

<220>
<223>  S4_2_bb2_03.1

<400>  36
tagcggaggc ggagggtcgg ctagccatat gccaaawacc hacgtawttg aagcaggcdt      60

cagcttcacc tgcttaggtg agaagtgcac catcgaggac                            100

<210>  37
<211>  97
<212>  DNA
<213>  Artificial Sequence

<220>
<223>  S4_2_bb2_O3.2

<400>  37
tagcggaggc ggagggtcgg ctagccatat gccaaawacc hacgtawttc cctcgdtcag      60

cttcacctgc ttaggtgaga agtgcaccat cgaggac                               97


<210>  38
<211>  100
<212>  DNA
<213>  Artificial Sequence

<220>
<223>  S4_2_bb2_O3.3

<400>  38
tagcggaggc ggagggtcgg ctagccatat gccaaawacc hacgtawttc cctcgdtcag      60

cttcacctgc cctaaggggg ggaagtgcac catcgaggac                           100


<210>  39
<211>  44
<212>  DNA
<213>  Artificial Sequence

<220>
<223>  S4_2_bb2_O4.1

<400>  39
cggtcttgat gatcccacgt tgtgagtcct cgatggtgca cttc                       44


<210>  40
<211>  41
<212>  DNA
<213>  Artificial Sequence

<220>
<223>  S4_2_bb2_O4.2

<400>  40
cggtcttgat gatcccacga tcgtcctcga tggtgcactt c                          41


<210>  41
<211>  44
<212>  DNA
<213>  Artificial Sequence

<220>
<223>  S4_2_bb2_O4.3

<400>  41
cggtcttgat gatcccacgc gagcggtcct cgatggtgca cttc                       44


<210>  42
<211>  90
<212>  DNA
<213>  Artificial Sequence

<220>
<223>  S4_2_bb2_O5.1

<400>  42
cgtgggatca tcaagaccgg caaaaatgcc gaggagkycd kggaagatbt cgagaagvrg      60

ghgcgtgccc agggctcgtg gggctcgcac                                        90


<210>  43
<211>  90
<212>  DNA
<213>  Artificial Sequence

<220>
<223>  S4_2_bb2_O5.2

<400>  43
cgtgggatca tcaagaccgg cacacatcca gaggagkycd kggaagatbt cgagaagvrg      60

ghgcgtgccc agggctcgtg gggctcgcac                                        90


<210>  44
<211>  90
<212>  DNA
<213>  Artificial Sequence

<220>
<223>  S4_2_bb2_O5.3

<400>  44
cgtgggatca tcaagaccgg caaaaataag gaggagkycd kggaagatbt cgagaagvrg      60

ghgcgtgccc agggctcgtg gggctcgcac                                        90


<210>  45
<211>  100
<212>  DNA
<213>  Artificial Sequence

<220>
<223>  S4_2_bb3_O3.1

<400>  45
tagcggaggc ggagggtcgg ctagccatat ggtctksagt kktgtagytg gggagaacta      60

ttcarytaag tgtactggcg acaagtgcac catcgaggac                           100


<210>  46
<211>  100
<212>  DNA
<213>  Artificial Sequence

<220>
<223>  S4_2_bb3_O3.2

<400>  46
tagcggaggc ggagggtcgg ctagccatat ggtctksagt kktgtagyta ccccgacatt      60

ttcarytaag tgtactggcg acaagtgcac catcgaggac                              100


<210>  47
<211>  100
<212>  DNA
<213>  Artificial Sequence

<220>
<223>  S4_2_bb3_O3.3

<400>  47
tagcggaggc ggagggtcgg ctagccatat gtksagtkkt gtagytgggg agaactattc        60

arytaagtgt cctaaggggg gcaagtgcac catcgaggac                              100


<210>  48
<211>  43
<212>  DNA
<213>  Artificial Sequence

<220>
<223>  S4 2 bb3 O4.1

<400>  48
ggtcttgatg atcccgcgct gtgagtcctc gatggtgcac ttg                          43


<210>  49
<211>  43
<212>  DNA
<213>  Artificial Sequence

<220>
<223>  S4 2 bb3 O4.2

<400>  49
ggtcttgatg atcccgcgat tcttgtcctc gatggtgcac ttg                          43


<210>  50
<211>  46
<212>  DNA
<213>  Artificial Sequence

<220>
<223>  S4 2 bb3 O4.3

<400>  50
ggtcttgatg atcccgcgcc cgccatagtc ctcgatggtg cacttg                       46


<210>  51
<211>  87
<212>  DNA
<213>  Artificial Sequence

<220>
<223>  S4_2_bb3_O5.1

<400>  51
cgcgggatca tcaagaccac gattggagat acatgtgags hgkygkmtaa ggcggytcaa        60

aaggctsvga aaggctcgtg gggctcg                                          87

<210>  52
<211>  87
<212>  DNA
<213>  Artificial Sequence

<220>
<223>  S4_2_bb3_O5.2

<400>  52
cgcgggatca tcaagaccgt tactggcagt cgctgtgags hgkygkmtaa ggcggytcaa      60

aaggctsvga aaggctcgtg gggctcg                                          87

<210>  53
<211>  72
<212>  PRT
<213>  Artificial Sequence

<220>
<223>  S4_1.1

<400>  53

Met Asp Gly Thr Leu Gln Ile Asn Ser Asn Gly Ile Lys Val Lys Cys
1               5                   10                  15

Pro Lys Gly Gly Glu Cys Thr Ile Lys Asp Ser Gln Arg Gly Ile Ile
            20                  25                  30

Lys Thr Phe Ser Ser Gly Thr Glu Glu Val Arg Ser Pro Ser Leu Gly
        35                  40                  45

Lys Val Thr Val Gly Asp Asn Thr Phe Glu Ala Ser Asn Gly Ser Trp
    50                  55                  60

Leu Glu His His His His His His
65                  70

<210>  54
<211>  70
<212>  PRT
<213>  Artificial Sequence

<220>
<223>  S4_1.2

<400>  54

Met His His His His His His Trp Gly Ser Pro Gly Thr Val Thr Leu
1               5                   10                  15

Asn Ser Asn Gly Leu Thr Val Thr Gly Asn Asp Asn Tyr Asn Leu Thr

20                      25                      30

Val Thr Gly Asn Asp Arg Gly Ile Ile Lys Thr Phe Ser Pro Ser Thr
        35                      40                      45

Glu Thr Thr Asn Asp Asp Gly Met Ser Thr Ile Thr Val Gly Asn Leu
    50                      55                      60

Thr Val Thr Leu Gly Asn
65                      70

<210>  55
<211>  70
<212>  PRT
<213>  Artificial Sequence

<220>
<223>  S4_1.3

<400>  55

Met His His His His His His Trp Gly Ser Gln Ser Thr Val Asn Val
1               5                   10                      15

Gln Asp Lys Asn Ile Arg Ile Glu Val Asp Asp Lys Asn Ser Val Gln
            20                      25                      30

Val Asn Gly Ser Asn Arg Gly Ile Ile Lys Thr Phe Ser Pro Gly Thr
        35                      40                      45

Val Gln Ile Ser Ser Lys Asn Gly Asp Thr Val Thr Val Gly Asn Val
    50                      55                      60

Arg Val Asn Met Gly Gly
65                      70

<210>  56
<211>  70
<212>  PRT
<213>  Artificial Sequence

<220>
<223>  S4_1.4

<400>  56

Met His His His His His His Trp Gly Ser Gln Ser Thr Val Asn Val
1               5                   10                      15

Gln Asp Lys Asn Ile Arg Ile Glu Cys Asp Asp Asn Cys Gly Val Gln
            20                      25                      30

Val Asn Gly Ser Asn Arg Gly Ile Ile Lys Thr Phe Ser Pro Gly Thr
        35                  40                  45

Val Gln Ile Ser Ser Lys Asn Gly Asp Thr Val Thr Val Gly Asn Val
    50                  55                  60

Arg Val Asn Met Gly Gly
65                  70

<210> 57
<211> 70
<212> PRT
<213> Artificial Sequence

<220>
<223> S4_1.5

<400> 57

Met His His His His His His Trp Gly Ser Asp Gly Thr Leu Gln Ile
1               5                   10                  15

Asn Ser His Gly Val Lys Val Lys Ala Pro Pro Gly Ser Gly Ala Thr
            20                  25                  30

Val Lys Asp Ser Gln Arg Gly Ile Ile Lys Thr Phe Ser Ser Gly Tyr
        35                  40                  45

Glu Glu Val Arg Ser Pro Ser Leu Gly Lys Val Thr Val Gly Asp Asn
    50                  55                  60

Thr Phe Glu Val Ser Asn
65                  70

<210> 58
<211> 70
<212> PRT
<213> Artificial Sequence

<220>
<223> S4_1.6

<400> 58

Met His His His His His His Trp Gly Ser Asp Gly Thr Leu Gln Ile
1               5                   10                  15

Asn Ser His Gly Val Lys Val Lys Cys Pro Lys Gly Ser Glu Cys Thr
            20                  25                  30

Val Lys Asp Ser Gln Arg Gly Ile Ile Lys Thr Phe Ser Ser Gly Tyr
        35                  40                  45

Glu Glu Val Arg Ser Pro Ser Leu Gly Lys Val Thr Val Gly Asp Asn
    50                  55                  60

Thr Phe Glu Val Ser Asn
65                  70

<210>  59
<211>  69
<212>  PRT
<213>  Artificial Sequence

<220>
<223>  S4_1.7

<400>  59

Met His His His His His His Gly Ser Lys Val Thr Phe Arg Gln Asp
1               5                   10                  15

Lys Asn Gly Ile Lys Ile Arg Val Asn Gly Asn Lys Gly Leu Val Ile
            20                  25                  30

Arg Thr Asn Asp Arg Gly Ile Ile Lys Thr Phe Ser Asn Gly Thr Tyr
        35                  40                  45

Asp Ile Pro Asn Ser Gly Tyr Asn Arg Phe Thr Val Gly Gly Thr Gln
    50                  55                  60

Phe Asp Trp Asn Glu
65

<210>  60
<211>  69
<212>  PRT
<213>  Artificial Sequence

<220>
<223>  S4_1.8

<400>  60

Met His His His His His His Gly Ser Lys Val Thr Phe Arg Gln Asp
1               5                   10                  15

Lys Asn Gly Ile Lys Phe Arg Val Asn Gly Asn Lys Gly Ala Val Ile
            20                  25                  30

Arg Thr Asn Asp Arg Gly Ile Ile Lys Thr Phe Ser Asn Gly Thr Tyr
        35                  40                  45

Asp Ile Pro Asn Ser Gly Tyr Asn Arg Phe Thr Val Gly Gly Asn Thr
    50                  55                  60

Phe Asp Trp Asn Glu
65

<210> 61
<211> 72
<212> PRT
<213> Artificial Sequence

<220>
<223> S4_1.9

<400> 61

Met Asp Gly Thr Leu Gln Ile Asn Ser Asn Gly Val Lys Val Lys Cys
1               5                   10                  15

Pro Lys Gly Val Glu Cys Thr Val Lys Asp Ser Gln Arg Gly Ile Ile
            20                  25                  30

Lys Thr Phe Ser Ser Gly Thr Glu Glu Val Arg Ser Pro Ser Leu Gly
        35                  40                  45

Lys Val Thr Val Gly Asp Asn Thr Phe Glu Val Ser Asn Gly Ser Trp
    50                  55                  60

Leu Glu His His His His His His
65                  70

<210> 62
<211> 72
<212> PRT
<213> Artificial Sequence

<220>
<223> S4_1.10

<400> 62

Met Asp Gly Thr Leu Gln Ile Asn Ser Asn Gly Val Lys Val Lys Cys
1               5                   10                  15

Pro Lys Gly Ala Glu Cys Thr Val Lys Val Ser Gln Arg Gly Ile Ile
            20                  25                  30

Lys Thr Phe Ser Ser Gly Thr Glu Glu Val Arg Ser Pro Ser Leu Gly
        35                  40                  45

Lys Val Thr Val Gly Asp Asn Thr Thr Glu Val Ser Asn Gly Ser Trp
    50                  55                  60

Leu Glu His His His His His His

65                  70

<210>  63
<211>  70
<212>  PRT
<213>  Artificial Sequence

<220>
<223>  S4_1.11

<400>  63

Met His His His His His His Trp Gly Ser Pro Gly Thr Val Lys Leu
1               5                   10                  15

Asn Ser Asn Gly Leu Thr Val Arg Gly Asn Asp Ser Tyr Gly Leu Thr
            20                  25                  30

Val Arg Gly Asn Asp Arg Gly Ile Ile Lys Thr Phe Ser Pro Ser Thr
        35                  40                  45

Glu Val Val Gln Ser Lys Gly Met Ser Thr Ile Thr Val Gly Asn Leu
    50                  55                  60

Asp Val Arg Leu Gly Glu
65                  70

<210>  64
<211>  129
<212>  PRT
<213>  Artificial Sequence

<220>
<223>  S0_1.1

<400>  64

Pro Glu Asp Ala Gln Lys Glu Ala Ser Lys Gly Ser Glu Val Arg Glu
1               5                   10                  15

Leu Lys Asn Ile Ile Asp Lys Gln Leu Leu Pro Ile Val Asn Lys Thr
            20                  25                  30

Ser Cys Ser Gly Ala Glu Gln Ala Ala Glu Ala Leu Arg Glu Ala Leu
        35                  40                  45

Glu Gly Ala Gly Ser Cys Asp Ala Val Glu Gln Leu Leu Gly Asn Ile
    50                  55                  60

Lys Glu Ile Lys Cys Gly Thr Asp Ala Gly Arg Ala Leu Ile Arg Ile
65                  70                  75                  80

Leu Ala Glu Val Ala Arg Glu Ile Gly Cys Pro Arg Ala Ile Asp Gln
                85                  90                  95

Val Ala Glu Trp Val Arg Arg Ile Ala Lys Ala Val Gly Gly Glu Glu
            100                 105                 110

Ala Lys Lys Gln Val Lys Glu Val Glu Lys Glu Ile Arg Arg Glu Lys
        115                 120                 125

Gly

<210>  65
<211>  93
<212>  PRT
<213>  Artificial Sequence

<220>
<223>  S0_1.17

<400>  65

Pro Glu Asp Ala Gln Lys Glu Ala Ser Lys Gly Ser Glu Val Arg Glu
1               5                   10                  15

Leu Lys Asn Ile Ile Asp Lys Gln Leu Leu Pro Ile Leu Asn Lys Ala
            20                  25                  30

Ser Cys Ser Gly Ala Glu Gln Leu Leu Glu Ala Leu Arg Glu Ala Leu
        35                  40                  45

Glu Gly Ala Gly Ser Cys Asp Ala Val Glu Gln Leu Leu Gly Asn Ile
    50                  55                  60

Lys Glu Ile Lys Cys Gly Thr Asp Ala Gly Arg Ala Leu Lys Arg Ile
65                  70                  75                  80

Leu Glu Glu Val Gln Arg Glu Ile Gly Cys Gly Ser Trp
                85                  90

<210>  66
<211>  77
<212>  PRT
<213>  Artificial Sequence

<220>
<223>  S0_1.37

<400>  66

Cys Asp Gln Leu Lys Asn Tyr Ile Asp Lys Gln Leu Leu Pro Ile Val
1               5                   10                  15

Asn Lys Gln Ser Cys Ala Asn Gly Glu Glu Ala Leu Lys Asp Ile Glu
            20                  25                  30

Lys Ala Leu Arg Gly Ala Gly Ser Lys Asp Cys Trp Lys Glu Leu Leu
        35                  40                  45

Ser Asn Ile Lys Glu Ile Lys Cys Gly Lys Glu Phe Ala Glu Lys Leu
    50                  55                  60

Lys Lys Glu Trp Glu Arg Ile Lys Lys Glu Ala Gly Asp
65                  70                  75

<210> 67
<211> 77
<212> PRT
<213> Artificial Sequence

<220>
<223> S0_1.38

<400> 67

Cys Asp Gln Ile Lys Asn Tyr Ile Asp Lys Gln Leu Leu Pro Ile Val
1               5                   10                  15

Asn Lys Ala Gly Cys Gly Ser Ala Glu Glu Ala Leu Lys Asp Ile Glu
            20                  25                  30

Lys Ala Leu Arg Leu Ala Gly Ser Lys Asp Cys Leu Lys Glu Ile Phe
        35                  40                  45

Ser Asn Ile Lys Glu Ile Lys Cys Gly Lys Glu Phe Ala Glu Lys Leu
    50                  55                  60

Lys Lys Glu Trp Glu Arg Ile Lys Lys Glu Ala Gly Asp
65                  70                  75

<210> 68
<211> 78
<212> PRT
<213> Artificial Sequence

<220>
<223> S0_1.39

<400> 68

Cys Asp Gln Ile Lys Asn Tyr Ile Asp Lys Gln Leu Leu Pro Ile Val
1               5                   10                  15

Asn Lys Ala Gly Cys Gly Ser Ala Glu Glu Val Leu Lys Asp Ile Glu
            20                  25                  30

Lys Ala Leu Arg Asn Ala Gly Ser Lys Asp Cys Leu Lys Glu Ile Phe
        35                  40                  45

Ser Gly Ile Lys Glu Ile Lys Cys Gly Lys Glu Gln Ala Glu Lys Leu
    50                  55                  60

Lys Lys Glu Trp Glu Arg Ile Lys Lys Glu Ala Gly Asp Gly
65                  70                  75

<210>  69
<211>  78
<212>  PRT
<213>  Artificial Sequence

<220>
<223>  S0_1.40

<400>  69

Ala Asp Gln Ile Lys Asn Tyr Ile Asp Lys Gln Leu Leu Pro Ile Val
1               5                   10                  15

Asn Lys Ala Gly Cys Gly Ser Ala Glu Glu Val Leu Lys Asp Ile Glu
            20                  25                  30

Lys Ala Leu Arg Asn Ala Gly Ser Lys Asp Ala Leu Lys Glu Ile Phe
        35                  40                  45

Ser Gly Ile Lys Glu Ile Lys Cys Gly Lys Glu Gln Ala Glu Lys Leu
    50                  55                  60

Lys Lys Glu Trp Glu Arg Ile Lys Lys Glu Ala Gly Asp Gly
65                  70                  75

<210>  70
<211>  61
<212>  PRT
<213>  Artificial Sequence

<220>
<223>  S4_2.07

<400>  70

Met Cys Ser Val Val Val Gly Glu Asn Tyr Ser Ile Lys Cys Asn Pro
1               5                   10                  15

Asp Gly Lys Cys Thr Ile Glu Asp Lys Asn Arg Gly Ile Ile Lys Thr
            20                  25                  30

Val Thr Gly Ser Arg Cys Glu Leu Leu Tyr Lys Ala Val Gln Lys Ala

        35                  40                  45

Gln Lys Gly Ser Trp Gly Ser His His His His His His
    50                  55                  60

<210> 71
<211> 61
<212> PRT
<213> Artificial Sequence

<220>
<223> S4_2.19

<400> 71

Met Pro Asn Thr Asn Val Phe Pro Ser Phe Ser Phe Thr Cys Leu Pro
1               5                   10                  15

Asp Gly Lys Cys Ile Ile Glu Asp Ser Gln Arg Gly Ile Ile Lys Thr
            20                  25                  30

Gly Lys Asn Lys Glu Glu Phe Met Glu Asp Phe Glu Lys Gln Val Arg
        35                  40                  45

Ala Gln Gly Ser Trp Gly Ser His His His His His His
    50                  55                  60

<210> 72
<211> 67
<212> PRT
<213> Artificial Sequence

<220>
<223> S4_2.20

<400> 72

Met Pro Ser Ile Tyr Ser Asp Val Pro Gly Gly Asn Ile Lys Val Lys
1               5                   10                  15

Cys His Glu Gly Lys Cys Thr Ile Glu Asp Ser Gln Arg Gly Ile Ile
            20                  25                  30

Lys Thr Val Pro Met Ile Glu Thr Gly Glu Glu Met Trp Lys Gln Val
        35                  40                  45

Gln Glu Val Leu Glu Glu Lys Arg Gly Ser Trp Gly Ser His His His
    50                  55                  60

His His His
65

<210>  73
<211>  59
<212>  PRT
<213>  Artificial Sequence

<220>
<223>  S4_2.21

<400>  73

Met Pro Lys Thr Asn Val Ile Pro Ser Phe Ser Phe Thr Cys Leu Gly
1               5                   10                  15

Glu Lys Cys Thr Ile Glu Asp Ser Gln Arg Gly Ile Ile Lys Thr Gly
            20                  25                  30

Lys Asn Lys Glu Glu Val Leu Glu Asp Phe Glu Lys Glu Glu Arg Ala
        35                  40                  45

Gln Gly Ser Trp Ser His His His His His His
    50                  55

<210>  74
<211>  66
<212>  PRT
<213>  Artificial Sequence

<220>
<223>  S4_2.22

<400>  74

Met Pro Ser Ile Tyr Ser Asp Val Pro Gly Asn Ile Lys Val Lys Cys
1               5                   10                  15

His Glu Gly Lys Cys Thr Ile Glu Asp Ser Gln Arg Gly Ile Ile Lys
            20                  25                  30

Thr Val Pro Met Ile Glu Thr Gly Glu Glu Met Trp Lys Gln Pro Gln
        35                  40                  45

Glu Leu Leu Glu Glu Lys Arg Gly Ser Trp Gly Ser His His His His
    50                  55                  60

His His
65

<210>  75
<211>  61
<212>  PRT
<213>  Artificial Sequence

<220>
<223>  S4_2.35

<400>  75

Met Pro Asn Thr Asn Val Phe Pro Ser Phe Ser Phe Thr Cys Leu Pro
1               5                   10                  15

Asp Gly Lys Cys Ile Ile Glu Asp Ser Gln Arg Gly Ile Ile Lys Thr
            20                  25                  30

Gly Lys Asn Lys Glu Glu Phe Met Glu Asp Phe Glu Lys Lys Val Arg
        35                  40                  45

Ala Gln Gly Ser Trp Gly Ser His His His His His His
    50                  55                  60

<210>  76
<211>  61
<212>  PRT
<213>  Artificial Sequence

<220>
<223>  S4_2.45

<400>  76

Met Val Cys Ser Val Val Val Gly Glu Asn Tyr Ser Ile Lys Cys Asp
1               5                   10                  15

Ala Thr Lys Cys Thr Ile Glu Asp Lys Asn Arg Gly Ile Ile Lys Thr
            20                  25                  30

Val Thr Gly Ser Arg Cys Glu Glu Leu Ala Lys Ala Val Gln Lys Ala
        35                  40                  45

Gln Lys Gly Ser Trp Gly Ser His His His His His His
    50                  55                  60

<210>  77
<211>  67
<212>  PRT
<213>  Artificial Sequence

<220>
<223>  S4_2.60

<400>  77

Met Pro Ser Ile Tyr Ser Asp Val Pro Gly Gly Asn Val Lys Val Lys
1               5                   10                  15

Cys His Glu Gly Lys Cys Thr Ile Glu Asp Ser Gln Arg Gly Ile Ile
            20                  25                  30

Lys Thr Val Pro Met Ile Glu Thr Gly Glu Glu Met Trp Lys Gln Val
        35                  40                  45

Gln Glu Val Val Glu Glu Lys Arg Gly Ser Trp Gly Ser His His His
    50                  55                  60

His His His
65

<210> 78
<211> 67
<212> PRT
<213> Artificial Sequence

<220>
<223> S4_2.68

<400> 78

Met Pro Ser Ile His Ser Val Val Val Gly Gly Asn Ile Lys Val Lys
1               5                   10                  15

Cys His Glu Gly Lys Cys Thr Ile Glu Asp Ser Gln Arg Gly Ile Ile
            20                  25                  30

Lys Thr Val Pro Met Ile Glu Thr Gly Glu Glu Met Gln Lys Gln Val
        35                  40                  45

Gln Glu Phe Leu Glu Ala Lys Arg Gly Ser Trp Gly Ser His His His
    50                  55                  60

His His His
65

<210> 79
<211> 61
<212> PRT
<213> Artificial Sequence

<220>
<223> S4_2.73

<400> 79

Met Val Cys Ser Val Val Val Gly Glu Asn Tyr Ser Ile Lys Cys Asp
1               5                   10                  15

Ala Thr Lys Cys Thr Ile Glu Asp Ser Gln Arg Gly Ile Ile Lys Thr
            20                  25                  30

Gly Thr His Pro Glu Glu Phe Leu Glu Asp Leu Glu Lys Lys Ala Arg
        35                  40                  45

Ala Gln Gly Ser Trp Gly Ser His His His His His His
    50                  55                  60

<210>  80
<211>  61
<212>  PRT
<213>  Artificial Sequence

<220>
<223>  S4_2.74

<400>  80

Met Val Phe Ser Cys Val Val Gly Glu Asn Tyr Ser Ile Lys Cys Asp
1               5                   10                  15

Ala Thr Lys Cys Thr Ile Glu Asp Ser Gln Arg Gly Ile Ile Lys Thr
            20                  25                  30

Gly Thr His Pro Glu Glu Phe Leu Glu Asp Leu Glu Lys Lys Ala Arg
        35                  40                  45

Ala Gln Gly Ser Trp Gly Ser His His His His His His
    50                  55                  60

<210>  81
<211>  67
<212>  PRT
<213>  Artificial Sequence

<220>
<223>  S4_2.84

<400>  81

Met Pro Ser Ile His Ser Val Val Pro Gly Gly Asn Ile Lys Val Lys
1               5                   10                  15

Cys His Glu Gly Lys Cys Thr Ile Glu Asp Ser Gln Arg Gly Ile Ile
            20                  25                  30

Lys Thr Val Pro Met Ile Glu Thr Gly Glu Glu Met Trp Lys Gln Pro
        35                  40                  45

Gln Glu Leu Leu Glu Glu Lys Arg Gly Ser Trp Gly Ser His His His
    50                  55                  60

His His His
65

<210>  82
<211>  61

<212>  PRT
<213>  Artificial Sequence

<220>
<223>  S4_2.85

<400>  82

Met Pro Asn Thr Asn Val Phe Pro Ser Phe Ser Phe Thr Cys Leu Pro
1               5                   10                  15

Asp Gly Lys Cys Ile Ile Glu Asp Ser Gln Arg Gly Ile Ile Lys Thr
            20                  25                  30

Gly Lys Asn Lys Glu Glu Phe Met Glu Asp Phe Glu Lys Gln Val Arg
        35                  40                  45

Ala Gln Gly Ser Trp Gly Ser His His His His His His
    50                  55                  60

<210>  83
<211>  66
<212>  PRT
<213>  Artificial Sequence

<220>
<223>  S4_2.94

<400>  83

His Met Pro Ser Ile His Ser Val Val Ala Gly Gly Asn Ile Lys Val
1               5                   10                  15

Lys Cys His Glu Gly Lys Cys Thr Ile Glu Asp Ser Gln Arg Gly Ile
            20                  25                  30

Ile Lys Thr Phe Thr Gly Phe Glu Pro Glu Glu Val Trp Lys Gln Ala
        35                  40                  45

Gln Glu Phe Leu Glu Glu Lys Arg Gly Ser Trp Gly Ser His His His
    50                  55                  60

His His
65

<210>  84
<211>  69
<212>  PRT
<213>  Artificial Sequence

<220>
<223>  S0_2.37

<400>  84

Met Ser Cys Asp Gln Ile Lys Asn Tyr Ile Asp Lys Gln Leu Leu Pro
1               5                   10                  15

Ile Val Asn Lys Ala Gly Cys Ser Arg Pro Glu Glu Leu Glu Glu Arg
            20                  25                  30

Ile Arg Arg Ala Leu Lys Lys Phe Gly Asp Thr Asp Cys Leu Lys Asp
        35                  40                  45

Ile Leu Leu Gly Ile Lys Glu Trp Lys Cys Gly Gly Ser Leu Glu His
    50                  55                  60

His His His His His
65

<210>  85
<211>  69
<212>  PRT
<213>  Artificial Sequence

<220>
<223>  S0_2.79

<400>  85

Met Pro Cys Asp Lys Gln Lys Asn Tyr Ile Asp Lys Gln Leu Leu Pro
1               5                   10                  15

Ile Val Asn Lys Ala Gly Cys Ser Arg Pro Glu Glu Val Glu Glu Met
            20                  25                  30

Val Arg Arg Ala Leu Lys Lys Leu Gly Glu Thr Pro Cys Leu Glu Asp
        35                  40                  45

Ile Leu Arg Gly Ile Lys Glu Ile Lys Cys Gly Gly Ser Leu Glu His
    50                  55                  60

His His His His His
65

<210>  86
<211>  69
<212>  PRT
<213>  Artificial Sequence

<220>
<223>  S0_2.10

<400>  86

Met Pro Cys Asp Asp Ala Lys Asn Tyr Ile Asp Lys Gln Leu Leu Pro
1               5                   10                  15

Ile Val Asn Lys Ala Gly Cys Ser Arg Pro Glu Glu Val Glu Arg Ala
            20                  25                  30

Val Arg Lys Met Leu Lys Lys Met Gly Asn Thr Asp Cys Leu Glu Asp
        35                  40                  45

Ile Leu Arg Gly Ile Lys Glu Ile Lys Cys Gly Gly Ser Leu Glu His
    50                  55                  60

His His His His His
65

<210>  87
<211>  69
<212>  PRT
<213>  Artificial Sequence

<220>
<223>  S0_2.102

<400>  87

Met Ser Cys Asp Gln Ile Lys Asn Tyr Ile Asp Lys Gln Leu Leu Pro
1               5                   10                  15

Ile Val Asn Lys Ala Gly Cys Gly Ser Ala Lys Glu Val Gln Lys Asp
            20                  25                  30

Ile Glu Lys Ala Leu Arg Asn Ala Gly Val Lys Asp Cys Leu Glu Asp
        35                  40                  45

Ile Leu Arg Gly Ile Lys Glu Trp Lys Cys Gly Gly Ser Leu Glu His
    50                  55                  60

His His His His His
65

<210>  88
<211>  69
<212>  PRT
<213>  Artificial Sequence

<220>
<223>  S0_2.31

<400>  88

Met Ser Cys Asp Glu Ser Lys Asn Tyr Ile Asp Lys Gln Leu Leu Pro
1               5                   10                  15

Ile Val Asn Lys Ala Gly Cys Asp Arg Pro Glu Asp Val Glu Arg Trp

20                  25                  30

Ile Arg Lys Ala Leu Lys Lys Met Gly Asp Thr Ser Cys Phe Asp Glu
        35                  40                  45

Ile Leu Lys Gly Leu Lys Glu Ile Lys Cys Gly Gly Ser Leu Glu His
    50                  55                  60

His His His His His
65

<210>  89
<211>  69
<212>  PRT
<213>  Artificial Sequence

<220>
<223>  S0_2.197

<400>  89

Met Ser Cys Asp Gln Ile Lys Asn Tyr Ile Asp Lys Gln Leu Leu Pro
1               5                   10                  15

Ile Val Asn Lys Ala Gly Cys Ser Arg Pro Glu Glu Val Glu Glu Arg
            20                  25                  30

Ile Arg Arg Ala Leu Lys Lys Met Gly Asp Thr Ser Cys Phe Asp Glu
        35                  40                  45

Ile Met Lys Gly Leu Lys Glu Ile Lys Cys Gly Gly Ser Leu Glu His
    50                  55                  60

His His His His His
65

<210>  90
<211>  69
<212>  PRT
<213>  Artificial Sequence

<220>
<223>  S0_2.57516

<400>  90

Met Ser Cys Asp Gln Ile Lys Asn Tyr Ile Asp Lys Gln Leu Leu Pro
1               5                   10                  15

Ile Val Asn Lys Ala Gly Cys Asn Arg Pro Glu Glu Phe Glu Glu Trp
            20                  25                  30

Ile Lys Arg Ala Leu Lys Lys Leu Gly Asp Thr Ser Cys Leu Glu Asp
        35                  40                  45

Ile Leu Arg Gly Ile Lys Glu Ile Lys Cys Gly Gly Ser Leu Glu His
    50                  55                  60

His His His His His
65

<210>  91
<211>  69
<212>  PRT
<213>  Artificial Sequence

<220>
<223>  S0_2.57575

<400>  91

Met Ser Cys Asp Gln Ile Lys Asn Tyr Ile Asp Lys Gln Leu Leu Pro
1               5                   10                  15

Ile Val Asn Lys Ala Gly Cys Ser Arg Pro Glu Glu Val Glu Glu Met
            20                  25                  30

Val Arg Arg Ala Leu Lys Lys Leu Gly Glu Thr Pro Cys Leu Glu Asp
        35                  40                  45

Ile Leu Arg Gly Ile Lys Glu Trp Lys Cys Gly Gly Ser Leu Glu His
    50                  55                  60

His His His His His
65

<210>  92
<211>  69
<212>  PRT
<213>  Artificial Sequence

<220>
<223>  S0_2.57588

<400>  92

Met Ser Cys Asp Gln Ile Lys Asn Tyr Ile Asp Lys Gln Leu Leu Pro
1               5                   10                  15

Ile Val Asn Lys Ala Gly Cys Ser Arg Pro Glu Glu Val Glu Arg Ala
            20                  25                  30

Val Arg Lys Met Leu Lys Lys Met Gly Asn Thr Asp Cys Leu Glu Asp
        35                  40                  45

Ile Leu Arg Gly Ile Lys Glu Ile Lys Cys Gly Gly Ser Leu Glu His
    50                  55                  60

His His His His His
65

<210>  93
<211>  69
<212>  PRT
<213>  Artificial Sequence

<220>
<223>  S0_2.57855

<400>  93

Met Ser Cys Asp Gln Ile Lys Asn Tyr Ile Asp Lys Gln Leu Leu Pro
1               5                   10                  15

Ile Val Asn Lys Ala Gly Cys Gly Ser Ala Lys Glu Val Gln Lys Asp
            20                  25                  30

Ile Glu Lys Ala Leu Arg Asn Ala Gly Val Lys Asp Cys Leu Lys Glu
        35                  40                  45

Ile Phe Ser Gly Ile Lys Glu Ile Lys Cys Gly Gly Ser Leu Glu His
    50                  55                  60

His His His His His
65

<210>  94
<211>  69
<212>  PRT
<213>  Artificial Sequence

<220>
<223>  S0_2.57910

<400>  94

Met Ser Cys Asp Gln Ile Lys Asn Tyr Ile Asp Lys Gln Leu Leu Pro
1               5                   10                  15

Ile Val Asn Lys Ala Gly Cys Gly Ser Ala Lys Glu Val Gln Lys Asp
            20                  25                  30

Ile Glu Lys Ala Leu Arg Asn Ala Gly Val Lys Asp Cys Leu Glu Asp
        35                  40                  45

Ile Leu Arg Gly Ile Lys Glu Ile Lys Cys Gly Gly Ser Leu Glu His
    50                  55                  60

His His His His His
65


<210>  95
<211>  69
<212>  PRT
<213>  Artificial Sequence

<220>
<223>  S0_2.57911

<400>  95

Met Ser Cys Glu Glu Ala Lys Asn Tyr Ile Asp Lys Gln Leu Leu Pro
1               5                   10                  15


Ile Val Asn Lys Ala Gly Cys Gly Ser Ala Glu Glu Val Gln Lys Asp
            20                  25                  30


Ile Glu Lys Ala Leu Arg Asn Ala Gly Val Lys Asp Cys Leu Glu Asp
        35                  40                  45


Ile Leu Arg Gly Ile Lys Glu Trp Lys Cys Gly Gly Ser Leu Glu His
    50                  55                  60


His His His His His
65


<210>  96
<211>  69
<212>  PRT
<213>  Artificial Sequence

<220>
<223>  S0_2.57

<400>  96

Met Pro Cys Asp Asp Ala Lys Asn Tyr Ile Asp Lys Gln Leu Leu Pro
1               5                   10                  15


Ile Val Asn Lys Ala Gly Cys Ser Arg Pro Glu Glu Val Glu Glu Arg
            20                  25                  30


Ile Arg Arg Ala Leu Lys Lys Met Gly Asp Thr Ser Cys Phe Asp Glu
        35                  40                  45


Ile Met Lys Gly Leu Lys Glu Ile Lys Cys Gly Gly Ser Leu Glu His
    50                  55                  60


His His His His His

65

<210>  97
<211>  69
<212>  PRT
<213>  Artificial Sequence

<220>
<223>  S0_2.58980

<400>  97

Met Ser Cys Glu Glu Ala Lys Asn Tyr Ile Asp Lys Gln Leu Leu Pro
1               5                   10                  15

Ile Val Asn Lys Ala Gly Cys Ser Arg Pro Glu Glu Leu Glu Glu Met
            20                  25                  30

Ile Arg Arg Ala Leu Lys Lys Met Gly Asp Thr Ser Cys Phe Asp Glu
        35                  40                  45

Ile Met Lys Gly Leu Lys Glu Ile Lys Cys Gly Gly Ser Leu Glu His
    50                  55                  60

His His His His His
65

<210>  98
<211>  69
<212>  PRT
<213>  Artificial Sequence

<220>
<223>  S0_2.611

<400>  98

Met Pro Cys Asp Lys Gln Lys Asn Tyr Ile Asp Lys Gln Leu Leu Pro
1               5                   10                  15

Ile Val Asn Lys Ala Gly Cys Gly Ser Ala Lys Glu Val Gln Lys Asp
            20                  25                  30

Ile Glu Lys Ala Leu Arg Asn Ala Gly Val Lys Asp Cys Leu Glu Asp
        35                  40                  45

Ile Leu Arg Gly Ile Lys Glu Trp Lys Cys Gly Gly Ser Leu Glu His
    50                  55                  60

His His His His His
65

<210>  99
<211>  69
<212>  PRT
<213>  Artificial Sequence

<220>
<223>  S0_2.126

<400>  99

Met Pro Cys Asp Lys Gln Lys Asn Tyr Ile Asp Lys Gln Leu Leu Pro
1               5                   10                  15

Ile Val Asn Lys Ala Gly Cys Ser Arg Pro Glu Glu Val Glu Glu Arg
            20                  25                  30

Ile Arg Arg Ala Leu Lys Lys Met Gly Asp Thr Ser Cys Phe Asp Glu
        35                  40                  45

Ile Leu Lys Gly Leu Lys Glu Ile Lys Cys Gly Gly Ser Leu Glu His
    50                  55                  60

His His His His His
65